(19) Europäisches Patentamt
European Patent Office
Office européen des brevets

(11) **EP 4 431 915 A1**

(12) **EUROPEAN PATENT APPLICATION**
published in accordance with Art. 153(4) EPC

(43) Date of publication:
**18.09.2024 Bulletin 2024/38**

(21) Application number: **22898163.5**

(22) Date of filing: **06.07.2022**

(51) International Patent Classification (IPC):
**G01N 21/41** (2006.01)  **G01N 21/45** (2006.01)
**G02B 21/00** (2006.01)  **G02B 21/36** (2006.01)

(52) Cooperative Patent Classification (CPC):
**G01N 21/41; G01N 21/45; G02B 21/00;
G02B 21/36**

(86) International application number:
**PCT/JP2022/026854**

(87) International publication number:
**WO 2023/095378 (01.06.2023 Gazette 2023/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.11.2021 JP 2021192923**

(71) Applicant: **Hamamatsu Photonics K.K.
Hamamatsu-shi, Shizuoka 435-8558 (JP)**

(72) Inventors:
• **TAKEUCHI Kozo**
  **Hamamatsu-shi, Shizuoka 435-8558 (JP)**
• **YASUHIKO Osamu**
  **Hamamatsu-shi, Shizuoka 435-8558 (JP)**
• **YAMADA Hidenao**
  **Hamamatsu-shi, Shizuoka 435-8558 (JP)**

(74) Representative: **Hoffmann Eitle
Patent- und Rechtsanwälte PartmbB
Arabellastraße 30
81925 München (DE)**

(54) **EVALUATION METHOD, EVALUATION DEVICE, AND INFORMATION PROCESSING PROGRAM**

(57) A refractive index distribution evaluation method, evaluation device, and information processing program, comprising: a data acquisition step or a data acquisition unit for acquiring refractive index distribution data for an object of observation; a selection step or a selection unit for selecting whether a target included in the refractive index distribution data is an evaluation target; and an evaluation step or an evaluation unit for evaluating the refractive index distribution of the evaluation target.

**Fig.4**

INTERFERENCE INTENSITY IMAGE ACQUISITION — S1

INTERFERENCE INTENSITY IMAGE FOR EACH LIGHT IRRADIATION DIRECTION

FIRST COMPLEX AMPLITUDE IMAGE GENERATION — S2

COMPLEX AMPLITUDE IMAGE FOR EACH LIGHT IRRADIATION DIRECTION

SECOND COMPLEX AMPLITUDE IMAGE GENERATION — S3

COMPLEX AMPLITUDE IMAGE FOR EACH POSITION AND EACH LIGHT IRRADIATION DIRECTION

TWO-DIMENSIONAL PHASE IMAGE GENERATION — S4

TWO-DIMENSIONAL PHASE IMAGE FOR EACH POSITION

THREE-DIMENSIONAL PHASE IMAGE GENERATION — S5

THREE-DIMENSIONAL PHASE IMAGE

REFRACTIVE INDEX DISTRIBUTION CALCULATION — S6

THREE-DIMENSIONAL REFRACTIVE INDEX DISTRIBUTION

EP 4 431 915 A1

## Description

### Technical Field

The present invention relates to an evaluation method, an evaluation device, and an information processing program.

### Background Art

One of the important functions of the liver is lipid metabolism. Abnormalities in lipid metabolism can lead not only to liver abnormalities such as cirrhosis and liver cancer, but also to disturbances in the lipid balance in the blood. If this abnormality in lipid metabolism continues chronically, fatal vascular diseases (arteriosclerosis, myocardial infarction, cerebral infarction, etc.) can be caused (Non-Patent Documents 1 and 2). Therefore, it is important to develop a medicament for suppressing the abnormality of lipid metabolism in order to keep the function of the liver normal and prevent diseases of the vascular system.

One method for studying lipid metabolism is to analyze lipid droplets in an inner part of a two-dimensional or three-dimensional cell culture based on the refractive index distribution. In particular, it is known that a three-dimensional culture (cell aggregate) of HepG2 cells exhibits behavior similar to that of a living body even when the diameters thereof are about 100 to 200 $\mu$m (Non-Patent Documents 3 and 4). Cell aggregates having such a size can be easily prepared, and the experiment can be performed with good reproducibility.

As a method for analyzing lipid droplets in an inner part of a culture, for example, a method may be considered in which a plurality of slice images of cells labeled with a chemical substance causing fluorescence or luminescence are acquired, characteristics of the cells are evaluated based on the acquired plurality of slice images, and the center of gravity of the cell aggregate is determined from a three-dimensional image of the cell aggregate constructed from the acquired plurality of slice images to evaluate the cell aggregate (Patent Document 1).

In general, however, it is preferred that the observation of the culture be performed non-staining and non-invasive. As non-staining and non-invasive imaging methods, optical coherence tomography (OCT), quantitative phase imaging (QPI), and the like are known.

### Citation List

### Patent Literature

Patent Document 1: JP 2016-223893

Non-Patent Document 1: Heyens et al. Liver fibrosis in non-alcoholic fatty liver disease: From liver biopsy to non-invasive biomarkers in diagnosis and treatment. Front Med. 2021.
Non-Patent Document 2: Deprince et al. Dysregulated lipid metabolism links NAFLD to cardiovascular disease. Mol Metab. 2020.
Non-Patent Document 3: Chang et al. Monolayer and spheroid culture of human liver hepatocellular carcinoma cell line cells demonstrate distinct global gene expression patterns and functional phenotypes. Tissue Eng Part A. 2009.
Non-Patent Document 4: Nishikawa et al. Optimization of albumin secretion and metabolic activity of cytochrome P450 1A1 of human hepatoblastoma HepG2 cells in multicellular spheroids by controlling spheroid size. BiolPharm Bull. 2017.
Non-Patent Document 5: Kim et al. Three-dimensional label-free imaging and quantification of lipid droplets in live hepatocytes. Sci Rep. 2016. Non-Patent Document 6: Gomez-Lechon et al. A human hepatocellular in vitro model to investigate steatosis. Chem Biol Interact.2007.

### Summary of Invention

### Technical Problem

However, in OCT and QPI, it is difficult to evaluate the refractive index distribution of a multiple scattering object such as a three-dimensional culture in particular.

An object of the present invention is to provide an evaluation method, an evaluation apparatus and an information processing program that are non-staining and non-invasive with respect to an observation object, and are capable of evaluating a refractive index distribution even when the observation object is a multiple scattering object.

**Solution to Problem**

One aspect of the present invention is a refractive index distribution evaluating method including: a data acquisition step of acquiring refractive index distribution data of an observation target; a selection step of selecting whether or not an object included in the refractive index distribution data is an evaluation object; and an evaluation step of evaluating the refractive index distribution of the evaluation object.

Another aspect of the present invention is an apparatus for evaluating a refractive index distribution, including: a data acquisition unit for acquiring refractive index distribution data of an observation object; a selection unit for obtaining an indicator for an object included in the refractive index distribution data from the refractive index distribution data and selecting whether or not the object is an evaluation object based on the indicator; and an evaluation unit for evaluating the refractive index distribution of the evaluation object.

Another aspect of the present invention is an information processing program for causing a computer to execute steps: a data acquisition step of acquiring refractive index distribution data of an observation object, a selection step of obtaining an indicator for an object included in the refractive index distribution data from the refractive index distribution data and selecting whether or not the object is an evaluation object based on the indicator, and an evaluation step of evaluating the refractive index distribution of the evaluation object.

The data acquisition step or the data acquisition unit may include acquiring for each of a plurality of light irradiation directions, an interference intensity image generated by interference between light irradiating the evaluation object along each of the plurality of light irradiation directions and passed through the evaluation object and reference light; a step of generating, for each of the plurality of light irradiation directions, a complex amplitude image at each of a plurality of positions based on the interference intensity image; a step of generating, for each of the plurality of positions, a complex differential interference image of each of the plurality of light irradiation directions based on the complex amplitude image of each of the plurality of light irradiation directions, and generating a two-dimensional phase image based on the complex differential interference image of each of the plurality of light irradiation directions; and a step of calculating a refractive index distribution based on the two-dimensional phase image at each of the plurality of positions.

The selection step or the selection unit may include a step of acquiring at least one refractive index cross sectional data in a certain direction from the refractive index distribution data; and a step of selecting whether or not an object included in the refractive index cross sectional data is an evaluation object.

The evaluation step or the evaluation method in the evaluation unit may include a step of extracting a region having a refractive index greater than or less than a threshold, and may further include a step of dividing the region by its shape, its size, its density or its position in the observation object.

The evaluation step or the evaluation method in the evaluation unit may include a step of extracting a region having a refractive index greater than or less than a threshold, and may further include a step of specifying one or more positions in the evaluation object and dividing the evaluation object into a region present at a predetermined distance from the position and a region present at a distance farther than the predetermined distance from the position

The observation object may be a three-dimensional culture.

The refractive index distribution data may include at least one refractive index data selected from the group consisting of a lipid droplet, a mitochondrion, a vesicle, a nucleolus and DNA.

The present invention provides the following [1] to [10]:

[1] A method for evaluating a refractive index distribution including: a data acquisition step of acquiring refractive index distribution data of an observation object; a selection step of selecting whether or not an object included in the refractive index distribution data is an evaluation object; and an evaluation step of evaluating the refractive index distribution of the evaluation object.

[2] The evaluation method according to [1], wherein the data acquisition step includes: acquiring for each of a plurality of light irradiation directions, an interference intensity image generated by interference between light irradiating the evaluation object along each of the plurality of light irradiation directions and passed through the evaluation object and reference light; a step of generating, for each of the plurality of light irradiation directions, a complex amplitude image at each of a plurality of positions based on the interference intensity image; a step of generating, for each of the plurality of positions, a complex differential interference image of each of the plurality of light irradiation directions based on the complex amplitude image of each of the plurality of light irradiation directions, and generating a two-dimensional phase image based on the complex differential interference image of each of the plurality of light irradiation directions; and a step of calculating a refractive index distribution based on the two-dimensional phase image at each of the plurality of positions.

[3] The evaluation method according to [1] or [2], wherein the selection step includes: acquiring at least one refractive index cross sectional data in a certain direction from the refractive index distribution data; and selecting whether or not an object included in the refractive index cross sectional data is an evaluation object.

[4] The evaluation method according to any one of [1] to [3], wherein an evaluation method in the evaluation step includes: extracting a region having a refractive index greater than or less than a threshold.

[5] The evaluation method according to [4], wherein an evaluation method in the evaluation step includes: a step of dividing the region by its shape, its size, its density or its position in the observation object.

[6] The evaluation method according to [4], wherein an evaluation method in the evaluation step includes: a step of specifying one or more positions in the evaluation object and dividing the evaluation object into a region present at a predetermined distance from the position and a region present at a distance farther than the predetermined distance from the position.

[7] The evaluation method according to any one of [1] to [6], wherein the observation object is a three-dimensional culture.

[8] The evaluation method according to any one of [1] to [7], wherein the refractive index distribution data includes: at least one refractive index data selected from the group consisting of a lipid droplet, a mitochondrion, a vesicle, a nucleolus and DNA.

[9] An apparatus for evaluating a refractive index distribution including: a data acquisition unit for acquiring refractive index distribution data of an observation object; a selection unit for obtaining an indicator for an object included in the refractive index distribution data from the refractive index distribution data and selecting whether or not the object is an evaluation object based on the indicator; and an evaluation unit for evaluating the refractive index distribution of the evaluation object.

[10] An information processing program for causing a computer to execute steps: a data acquisition step of acquiring refractive index distribution data of an observation object; a selection step of obtaining an indicator for an object included in the refractive index distribution data from the refractive index distribution data and selecting whether or not the object is an evaluation object based on the indicator; and an evaluation step of evaluating the refractive index distribution of the evaluation object.

**Advantageous Effects of Invention**

According to the present invention, it is possible to provide an evaluation method, an evaluation apparatus, and an information processing program that are non-staining and non-invasive with respect to an observation object and are capable of evaluating a refractive index distribution even when the observation object is a multiple scattering object.

**Brief Description of Drawings**

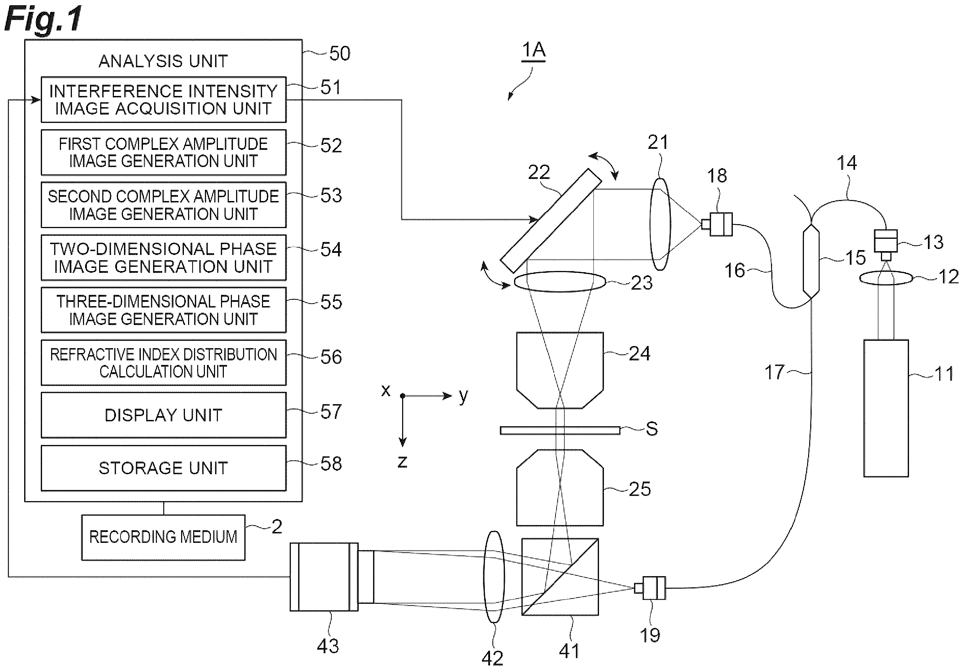

[FIG. 1] FIG. 1 (hereinafter also referred to as "FIG. A01 ") is a diagram illustrating a configuration of an observation apparatus 1A.

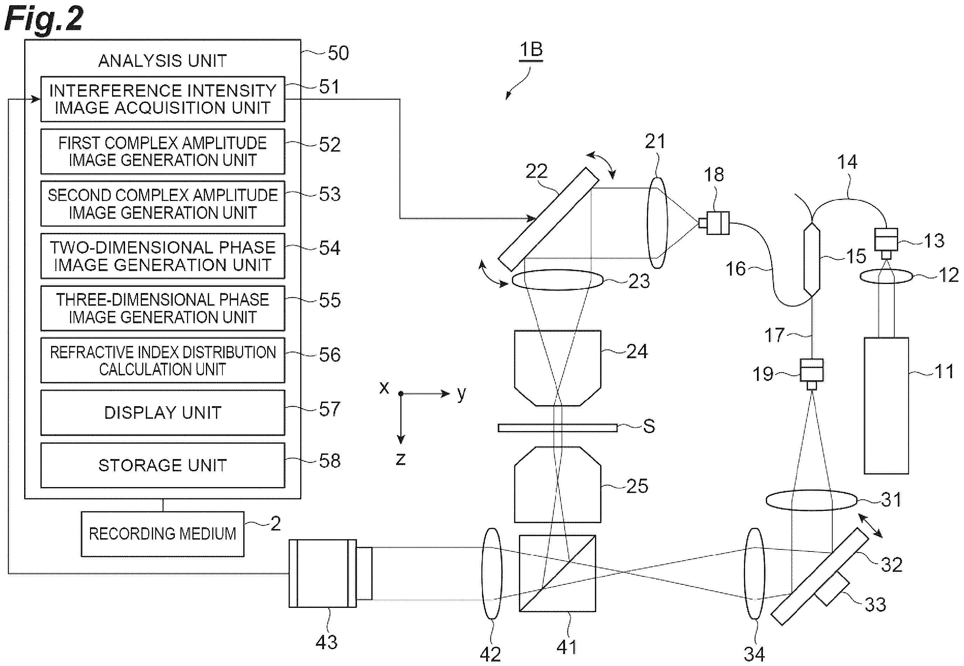

[FIG. 2] FIG. 2 (hereinafter also referred to as "FIG. A02") is a diagram illustrating a configuration of an observation apparatus 1B.

[FIG. 3] FIG. 3 (hereinafter also referred to as "FIG. A03") is a diagram illustrating a configuration of an observation apparatus 1C.

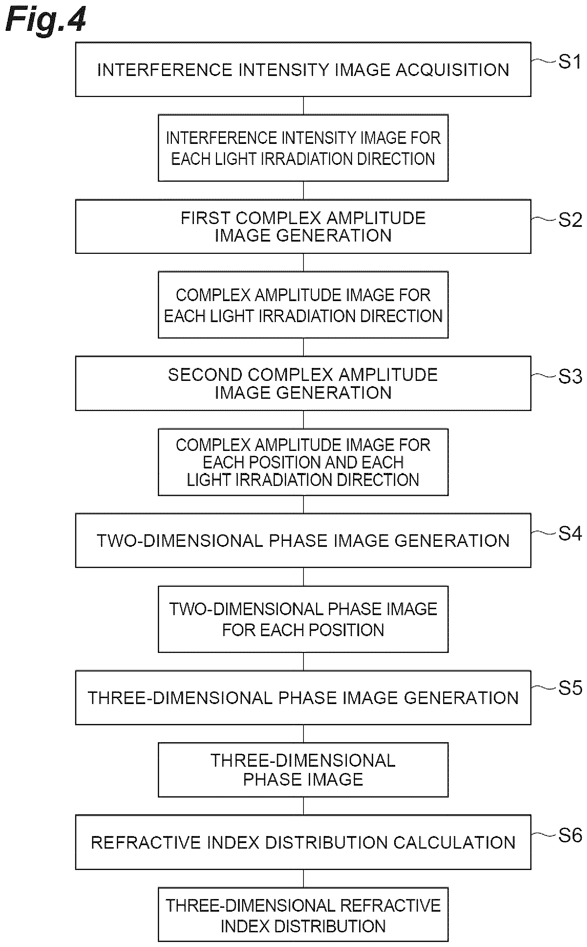

[FIG. 4] FIG. 4 (hereinafter also referred to as "FIG. A04") is a flowchart of a refractive index distribution measuring method A.

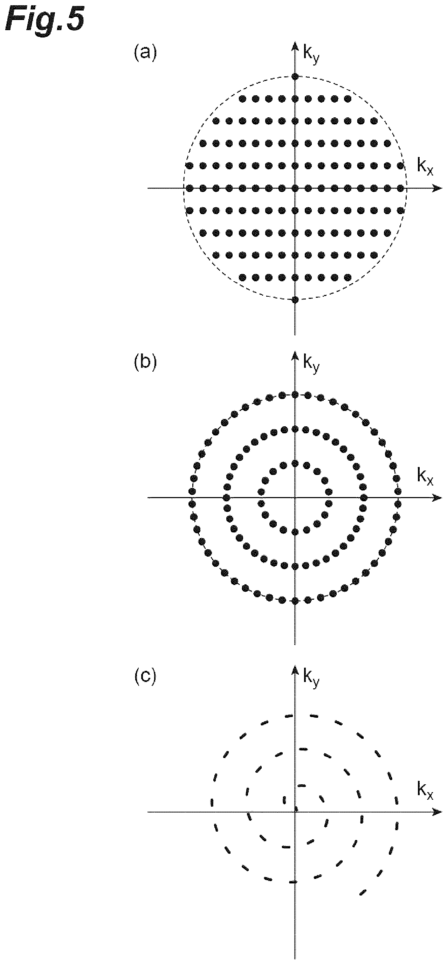

[FIG. 5] FIG. 5 (hereinafter also referred to as "FIG. A05") includes (a) - (c) diagrams illustrating examples of scanning of a light irradiation direction on an observation object S in an interference intensity image acquisition step S1.

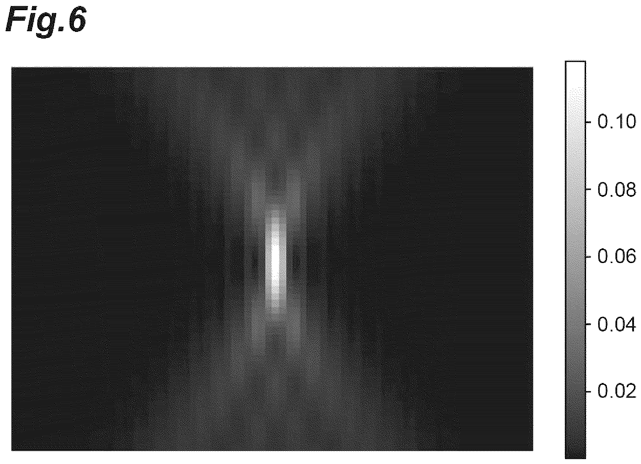

[FIG. 6] FIG. 6 (hereinafter also referred to as "FIG. A06") is a diagram showing a kernel function g.

[FIG. 7] FIG. 7 (hereinafter also referred to as "FIG. A07") includes (a) - (b) diagrams illustrating examples of scanning of a light irradiation direction on an observation object S in an interference intensity image acquisition step S1.

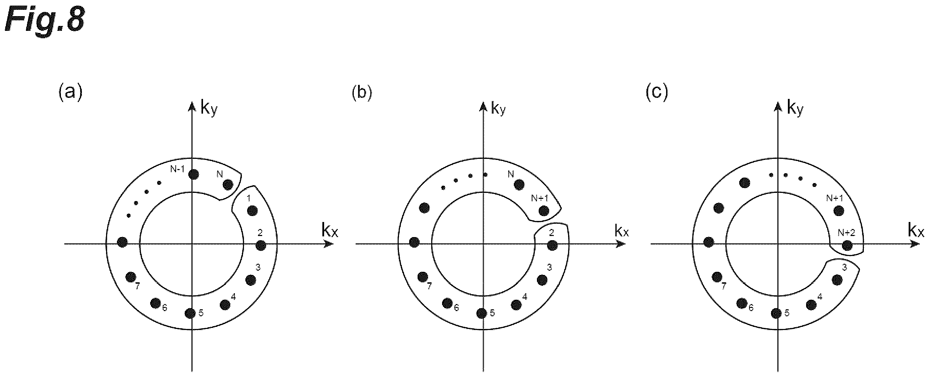

[FIG. 8] FIG. 8 (hereinafter also referred to as "FIG. A08") includes (a) - (c) diagrams illustrating examples of scanning of a light irradiation direction on an observation object S in an interference intensity image acquisition step S1.

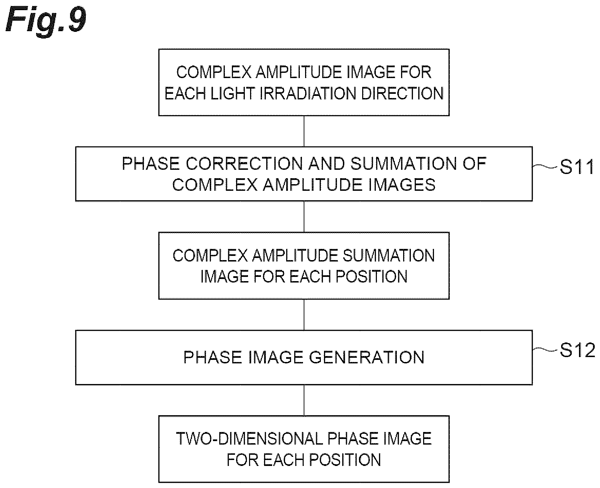

[FIG. 9] FIG. 9 (hereinafter also referred to as "FIG. A09") is a flowchart of a two-dimensional phase image generation step S4 in a refractive index distribution measuring method A1.

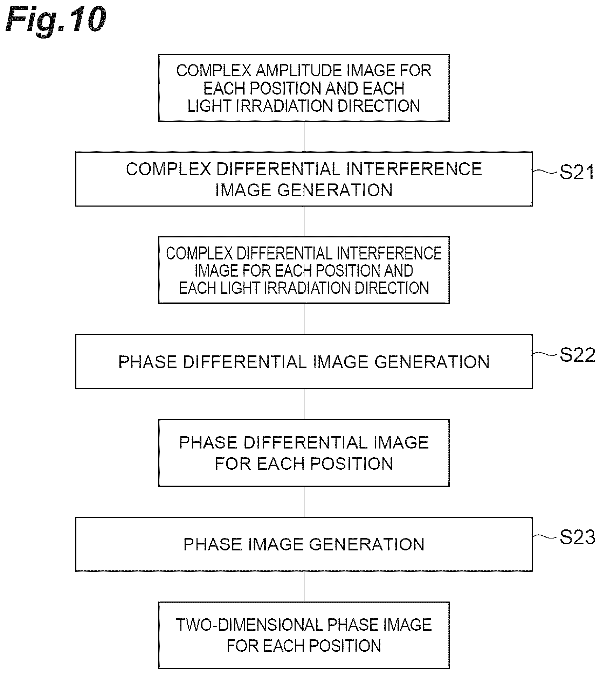

[FIG. 10] FIG. 10 (hereinafter also referred to as "FIG. A10") is a flowchart of a two-dimensional phase image generation step S4 in a refractive index distribution measuring method A2.

[FIG. 11] FIG. 11 (hereinafter also referred to as "FIG. A11") is a diagram showing the kernel function.

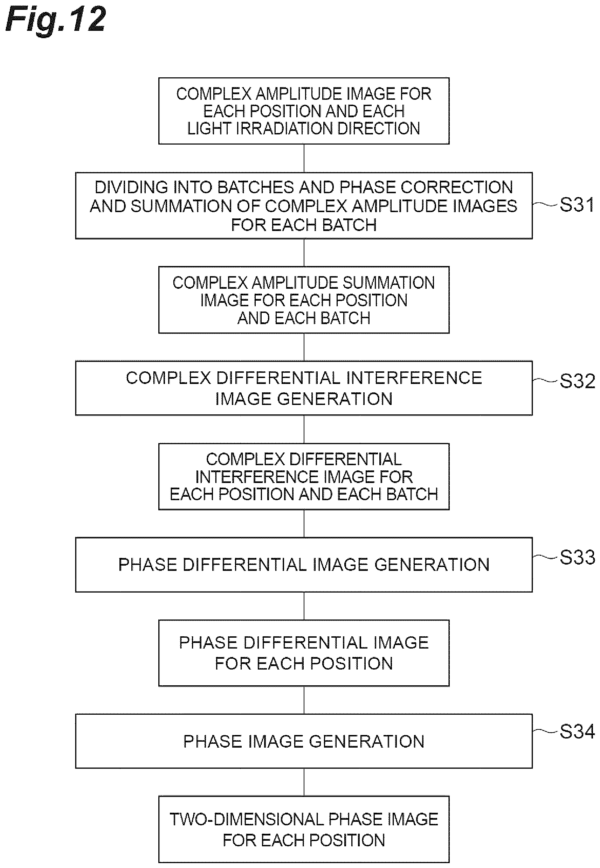

[FIG. 12] FIG. 12 (hereinafter also referred to as "FIG. A12") is a flowchart of a two-dimensional phase image generation step S4 in a refractive index distribution measuring method A3.

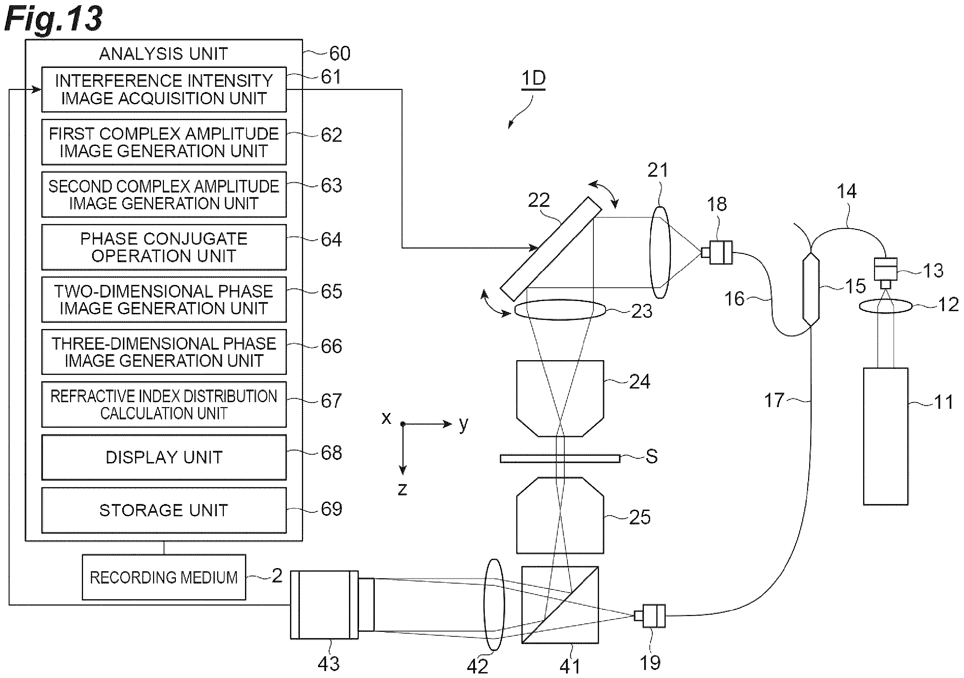

[FIG. 13] FIG. 13 (hereinafter also referred to as "FIG. B01") is a diagram illustrating a configuration of an observation apparatus 1D.

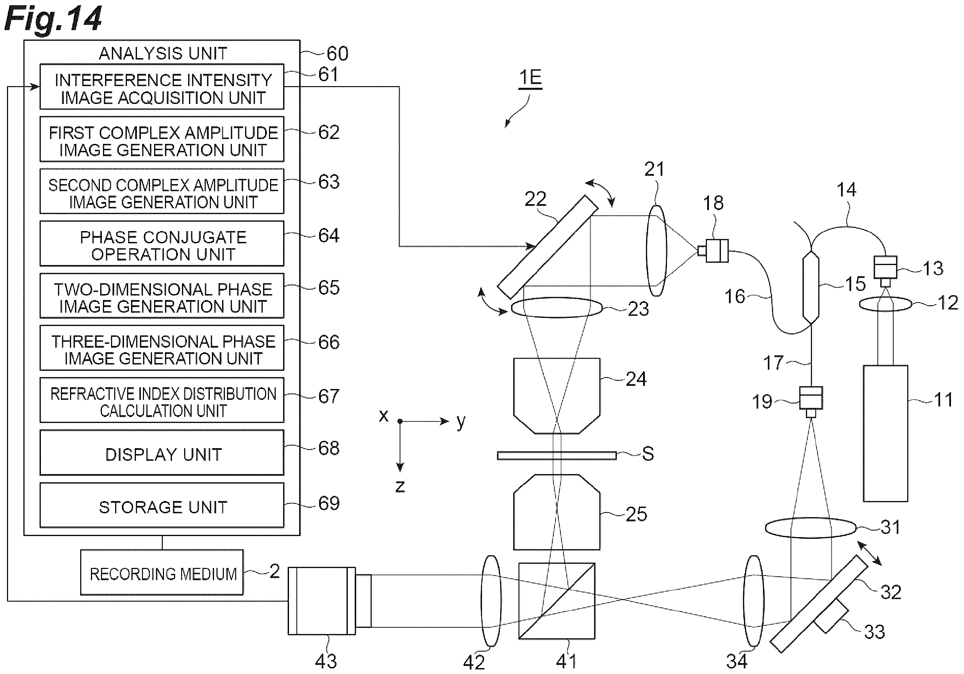

[FIG. 14] FIG. 14 (hereinafter also referred to as "FIG. B02") is a diagram illustrating a configuration of an observation apparatus 1E.

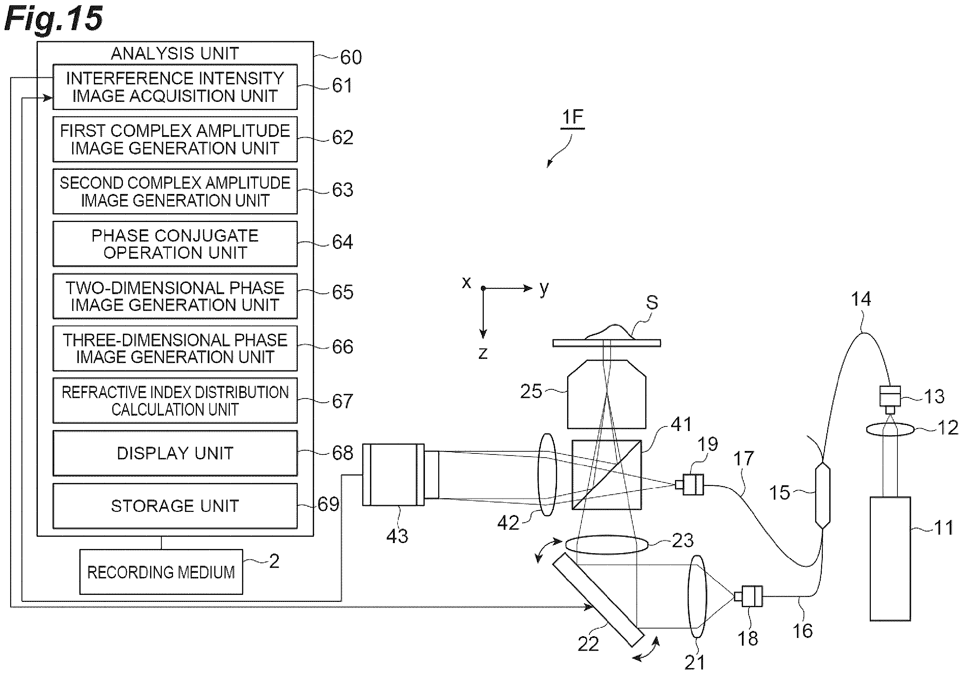

[FIG. 15] FIG. 15 (hereinafter also referred to as "FIG. B03") is a diagram illustrating a configuration of an observation apparatus 1F.

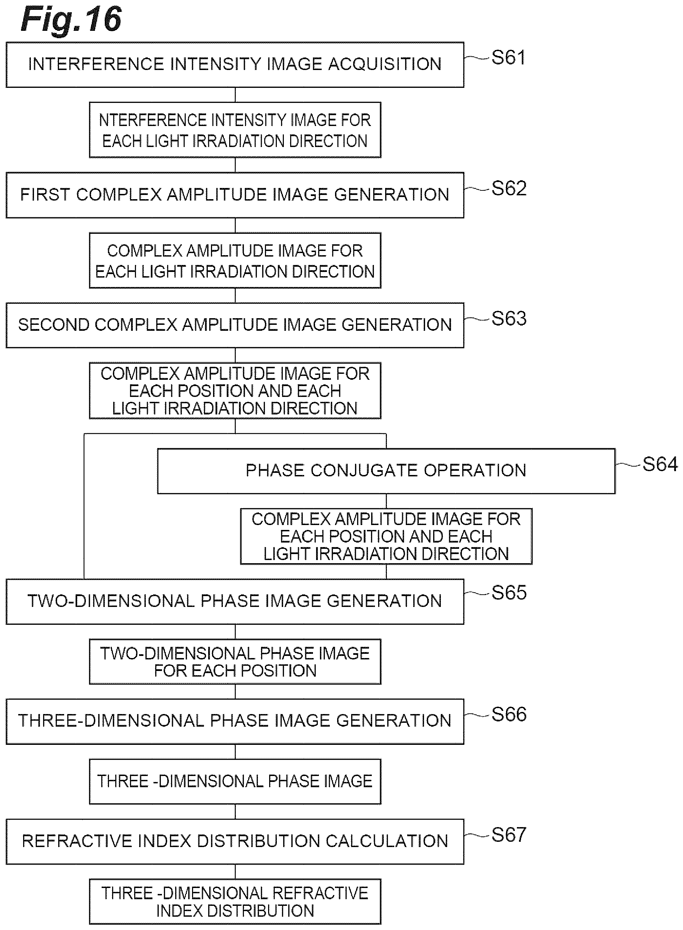

[FIG. 16] FIG. 16 (hereinafter also referred to as "FIG. B04") is a flowchart of a refractive index distribution measuring method B.

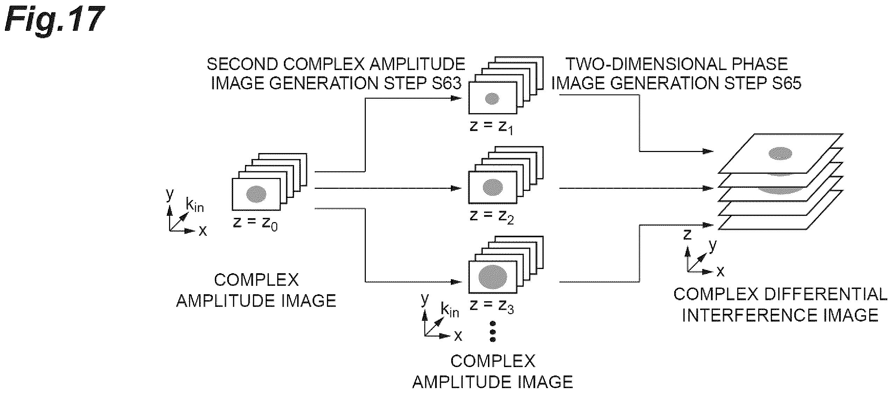

[FIG. 17] FIG. 17 (hereinafter also referred to as "FIG. B05") is a diagram illustrating images and an order of processing steps of a second complex amplitude image generation step S63 and a two-dimensional phase image generation step S65.

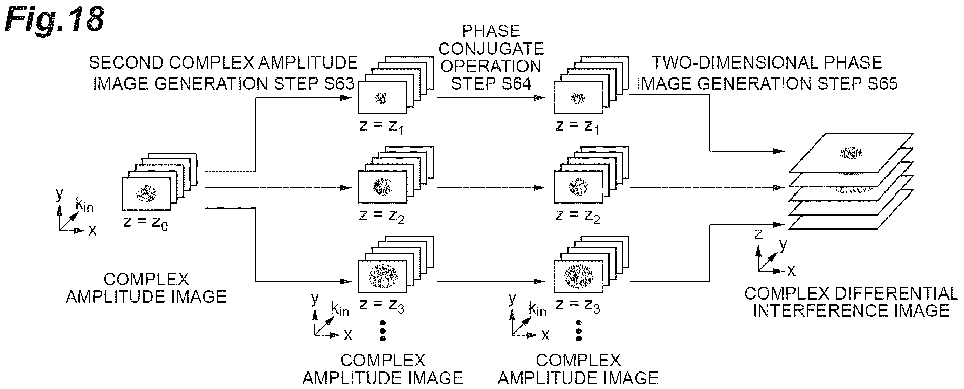

[FIG. 18] FIG. 18 (hereinafter also referred to as "FIG. B06") is a diagram illustrating images and an order of processing steps of the second complex amplitude image generation step S63, a phase conjugate operation step S64, and the two-dimensional phase image generation step S65.

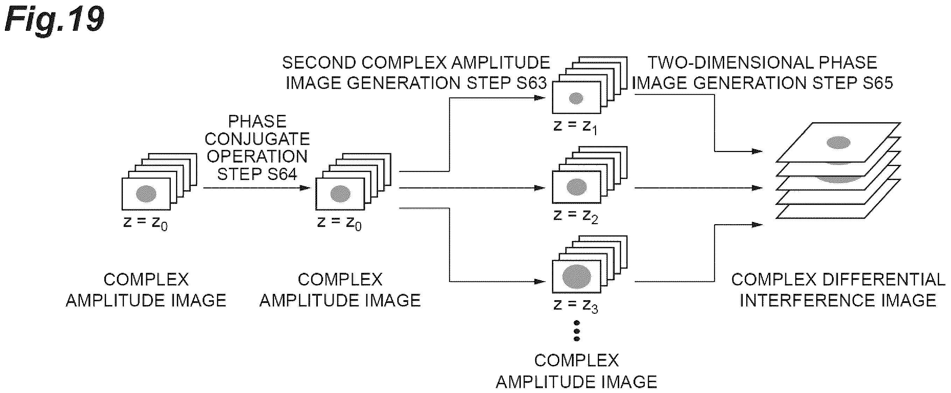

[FIG. 19] FIG. 19 (hereinafter also referred to as "FIG. B07") is a diagram illustrating images and an order of processing steps of the second complex amplitude image generation step S63, the phase conjugate operation step S64, and the two-dimensional phase image generation step S65.

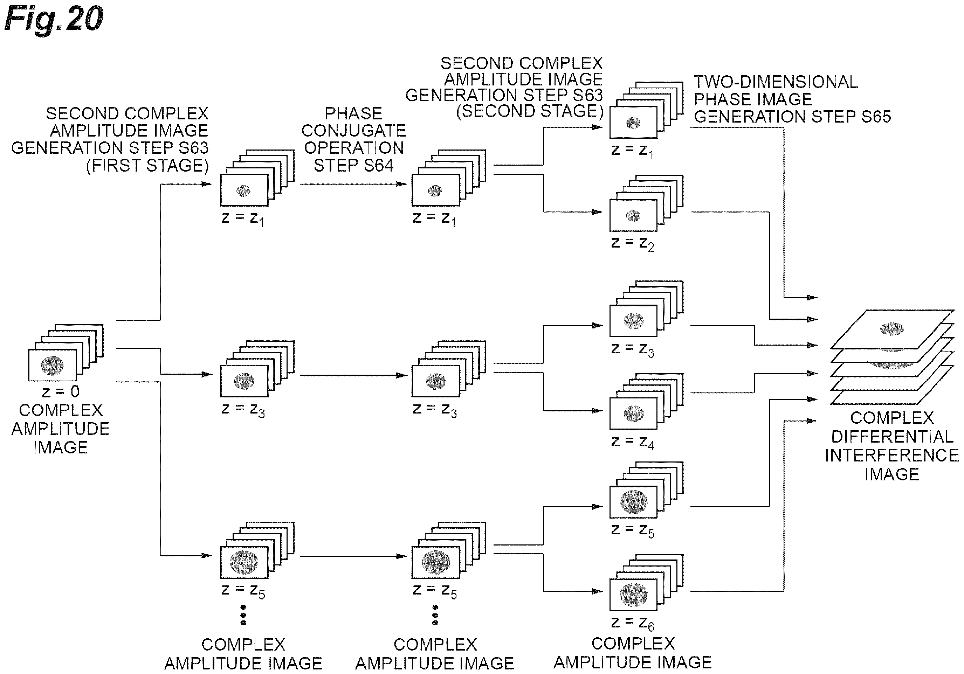

[FIG. 20] FIG. 20 (hereinafter also referred to as "FIG. B08") is a diagram illustrating images and an order of processing steps of the second complex amplitude image generation step S63, the phase conjugate operation step S64, and the two-dimensional phase image generation step S65.

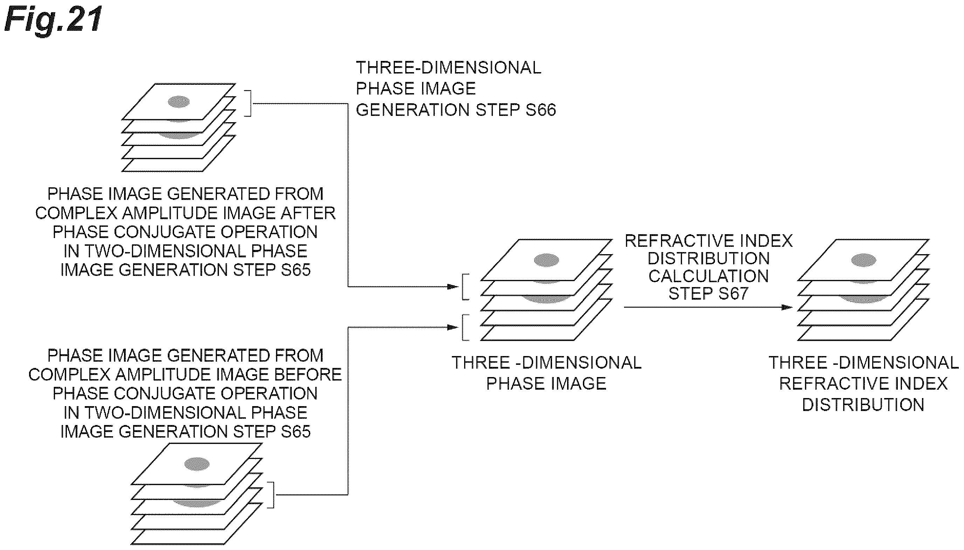

[FIG. 21] FIG. 21 (hereinafter also referred to as "FIG. B09") is a diagram illustrating images and an order of processing steps of a three-dimensional phase image generation step S66 and a refractive index distribution calculation step S67.

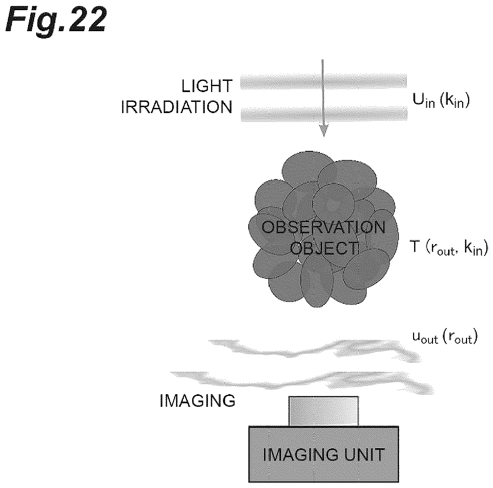

[FIG. 22] FIG. 22 (hereinafter also referred to as "FIG. B10") is a diagram for describing an outline of a phase conjugate operation, and is the diagram illustrating input light and output light when an interference intensity image is imaged by an imaging unit.

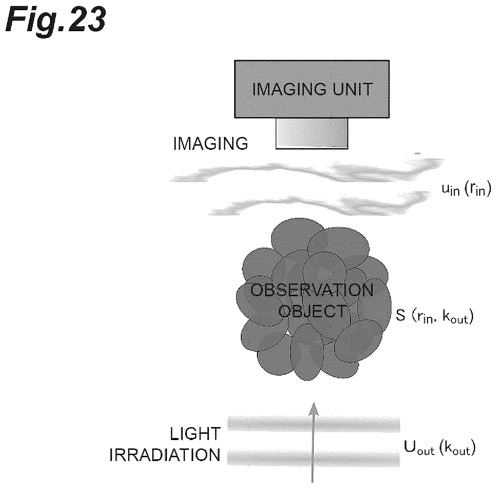

[FIG. 23] FIG. 23 (hereinafter also referred to as "FIG. B11") is a diagram for describing the outline of the phase conjugate operation, and is the diagram illustrating input light and output light in a case in which a relationship between light irradiation and imaging is reversed.

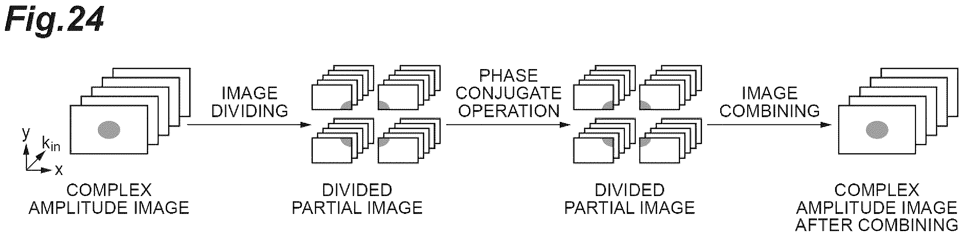

[FIG. 24] FIG. 24 (hereinafter also referred to as "FIG. B12") is a diagram illustrating image dividing, the phase conjugate operation, and image combining in the phase conjugate operation step S64.

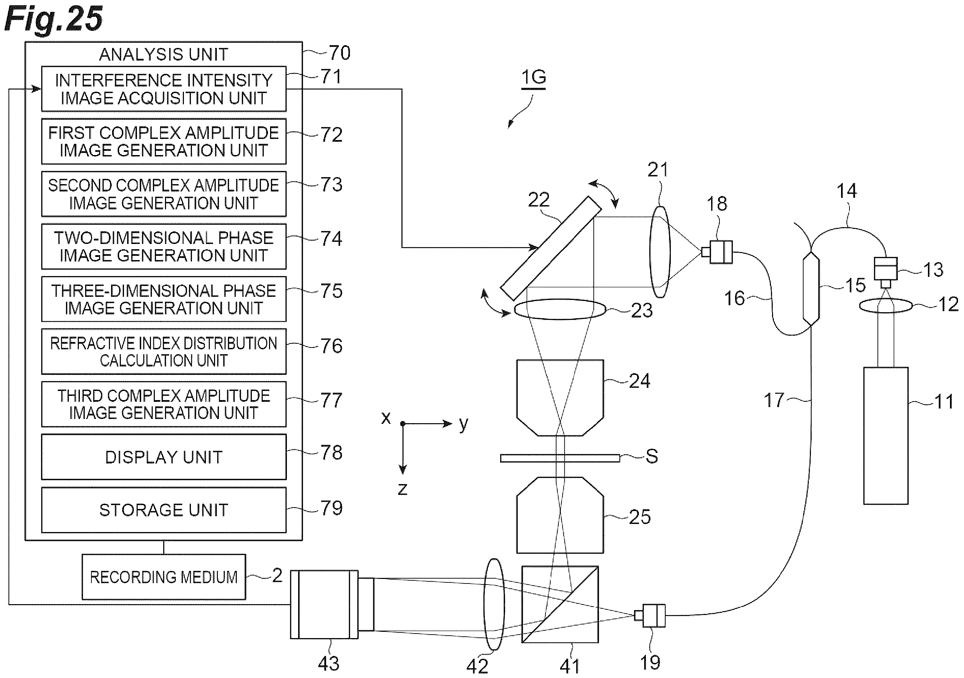

[FIG. 25] FIG. 25 (hereinafter also referred to as "FIG. C0 1 ") is a diagram illustrating a configuration of an observation apparatus 1G.

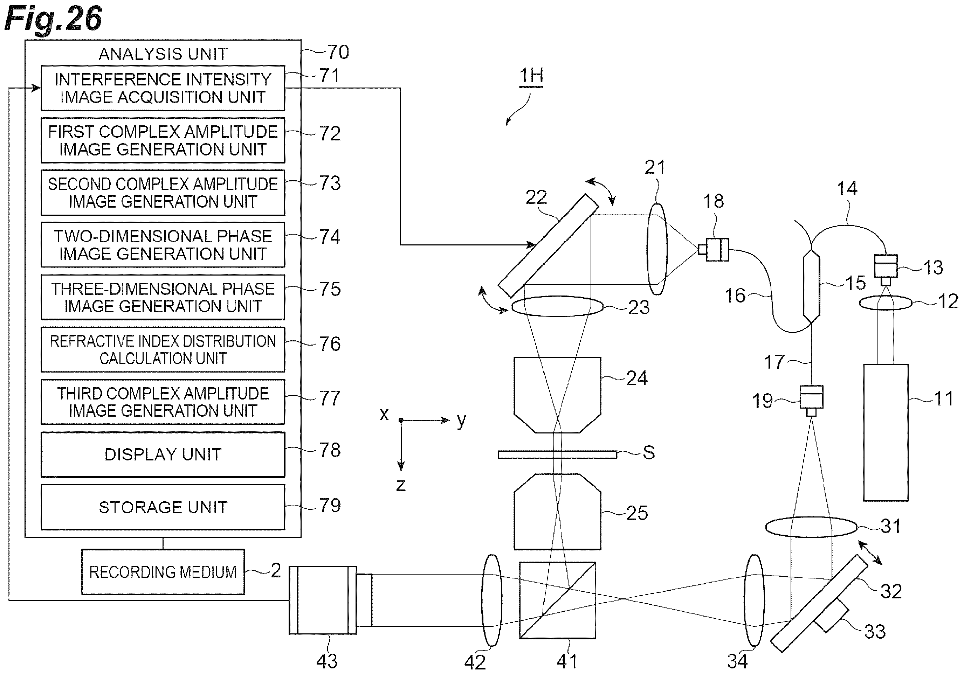

[FIG. 26] FIG. 26 (hereinafter also referred to as "FIG. C02") is a diagram illustrating a configuration of an observation apparatus 1H.

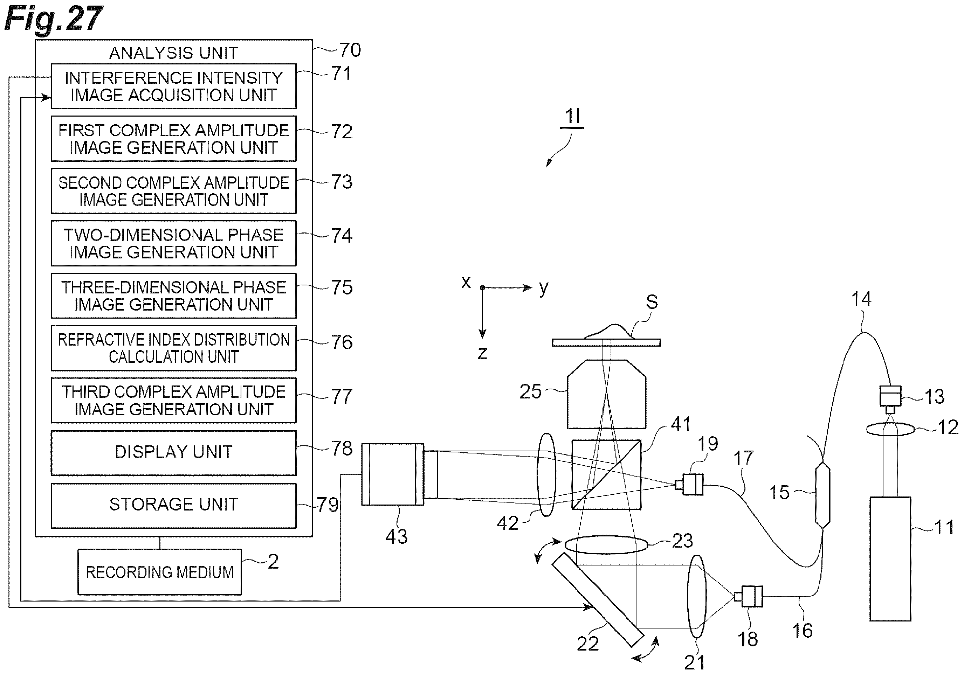

[FIG. 27] FIG. 27 (hereinafter also referred to as "FIG. C03") is a diagram illustrating a configuration of an observation apparatus 1I.

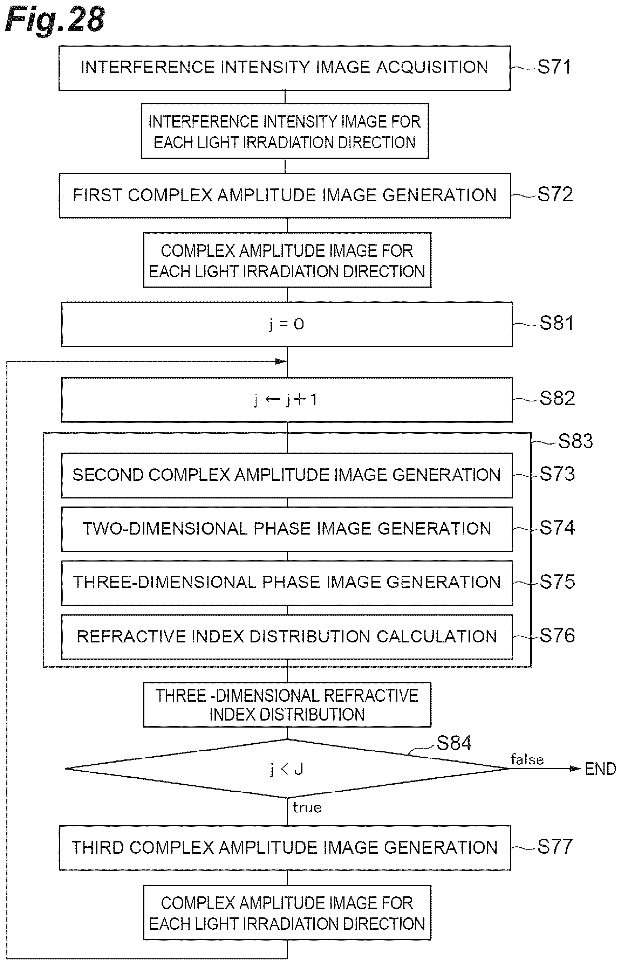

[FIG. 28] FIG. 28 (hereinafter also referred to as "FIG. C04") is a flowchart of a refractive index distribution measuring method C.

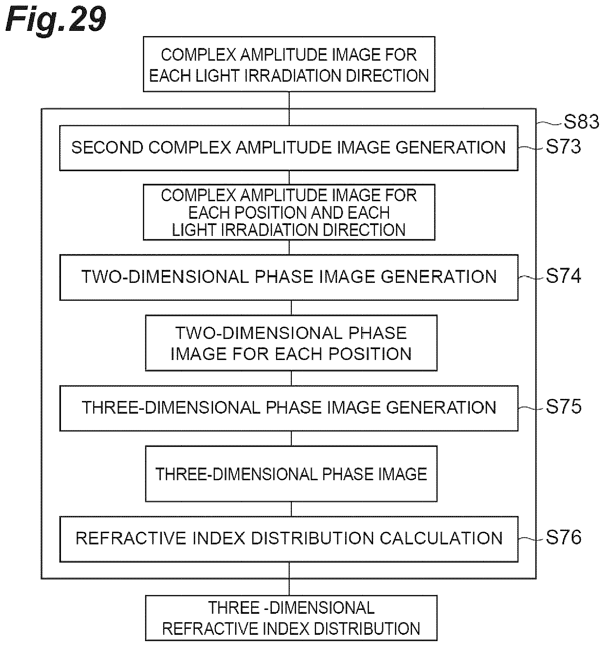

[FIG. 29] FIG. 29 (hereinafter also referred to as "FIG. C05") is a flowchart of a refractive index distribution measuring method C.

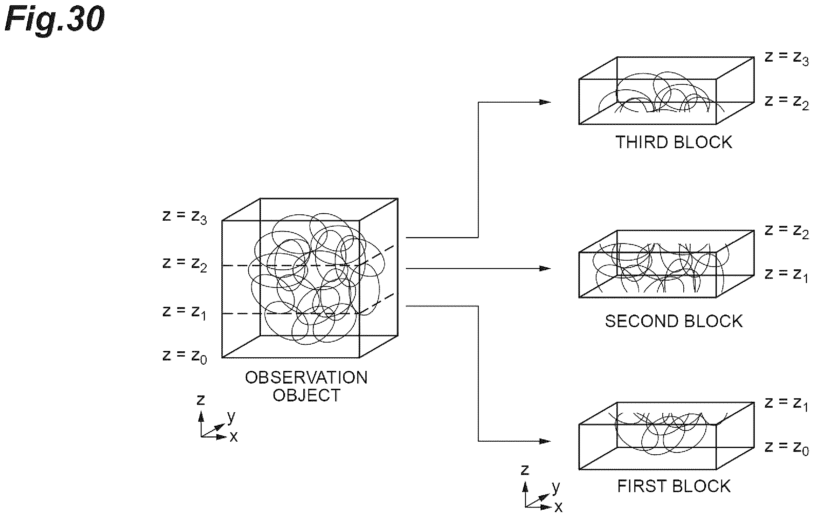

[FIG. 30] FIG. 30 (hereinafter also referred to as "FIG. C06") is a diagram illustrating a relationship between a region including an observation object and first to J-th blocks.

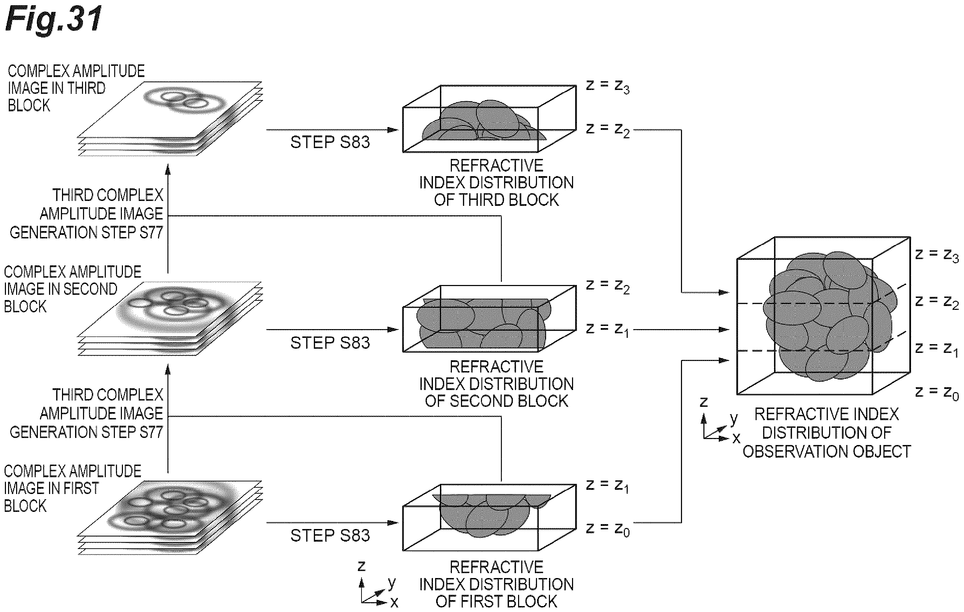

[FIG. 31] FIG. 31 (hereinafter also referred to as "FIG. C07") is a diagram illustrating a processing procedure for the first to J-th blocks.

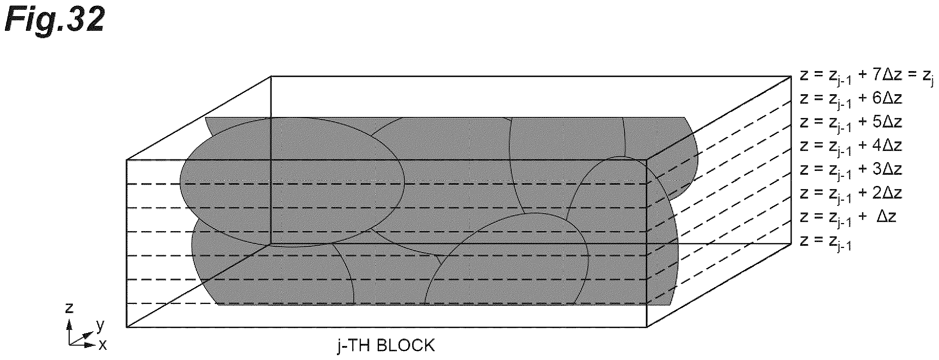

[FIG. 32] FIG. 32 (hereinafter also referred to as "FIG. C08") is a diagram illustrating processing contents of a BPM.

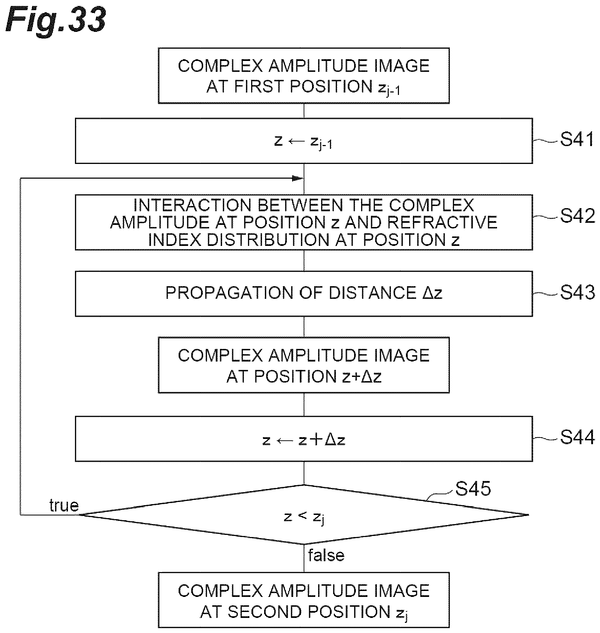

[FIG. 33] FIG. 33 (hereinafter also referred to as "FIG. C09") is a flowchart of a third complex amplitude image generation step S77.

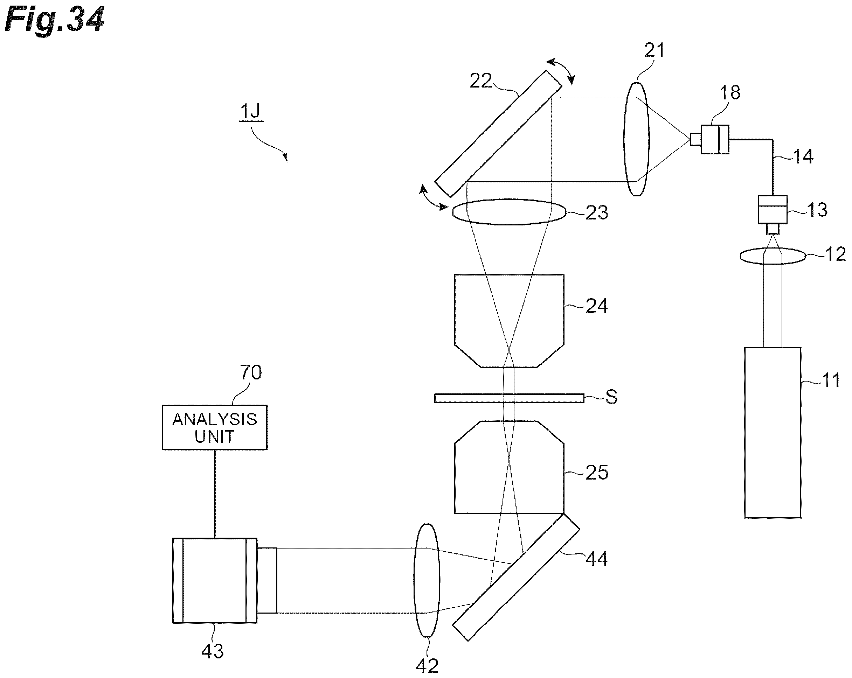

[FIG. 34] FIG. 34 (hereinafter also referred to as " FIG. C10") is a diagram illustrating a configuration of an observation apparatus 1J.

[FIG. 35] FIG. 35 is a diagram illustrating an outline of Embodiment T1.

[FIG. 36] FIG. 36 is a diagram illustrating an outline of Embodiment T2.

[FIG. 37] FIG. 37 is a diagram showing equidistant lines from Periphery P or an arbitrary plane Q in the embodiment T2.

[FIG. 38] FIG. 38 is a diagram illustrating an outline of Embodiment T3.

[FIG. 39] FIG. 39 is a diagram illustrating an outline of Embodiment T4.

[FIG. 40] FIG. 40 is a diagram illustrating an example of evaluations based on distances from the center O or the periphery P in Embodiment T4.

[FIG. 41] FIG. 41 is a graph showing the results of evaluating the refractive index distribution data in Example 1 and Comparative Example 1.

[FIG. 42] FIG. 42 is a graph showing the results of evaluating the refractive index distribution data in Example 2.

[FIG. 43] FIG. 43 is a graph showing the results of evaluating the refractive index distribution data in Example 3.
[FIG. 44] FIG. 44 is a graph showing the diameter and volume of a cell aggregate to be evaluated in Example 3.
[FIG. 45] FIG. 45 is a graph showing the correlation between the concentration ($\mu$M) of oleic acid and the percentage (%) of lipid droplet region.

**Description of Embodiments**

Hereinafter, embodiments of the present invention will be described in detail with reference to the accompanying drawings. In the description of the drawings, the same elements will be denoted by the same reference signs, and redundant description will be omitted. The present invention is not limited to these examples.

One aspect of the present invention is a method for evaluating a refractive index distribution including: a data acquisition step of acquiring refractive index distribution data of an observation object; a selection step of selecting whether or not an object included in the refractive index distribution data is an evaluation object; and an evaluation step of evaluating the refractive index distribution of the evaluation object.

Another aspect of the present invention is an apparatus for evaluating a refractive index distribution including: a data acquisition unit for acquiring refractive index distribution data of an observation object; a selection unit for obtaining an indicator for a object included in the refractive index distribution data from the refractive index distribution data and selecting whether or not the object is an evaluation object based on the indicator; and an evaluating unit for evaluating the refractive index distribution of the evaluation object.

Another aspect of the present invention is an information processing program for causing a computer to execute steps: a data acquisition step of acquiring refractive index distribution data of an observation object; a selection step of obtaining an indicator for an object included in the refractive index distribution data from the refractive index distribution data and selecting whether or not the object is an evaluation object based on the indicator; and an evaluation step of evaluating the refractive index distribution of the evaluation object.

The observation object in the present invention may include a three-dimensional culture, a two-dimensional culture, a multicellular organism, a unicellular organism, an embryo, a tissue piece, a clinical specimen, and the like. The observation object may be a three-dimensional culture. Where the observation object is a three-dimensional culture, the maximum diameter thereof is preferably 100 to 200 $\mu$m. Where the observation object is a cell culture, the culture may be composed of one type of cells or may contain two or more types of cells. The refractive index distribution data in the present invention may contain at least one refractive index data selected from the group consisting of a lipid droplet, a mitochondrion, a vesicle including a vacuole, a nucleolus and DNA.

A method for acquiring the refractive index distribution data of an observation object (hereinafter also referred to as "refractive index distribution measuring method") is described. Optical Diffraction Tomography (ODT) is known as a method for measuring the refractive index distribution of an observation object in a non-staining and non-invasive manner. ODT is a technique capable of three-dimensional imaging developed from Quantitative Phase Imaging (QPI), and can realize three-dimensional refractive index tomography of an observation object. By performing cell observation using ODT, it is possible to identify organelles such as a lipid droplet, a mitochondrion, a vesicle including a vacuole, and a nucleolus, and it is also possible to trace three-dimensional morphological changes, and it is expected that analysis with a higher content than QPI can be performed.

More preferably, refractive index distribution measuring methods A to C described below are used. The refractive index distribution measuring method A includes the modes of the refractive index distribution measuring methods A1 to A3. A1 to A3 are collectively referred to as a refractive index distribution measuring method A. These refractive index distribution measuring methods A to C can realize three-dimensional refractive index tomography in which the influence of multiple scattered light is reduced even when the observation object is a multiple scattering object.

Optical Coherence Tomography (OCT) is also known as another staining and non-invasive imaging technique. However, the resolution of OCT is about 10 $\mu$m, whereas the resolution of ODT and refractive index distribution measuring methods A to C is about 1 $\mu$m. In addition, OCT does not obtain a refractive index distribution, and it is difficult to biologically interpret a signal obtained by imaging. In these respects, ODT and the refractive index distribution measuring methods A to C are superior to OCT.

First, the refractive index distribution measuring method A (A1 to A3) will be described. FIG. A01 to A03 are diagrams showing respective configurations of observation apparatuses 1A to 1C that can be used when measuring the refractive index distribution by the refractive index distribution measuring method A.

FIG. A01 is a diagram illustrating a configuration of an observation apparatus 1A. The observation apparatus 1A includes a light source 11, a lens 12, a lens 21, a mirror 22, a lens 23, a condenser lens 24, an objective lens 25, a beam splitter 41, a lens 42, an imaging unit 43, and an analysis unit 50.

The light source 11 outputs spatially and temporally coherent light, and is preferably a laser light source. The lens 12 is optically coupled to the light source 11, focuses the light output from the light source 11 on a light input end 13 of an optical fiber 14, and inputs the light to the light input end 13. The optical fiber 14 guides the light input to the light input end 13 by the lens 12 to a fiber coupler 15. The fiber coupler 15 couples the light between the optical fiber 14 and optical fibers 16 and 17, splits the light guided by and arriving from the optical fiber 14 into two light beams, guides one split light by the optical fiber 16, and guides the other split light by the optical fiber 17. The light guided by the optical fiber 16 is output as diverging light from a light output end 18. The light guided by the optical fiber 17 is output as diverging light from a light output end 19.

The lens 21 is optically coupled to the light output end 18, and collimates the light output as the diverging light from the light output end 18. The mirror 22 is optically coupled to the lens 21, and reflects the light arriving from the lens 21 to the lens 23. An orientation of a reflection surface of the mirror 22 is changeable. The lens 23 is optically coupled to the mirror 22. The condenser lens 24 is optically coupled to the lens 23. The lens 23 and the condenser lens 24 preferably constitute a 4f optical system. The lens 23 and the condenser lens 24 irradiate an observation object S with the light from a light irradiation direction according to the orientation of the reflection surface of the mirror 22. The objective lens 25 is optically coupled to the condenser lens 24. The observation object S is disposed between the objective lens 25 and the condenser lens 24. The objective lens 25 inputs the light (object light) output from the condenser lens 24 and passed through the observation object S, and outputs the light to the beam splitter 41.

The beam splitter 41 is optically coupled to the objective lens 25, and further, is optically coupled also to the light output end 19. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images an interference fringe image (interference intensity image) generated by interference between the object light and the reference light arriving from the lens 42. An incident direction of the reference light is inclined with respect to an incident direction of the object light on an imaging plane of the imaging unit 43. A position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

The analysis unit 50 is electrically connected to the imaging unit 43, and inputs the interference intensity image captured by the imaging unit 43. The analysis unit 50 calculates a three-dimensional refractive index distribution of the observation object S by processing the input interference intensity image. The analysis unit 50 may be a computer. The analysis unit 50 includes an interference intensity image acquisition unit 51, a first complex amplitude image generation unit 52, a second complex amplitude image generation unit 53, a two-dimensional phase image generation unit 54, a three-dimensional phase image generation unit 55, a refractive index distribution calculation unit 56, a display unit 57, and a storage unit 58.

The interference intensity image acquisition unit 51 irradiates the observation object S with the light along each of a plurality of light irradiation directions by changing the orientation of the reflection surface of the mirror 22. Further, the interference intensity image acquisition unit 51 acquires the interference intensity image at a reference position for each of the plurality of light irradiation directions from the imaging unit 43. The interference intensity image acquisition unit 51 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 52, the second complex amplitude image generation unit 53, the two-dimensional phase image generation unit 54, the three-dimensional phase image generation unit 55, and the refractive index distribution calculation unit 56 perform processing based on the interference intensity images, and include a processing device such as a CPU, a GPU, a DSP, or an FPGA. The display unit 57 displays an image to be processed, an image in the middle of the processing, an image after the processing, and the like, and includes, for example, a liquid crystal display. The storage unit 58 stores data of various images, and includes a hard disk drive, a flash memory, a RAM, a ROM, and the like. The first complex amplitude image generation unit 52, the second complex amplitude image generation unit 53, the two-dimensional phase image generation unit 54, the three-dimensional phase image generation unit 55, the refractive index distribution calculation unit 56, and the storage unit 58 may be constituted by a cloud computing.

The storage unit 58 also stores a program for causing the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56, to execute respective steps of the processing. The program may be stored in the storage unit 58 at the time of manufacture or shipment of the observation apparatus 1A, may be acquired via a communication line after shipment and then stored in the storage unit 58, or may be recorded in a computer readable recording medium 2 and then stored in the storage unit 58. The recording medium 2 may be an arbitrary medium such as a flexible disk, a CD-ROM, a DVD-ROM, a BD-ROM, a USB memory, or the like.

The details of the processing step of each of the interference intensity image acquisition unit 51, the first complex amplitude image generation unit 52, the second complex amplitude image generation unit 53, the two-dimensional phase image generation unit 54, the three-dimensional phase image generation unit 55, and the refractive index distribution calculation unit 56 will be described later.

FIG. A02 is a diagram illustrating a configuration of an observation apparatus 1B. The observation apparatus 1B illustrated in FIG. A02 includes a lens 31, a mirror 32, and a lens 34 in addition to the configuration of the observation apparatus 1A illustrated in FIG. A01.

The lens 31 is optically coupled to the light output end 19, and collimates the light (reference light) output as diverging light from the light output end 19. The mirror 32 is optically coupled to the lens 31, and reflects the light arriving from the lens 31 to the lens 34. The lens 34 is optically coupled to the mirror 32, and outputs the light arriving from the mirror 32 to the beam splitter 41. The light output from the lens 34 is once focused before the beam splitter 41, and then input to the beam splitter 41 as diverging light. The beam splitter 41 combines the light (object light) output and arriving from the objective lens 25 and the light (reference light) output and arriving from the lens 34, and outputs the light to the lens 42 in a coaxial manner. The imaging unit 43 images the interference fringe image (interference intensity image) generated by interference between the object light and the reference light arriving from the lens 42. The incident direction of the reference light is parallel to the incident direction of the object light on the imaging plane of the imaging unit 43.

A drive unit 33 moves the mirror 32 in a direction perpendicular to a reflection surface of the mirror 32. The drive unit 33 is, for example, a piezoelectric actuator. The movement of the mirror 32 changes an optical path difference (phase difference) of the object light and the reference light from light splitting by the fiber coupler 15 to combining by the beam splitter 41. When the optical path difference is different, the interference intensity image captured by the imaging unit 43 is also different.

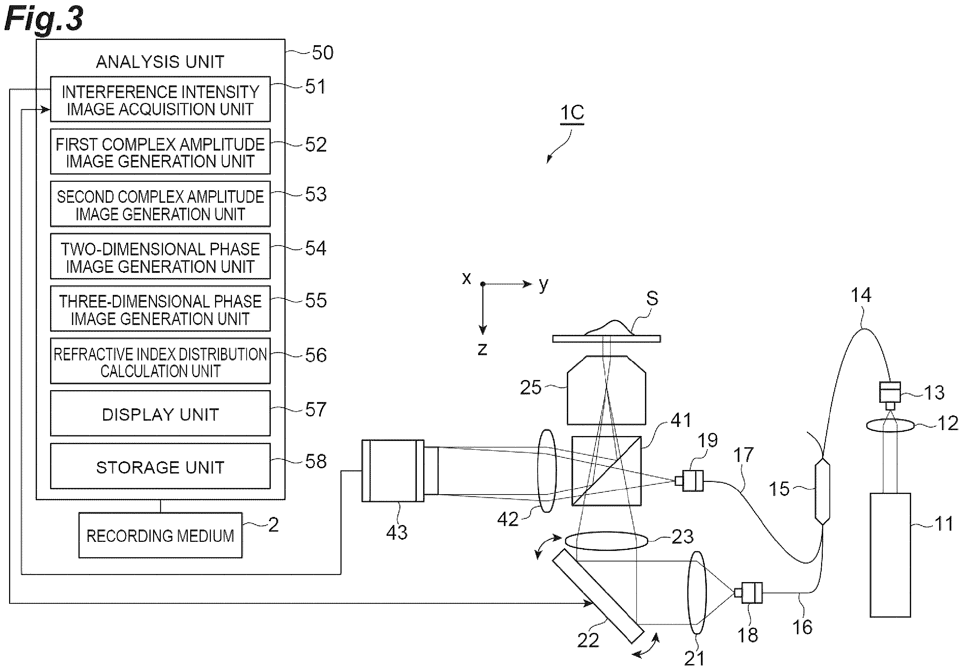

The observation apparatus is not limited to the configuration examples illustrated in FIG. A01 and FIG. A02, and various modifications are possible. In the configuration of the observation apparatus 1A (FIG. A01) and the observation apparatus 1B (FIG. A02), the object light transmitted through the observation object S is observed, and the object light reflected by the observation object S may be observed as in a configuration of an observation apparatus 1C (FIG. 3) described below.

FIG. A03 is a diagram illustrating a configuration of an observation apparatus 1C. The observation apparatus 1C includes the light source 11, the lens 12, the lens 21, the mirror 22, the lens 23, the objective lens 25, the beam splitter 41, the lens 42, the imaging unit 43, and the analysis unit 50. Hereinafter, differences from the observation apparatus 1A (FIG. A01) will be mainly described.

The lens 21 is optically coupled to the light output end 18 of the optical fiber 16, and collimates the light output as diverging light from the light output end 18. The mirror 22 is optically coupled to the lens 21, and reflects the light arriving from the lens 21 to the lens 23. The orientation of the reflection surface of the mirror 22 is changeable. The lens 23 is optically coupled to the mirror 22. The objective lens 25 is optically coupled to the lens 23. The beam splitter 41 is disposed between the lens 23 and the objective lens 25. The lens 23 and the objective lens 25 preferably constitute a 4f optical system. The lens 23 and the objective lens 25 irradiate the observation object S with the light from the light irradiation direction according to the orientation of the reflection surface of the mirror 22. The objective lens 25 inputs the light (object light) reflected from the observation object S, and outputs the light to the beam splitter 41.

The beam splitter 41 is optically coupled to the objective lens 25, and further, is optically coupled also to the light output end 19 of the optical fiber 17. The beam splitter 41 combines the light (object light) output and arriving from the objective lens 25 and the light (reference light) output and arriving from the light output end 19, and outputs the light to the lens 42. The lens 42 is optically coupled to the beam splitter 41, collimates the object light and the reference light arriving from the beam splitter 41, and outputs the light to the imaging unit 43. The imaging unit 43 is optically coupled to the lens 42, and images the interference fringe image (interference intensity image) generated by interference between the object light and the reference light arriving from the lens 42. The incident direction of the reference light is inclined with respect to the incident direction of the object light on the imaging plane of the imaging unit 43. The position at which the object light and the reference light are combined by the beam splitter 41 may be in the subsequent stage of the imaging lens, and in addition, in consideration of the influence of aberration, it is desirable that the position is set between the objective lens 25 and the lens 42 as illustrated in the diagram.

In the configuration of the observation apparatus 1C (FIG. A03), as in the observation apparatus 1B (FIG. A02), the mechanism (the lens 31, the mirror 32, the drive unit 33, and the lens 34 in FIG. A02) for changing the optical path length of the reference light may be provided for changing the optical path difference (phase difference) of the object light and the reference light from light splitting by the fiber coupler 15 to combining by the beam splitter 41. In this case, the incident direction of the reference light may be parallel to the incident direction of the object light on the imaging plane of the imaging unit 43.

FIG. A04 is a flowchart of a refractive index distribution measuring method A. The refractive index distribution measuring method A can be applied to each of the observation apparatus 1A to 1C. The refractive index distribution measuring method A includes an interference intensity image acquisition step S1, a first complex amplitude image generation step S2, a second complex amplitude image generation step S3, a two-dimensional phase image generation step S4, a three-dimensional phase image generation step S5, and a refractive index distribution calculation step S6.

The processing step of the interference intensity image acquisition step S1 is performed by the interference intensity image acquisition unit 51. The processing step of the first complex amplitude image generation step S2 is performed by the first complex amplitude image generation unit 52. The processing step of the second complex amplitude image generation step S3 is performed by the second complex amplitude image generation unit 53. The processing step of the two-dimensional phase image generation step S4 is performed by the two-dimensional phase image generation unit 54. The processing step of the three-dimensional phase image generation step S5 is performed by the three-dimensional phase image generation unit 55. The processing step of the refractive index distribution calculation step S6 is performed by the refractive index distribution calculation unit 56.

In the interference intensity image acquisition step S1, the interference intensity image acquisition unit 51 irradiates the observation object S with the light along each of the plurality of light irradiation directions by changing the orientation of the reflection surface of the mirror 22. Further, the interference intensity image acquisition unit 51 acquires the interference intensity image at the reference position for each of the plurality of light irradiation directions from the imaging unit 43.

In each of FIG. A01 to FIG. A03, an xyz orthogonal coordinate system is illustrated for convenience of explanation. The z axis is parallel to the optical axis of the objective lens 25. The reference position is the image plane position having a conjugate relationship with respect to the imaging plane of the imaging unit 43. This position is set to z = 0. The light irradiation direction on the observation object S can be represented by $k_x$ and $k_y$ in a wavenumber vector ($k_x$, $k_y$, $k_z$) of the irradiation light.

(a) to (c) in FIG. A05 are diagrams illustrating examples of scanning of the light irradiation direction on the observation object S in the interference intensity image acquisition step S1. In the diagram, a position of each circular point represents the light irradiation direction in the $k_x k_y$ plane in which the horizontal axis is set to $k_x$ and the vertical axis is set to $k_y$. The scanning of the light irradiation direction may be arranged in a rectangular lattice shape in the $k_x k_y$ plane as illustrated in (a) in FIG. A05, may be arranged on a circumference of each of a plurality of concentric circles in the $k_x k_y$ plane as illustrated in (b) in FIG. A05, or may be arranged in a spiral shape in the $k_x k_y$ plane as illustrated in (c) in FIG. A05. In any of the cases, the light irradiation direction can be scanned as far as it is allowed by Numerical Aperture (NA) of the condenser lens 24. Raster scan or random scan may be used. In the case of the raster scan, return scan may be performed or may not be performed.

In the first complex amplitude image generation step S2, the first complex amplitude image generation unit 52 generates, for each of the plurality of light irradiation directions, a complex amplitude image based on the interference intensity image acquired by the interference intensity image acquisition unit 51. In the case of the observation apparatus 1A (FIG. A01) or the observation apparatus 1C (FIG. A03), the first complex amplitude image generation unit 52 can generate the complex amplitude image based on one interference intensity image by a Fourier fringe analysis method. In the case of the observation apparatus 1B (FIG. A02), the first complex amplitude image generation unit 52 can generate the complex amplitude image based on three or more interference intensity images having different optical path differences (phase differences) between the object light and the reference light by a phase shift method.

In the second complex amplitude image generation step S3, the second complex amplitude image generation unit 53 generates, for each of the plurality of light irradiation directions, a complex amplitude image at each of a plurality of z direction positions based on the complex amplitude image at a reference position (z = 0) generated by the first complex amplitude image generation unit 52. Assuming that a two-dimensional Fourier transform of the complex amplitude image u(x, y, 0) at the reference position is U($k_x$, $k_y$, 0), the complex amplitude image u(x, y, d) at the position of z = d and the two-dimensional Fourier transform U($k_x$, $k_y$, d) of the complex amplitude image u(x, y, d) are represented by the following Formulas. i is an imaginary unit, and $k_0$ is a wavenumber of the light in the observation object.

[Formula 1]

$$U(k_x, k_y, d) = U(k_x, k_y, 0) \exp\left(i\sqrt{k_0^2 - k_x^2 - k_y^2}\, d\right) \qquad (1)$$

[Formula 2]

$$u(x, y, d) = \int U(k_x, k_y, d) \exp(-ik_x x - ik_y y)\, dk_x dk_y \qquad (2)$$

In the two-dimensional phase image generation step S4, the two-dimensional phase image generation unit 54 generates, for each of the plurality of positions, a two-dimensional phase image based on the complex amplitude image of each of the plurality of light irradiation directions generated by the second complex amplitude image generation unit 53. The two-dimensional phase image generated in this step corresponds to a phase image centered on the focused z direction position. The details of the two-dimensional phase image generation step S4 will be described below.

In addition, the two-dimensional phase image generation step S4 and the subsequent steps may be performed after all the complex amplitude images at the plurality of positions are generated for each of the plurality of light irradiation directions in the second complex amplitude image generation step S3. Further, processes of generating the complex amplitude image at one certain z direction position for each of the plurality of light irradiation directions in the second complex amplitude image generation step S3 and generating the two-dimensional phase image at the position in the two-dimensional phase image generation step S4 may be set as a unit, and the unit process may be repeatedly performed while scanning the z direction position. The latter case is preferable in that a capacity of image data to be stored in the storage unit 58 can be reduced.

In the three-dimensional phase image generation step S5, the three-dimensional phase image generation unit 55 generates a three-dimensional phase image based on the two-dimensional phase image at each of the plurality of positions generated by the two-dimensional phase image generation unit 54. The three-dimensional phase image generated in this step is an image in which the positions x and y in the two-dimensional phase image and the position z of the two-dimensional phase image are variables.

In the refractive index distribution calculation step S6, the refractive index distribution calculation unit 56 obtains a three-dimensional refractive index distribution of the observation object by deconvolution based on the three-dimensional phase image generated by the three-dimensional phase image generation unit 55. Assuming that the refractive index distribution of the observation object is $n(x, y, z)$, an electric susceptibility distribution is $f(x, y, z)$, and a refractive index of a background medium is $n_m$, there is a relationship of the following Formula (3) between them. The three-dimensional phase image $\Phi(x, y, z)$ generated by the three-dimensional phase image generation unit 55 is represented by convolution of a kernel function $g(x, y, z)$ and the electric susceptibility distribution $f(x, y, z)$ as shown in the following Formula (4). Therefore, the three-dimensional refractive index distribution $n(x, y, z)$ of the observation object can be obtained by deconvolution based on the three-dimensional phase image $\Phi(x, y, z)$.

[Formula 3]

$$f(x,y,z) = k_0^2 \left[ \left( n(x,y,z)/n_m \right)^2 - 1 \right] \qquad (3)$$

[Formula 4]

$$\Phi(x,y,z) = \int g(x-x',y-y',z-z')f(x',y',z')dx'dy'dz' \qquad (4)$$

In addition, the kernel function g is a function based on a Green function corresponding to a solution of a wave equation. FIG. A06 is a diagram showing the kernel function g. In this diagram, a center position having the largest value of the kernel function g is the origin, the vertical direction is the z axis, and the horizontal direction is the direction perpendicular to the z axis.

Each of the processing steps of the first complex amplitude image generation step S2, the second complex amplitude image generation step S3, the two-dimensional phase image generation step S4, the three-dimensional phase image generation step S5, and the refractive index distribution calculation step S6 may be performed each time the interference intensity image of each of a predetermined number of light irradiation directions is acquired in the interference intensity image acquisition step S1 (FIG. A07), or may be performed each time the interference intensity image of one light irradiation direction is acquired in the interference intensity image acquisition step S1 (FIG. A08).

FIG. A07 and FIG. A08 are diagrams illustrating examples of scanning of the light irradiation direction on the observation object S in the interference intensity image acquisition step S1. In these diagrams, a position of each circular point represents the light irradiation direction in the $k_x k_y$ plane in which the horizontal axis is set to $k_x$ and the vertical axis is set to $k_y$. In the examples of scanning of the light irradiation direction illustrated in these diagrams, the light irradiation direction is sequentially changed, and the light irradiation direction at the time of acquisition of the (N+n)-th interference intensity image is made to coincide with the light irradiation direction at the time of acquisition of the n-th interference intensity image. n is a positive integer, and N is an integer of 2 or more.

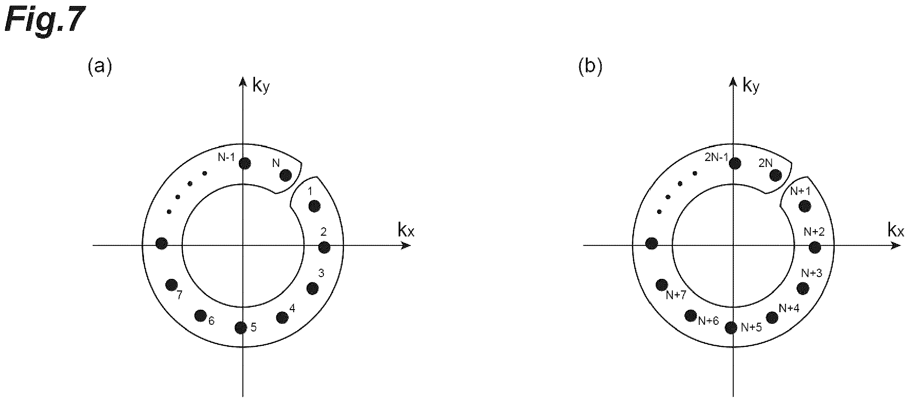

In the example illustrated in FIG. A07, when the first to N-th interference intensity images are acquired in the interference intensity image acquisition step S61, the respective processing steps of the steps S2 to S6 are performed based on the first to N-th interference intensity images ((a) in FIG. 7). Next, when the (N+1)-th to 2N-th interference intensity images are acquired in the interference intensity image acquisition step S1, the respective processing steps of the steps S2 to S6 are performed based on the (N+1)-th to 2N-th interference intensity images ((b) in FIG. A07). Next, when the (2N+1)-th to 3N-th interference intensity images are acquired in the interference intensity image acquisition step S1, the respective processing steps of the steps S2 to S6 are performed based on the (2N+1)-th to 3N-th interference intensity images. The same applies thereafter.

In the example illustrated in FIG. A08, when the first to N-th interference intensity images are acquired in the interference intensity image acquisition step S1, the respective processing steps of the steps S2 to S6 are performed based on the first to N-th interference intensity images ((a) in FIG. A08). Next, when the (N+1)-th interference intensity image is acquired in the interference intensity image acquisition step S61, the respective processing steps of the steps S2 to S6 are performed based on the latest N interference intensity images (the second to (N+1)-th interference intensity images) including the (N+1)-th interference intensity image ((b) in FIG. A08). Next, when the (N+2)-th interference intensity image is acquired in the interference intensity image acquisition step S1, the respective processing steps of the steps S2 to S6 are performed based on the latest N interference intensity images (the third to (N+2)-th interference intensity images) including the (N+2)-th interference intensity image ((c) in FIG. A08). The same applies thereafter, and when the (N+n)-th interference intensity image is acquired in the interference intensity image acquisition step S1, the respective processing steps of the steps S2 to S6 are performed based on the latest N interference intensity images (the (1+n)-th to (N+n)-th interference intensity images) including the (N+n)-th interference intensity image.

Compared with the example illustrated in FIG. A07, in the example illustrated in FIG. A08, each time the interference intensity image of one light irradiation direction is acquired in the interference intensity image acquisition step S1, the respective processing steps of the steps S2 to S6 are performed based on the plurality of latest interference intensity images including the acquired interference intensity image, and thus, the number of images obtained per unit time by the respective processing steps of the steps S2 to S6 is large.

Next, the details of the two-dimensional phase image generation step S4 in the refractive index distribution measuring method A will be described. In the two-dimensional phase image generation step S4, the two-dimensional phase image generation unit 54 generates, for each of the plurality of positions, the two-dimensional phase image based on the complex amplitude image of each of the plurality of light irradiation directions generated by the second complex amplitude image generation unit 53. The two-dimensional phase image generation step S4 depends on the refractive index distribution measuring method A1 to A3.

FIG. A09 is a flowchart of the two-dimensional phase image generation step S4 in the refractive index distribution measuring method A1. In the refractive index distribution measuring method A1, for each of the plurality of positions, the two-dimensional phase image generation step S4, in a step S11, corrects the phase of the complex amplitude image of each of the plurality of light irradiation directions based on the light irradiation direction, and then generates a complex amplitude summation image representing a summation of the complex amplitude images after the correction, and in a step S12, generates the two-dimensional phase image based on the complex amplitude summation image.

The processing of the step S11 is based on a CASS (Collective Accumulation of Single Scattering; Sungsam Kang, et al, "Imaging deep within a scattering medium using collective accumulation of single-scattered waves," NATURE PHOTONICS, Vol.9, pp.253-258 (2015)) technique. In the light with which the object is irradiated along a certain light irradiation direction and passed through the object, a spatial frequency distribution of the single scattered light which interacts with the object only once is shifted according to the light irradiation direction, whereas a spatial frequency distribution of the multiple scattered light which interacts with the object a plurality of times randomly changes according to the light irradiation direction. The CASS technique uses the above difference between the light irradiation direction dependencies of the spatial frequency distributions of the single scattered light and the multiple scattered light.

That is, in the step S11, the phase of the complex amplitude image of each of the plurality of light irradiation directions is corrected based on the light irradiation direction (that is, the spatial frequency distribution of the complex amplitude image is shifted in parallel according to the light irradiation direction in the spatial frequency domain), so that the spatial frequency distribution of the single scattered light component in the complex amplitude image has a shape and arrangement independent of the light irradiation direction, while the spatial frequency distribution of the multiple scattered light component in the complex amplitude image has a random shape and arrangement. Further, in the step S11, the complex amplitude summation image representing the summation of the plurality of complex amplitude images after the above correction is generated (that is, synthetic aperture processing is performed) to coherently sum the single scattered light components in the complex amplitude images, while the multiple scattered light components in the complex amplitude images cancel each other out.

Therefore, the influence of the multiple scattered light is reduced in the complex amplitude summation image generated in the step S11. Further, the three-dimensional refractive index distribution obtained finally in the refractive index distribution calculation step S6 also reduces the influence of the multiple scattered light, suppresses the speckles, and improves the Single-scattering to Multi-scattering Ratio (SMR).

FIG. A10 is a flowchart of the two-dimensional phase image generation step S4 in the refractive index distribution measuring method A2. In the refractive index distribution measuring method A2, for each of the plurality of positions, the two-dimensional phase image generation step S4, in a step S21, generates a complex differential interference image of each of the plurality of light irradiation directions based on the complex amplitude image of each of the plurality of light irradiation directions. In a step S22, the step generates a phase differential image based on a summation of the complex differential interference images of the plurality of light irradiation directions. In a step S23, the step generates the two-dimensional phase image based on the phase differential image.

Assuming that the complex amplitude image at the position of z = d is u(x, y, d), the complex differential interference image q(x, y, d) generated in the step S21 is represented by the following Formula (5). At least one of $\delta x$ and $\delta y$ is non-zero. When $\delta x \neq 0$ and $\delta y = 0$, the complex differential interference image q in which the x direction is a shear direction is obtained. When $\delta x = 0$ and $\delta y \neq 0$, the complex differential interference image q in which the y direction is the shear direction is obtained. When $\delta x \neq 0$ and $\delta y \neq 0$, the complex differential interference image q with the shear direction different from both of the x direction and the y direction is obtained. In addition, the complex differential interference image q(x, y, d) may be obtained by Formula (5) after transforming the complex amplitude image u(x, y, d) as in the following Formula (6).

[Formula 5]

$$q(x,y,d) = u^*(x+\delta x, y+\delta y, d) \cdot u(x,y,d) \qquad (5)$$

[Formula 6]

$$u(x,y,d)\exp(-ik_x x - ik_y y) \qquad (6)$$

Assuming that the summation of the complex differential interference images q of the plurality of light irradiation directions is $q_{sum}(x, y, d)$, the phase differential image $\phi(x, y, z)$ generated in the step S22 is represented by the following Formula (7) as the phase of $q_{sum}(x, y, d)$. In the step S23, the two-dimensional phase image can be generated by performing integration or deconvolution of the phase differential image $\phi(x, y, z)$.

[Formula 7]

$$\phi(x,y,d) = \angle q_{sum}(x,y,d) \qquad (7)$$

In addition, in the step S21, the complex differential interference image may be generated for each of a plurality of shear directions different from each other on the complex amplitude image. In this case, for each of the plurality of positions, the two-dimensional phase image generation step S4, in the step S21, generates the complex differential interference image of each of the plurality of light irradiation directions for each of the plurality of shear directions on the image different from each other based on the complex amplitude image of each of the plurality of light irradiation directions. In the step S22, the step generates the phase differential image based on the summation of the complex differential interference images of the plurality of light irradiation directions for each of the plurality of shear directions. In the step S23, the step generates the two-dimensional phase image based on the phase differential image of each of the plurality of shear directions.

The influence of the multiple scattered light is reduced in the phase differential image generated based on the summation of the complex differential interference image of each of the plurality of light irradiation directions in the step S22. Further, the three-dimensional refractive index distribution obtained finally in the refractive index distribution calculation step S6 also reduces the influence of the multiple scattered light, and suppresses the speckles. Further, when the complex differential interference image is generated for each of the plurality of shear directions different from each other on the complex amplitude image in the step S21, it is possible to suppress the appearance of linear noises in the two-dimensional phase image obtained in the step S23.

In the above description, the case in which the two-dimensional phase image is generated by performing integration or deconvolution of the phase differential image in the step S23 is described. However, the phase differential image may also be treated as the two-dimensional phase image. In this case, the three-dimensional refractive index distribution of the observation object can be obtained from the phase differential image (two-dimensional phase image) generated in the step S22 by using a kernel (FIG. A11) including a kernel used in deconvolution of the step S23, in deconvolution of the refractive index distribution calculation step S67, without performing the step S23. The kernel shown in FIG. A11 is obtained by convolution integration of the kernel shown in FIG. A06 and the kernel used in deconvolution of the step S23.

FIG. A12 is a flowchart of the two-dimensional phase image generation step S4 in the refractive index distribution measuring method A3. In the refractive index distribution measuring method A3, for each of the plurality of positions, the two-dimensional phase image generation step S4, in a step S31, divides the complex amplitude image of each of the plurality of light irradiation directions into a plurality of batches, corrects the phase of the complex amplitude image included in the batch based on the light irradiation direction for each of the plurality of batches, and then generates the complex amplitude summation image representing the summation of the complex amplitude images after the correction, in a step S32, generates the complex differential interference image of each of the plurality of batches based on the complex amplitude summation image of each of the plurality of batches, in a step S33, generates the phase differential image based on the summation of the complex differential interference images of the plurality of batches, and in a step S34, generates the two-dimensional phase image based on the phase differential image.

The processing of the step S31 in the refractive index distribution measuring method A3 corresponds to dividing the complex amplitude image of each of the plurality of light irradiation directions into the plurality of batches, and then performing the processing of the step S11 in the refractive index distribution measuring method A1 for each of the plurality of batches. The processing of the steps S32 and S33 in the refractive index distribution measuring method A3 corresponds to performing the processing of the steps S21 and S22 in the refractive index distribution measuring method A2 for each of the plurality of batches. The processing of the step S34 in the refractive index distribution measuring method A3 corresponds to performing the processing of the step S23 in the refractive index distribution measuring method A2.

In addition, in the step S32, the complex differential interference image may be generated for each of the plurality of shear directions different from each other on the complex amplitude image. In this case, the two-dimensional phase image generation step S4, in the step S32, generates the complex differential interference image of each of the plurality of batches for each of the plurality of shear directions on the image different from each other based on the complex amplitude summation image of each of the plurality of batches, in the step S33, generates the phase differential image based on the summation of the complex differential interference images of the plurality of batches for each of the plurality of shear directions, and in the step S34, generates the two-dimensional phase image based on the phase differential image of each of the plurality of shear directions.

The suppression of the speckles in the refractive index distribution measuring method A3 is comparable with the refractive index distribution measuring method A1 and the refractive index distribution measuring method A2. The improvement of the SMR in the refractive index distribution measuring method A3 is an intermediate degree between the refractive index distribution measuring method A1 and the refractive index distribution measuring method A2.

In the above description also, the case in which the two-dimensional phase image is generated by performing integration or deconvolution of the phase differential image in the step S34 is described. However, the phase differential image may also be treated as the two-dimensional phase image. In this case, the three-dimensional refractive index distribution of the observation object can be obtained from the phase differential image (two-dimensional phase image) generated in the step S33 by using the kernel including the kernel used in deconvolution of the step S34, in deconvolution of the refractive index distribution calculation step S6, without performing the step S34.

Next, the refractive index distribution measuring method B will be will be described. FIG. B01 to B03 are diagrams showing respective configurations of observation apparatuses 1D to 1F that can be used when measuring the refractive index distribution by the refractive index distribution measuring method B. The observation apparatus 1D illustrated in FIG. B01, as compared with the configuration of the observation apparatus 1A illustrated in FIG. A01, has the common configuration for the optical system from the light source 11 to the imaging unit 43, and further, is different in that an analysis unit 60 is provided instead of the analysis unit 50. The observation apparatus 1E illustrated in FIG. B02, as compared with the configuration of the observation apparatus 1B illustrated in FIG. A02, has the common configuration for the optical system from the light source 11 to the imaging unit 43, and further, is different in that the analysis unit 60 is provided instead of the analysis unit 50. The observation apparatus 1F illustrated in FIG. B03, as compared with the configuration of the observation apparatus 1C illustrated in FIG. A03, has the common configuration for the optical system from the light source 11 to the imaging unit 43, and further, is different in that the analysis unit 60 is provided instead of the analysis unit 50.

The analysis unit 60 is electrically connected to the imaging unit 43, and inputs the interference intensity image output from the imaging unit 43. The analysis unit 60 calculates a three-dimensional refractive index distribution of the observation object S by processing the input interference intensity image. The analysis unit 60 may be a computer. The analysis unit 60 includes an interference intensity image acquisition unit 61, a first complex amplitude image generation unit 62, a second complex amplitude image generation unit 63, a phase conjugate operation unit 64, a two-dimensional phase image generation unit 65, a three-dimensional phase image generation unit 66, a refractive index distribution calculation unit 67, a display unit 68, and a storage unit 69.

The interference intensity image acquisition unit 61 irradiates the observation object S with the light along each of a plurality of light irradiation directions by changing the orientation of the reflection surface of the mirror 22. Further, the interference intensity image acquisition unit 61 acquires the interference intensity image at a reference position for each of the plurality of light irradiation directions from the imaging unit 43. The interference intensity image acquisition unit 61 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 perform processing based on the interference intensity images, and include a processing device such as a CPU, a GPU, a DSP, or an FPGA. The display unit 68 displays an image to be processed, an image in the middle of the processing, an image after the processing, and the like, and includes, for example, a liquid crystal display. The storage unit 69 stores data of various images, and includes a hard disk drive, a flash memory, a RAM, a ROM, and the like. The first complex amplitude image generation unit 62, the second complex amplitude image generation unit 63, the phase conjugate operation unit 64, the two-dimensional phase image generation unit 65, the three-dimensional phase image generation unit 66, the refractive index distribution calculation unit 67, and the storage unit 68 may be constituted by a cloud computing.

The storage unit 69 also stores a program for causing the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67, to execute respective steps of the processing. The program may be stored in the storage unit 69 at the time of manufacture or shipment of the observation apparatus 1D to 1F, may be acquired via a communication line after shipment and then stored in the storage unit 69, or may be recorded in a computer readable recording medium 2 and then stored in the storage unit 69. The recording medium 2 may be an arbitrary medium such as a flexible disk, a CD-ROM, a DVD-ROM, a BD-ROM, a USB memory, or the like.

The details of the processing step of each of the interference intensity image acquisition unit 61, the first complex amplitude image generation unit 62, the second complex amplitude image generation unit 63, the phase conjugate operation unit 64, the two-dimensional phase image generation unit 65, the three-dimensional phase image generation unit 66, and the refractive index distribution calculation unit 67 will be described later.

FIG. B04 is a flowchart of the refractive index distribution measuring method B. The refractive index distribution measuring method B can be applied to each of the observation apparatus 1D to 1F. The refractive index distribution measuring method B includes an interference intensity image acquisition step S61, a first complex amplitude image generation step S62, a second complex amplitude image generation step S63, a phase conjugate operation step S64, a two-dimensional phase image generation step S65, a three-dimensional phase image generation step S66, and a refractive index distribution calculation step S67.

The processing step of the interference intensity image acquisition step S61 is performed by the interference intensity image acquisition unit 61. The processing step of the first complex amplitude image generation step S62 is performed by the first complex amplitude image generation unit 62. The processing step of the second complex amplitude image generation step S63 is performed by the second complex amplitude image generation unit 63. The processing step of the phase conjugate operation step S64 is performed by the phase conjugate operation unit 64. The processing step of the two-dimensional phase image generation step S65 is performed by the two-dimensional phase image generation unit 65. The processing step of the three-dimensional phase image generation step S66 is performed by the three-dimensional phase image generation unit 66. The processing step of the refractive index distribution calculation step S67 is performed by the refractive index distribution calculation unit 67.

In the interference intensity image acquisition step S61, the interference intensity image acquisition unit 61 irradiates the observation object S with the light along each of the plurality of light irradiation directions by changing the orientation of the reflection surface of the mirror 22. Further, the interference intensity image acquisition unit 61 acquires the interference intensity image at the reference position for each of the plurality of light irradiation directions from the imaging unit 43.

In the first complex amplitude image generation step S62, the first complex amplitude image generation unit 62 generates, for each of the plurality of light irradiation directions, a complex amplitude image based on the interference intensity image of the reference position acquired by the interference intensity image acquisition unit 61. In the case of the observation apparatus 1D (FIG. B01) or the observation apparatus 1F (FIG. B03), the first complex amplitude image generation unit 62 can generate the complex amplitude image based on one interference intensity image by a Fourier fringe analysis method. In the case of the observation apparatus 1E (FIG. B02), the first complex amplitude image generation unit 62 can generate the complex amplitude image based on three or more interference intensity images having different optical path differences (phase differences) between the object light and the reference light by a phase shift method.

In the second complex amplitude image generation step S63, the second complex amplitude image generation unit 63 generates, for each of the plurality of light irradiation directions, a complex amplitude image at each of a plurality of z direction positions based on the complex amplitude image at the reference position (z = 0) generated by the first complex amplitude image generation unit 62.

The interference intensity image acquisition step S61, the first complex amplitude image generation step S62, and the second complex amplitude image generation step S63 in the refractive index distribution measuring method B respectively perform the same processing steps as the interference intensity image acquisition step S1, the first complex amplitude image generation step S2, and the second complex amplitude image generation step S3 in the refractive index distribution measuring method A.

The phase conjugate operation step S64 is performed after the processing step of the second complex amplitude image generation step S63. The phase conjugate operation step S64 may be performed before the processing step of the second complex amplitude image generation step S63 (which will be described later). Further, when the second complex amplitude image generation step S63 generates the complex amplitude image at a certain z position through a plurality of stages from the complex amplitude image at the reference position, the phase conjugate operation step S64 may be performed between a certain stage and a next stage in the plurality of stages (which will be described later). In the phase conjugate operation step S64, the phase conjugate operation unit 64 performs a phase conjugate operation on the complex amplitude image of each of the plurality of light irradiation directions to generate a complex amplitude image of each of the plurality of light irradiation directions when the relationship between the light irradiation and the imaging for the observation object is reversed.

In addition, the phase conjugate operation is an operation for the complex amplitude image based on a phase conjugate method, and is an operation of calculating a transmission matrix representing the relationship between the light irradiation and the light output for the object, and including an inverse matrix calculation thereof and coordinate conversion. The phase conjugate method may be referred to as a phase conjugation, a time reversal method, a time reversal, a digital phase conjugation, a digital phase conjugate method, or the like. The details will be described later.

In the two-dimensional phase image generation step S65, the two-dimensional phase image generation unit 65 generates, for each of the plurality of positions, a two-dimensional phase image based on the complex amplitude image of each of the plurality of light irradiation directions generated by the second complex amplitude image generation unit 63 or the phase conjugate operation unit 64. The two-dimensional phase image generated in this step corresponds to a phase image centered on the focused z direction position.

In the two-dimensional phase image generation step S65, when a phase image generated based on the complex amplitude image before performing the processing step of the phase conjugate operation step S64 is set as a first phase image, and a phase image generated based on the complex amplitude image obtained by performing the processing step of the phase conjugate operation step S64 is set as a second phase image, for the plurality of positions, the two-dimensional phase image is generated mainly based on the first phase image at a position relatively close to the imaging unit, and the two-dimensional phase image is generated mainly based on the second phase image at a position relatively far from the imaging unit.

In addition, the phase conjugate operation unit 64 and the subsequent processing steps may be performed after all the complex amplitude images at the plurality of positions are generated for each of the plurality of light irradiation directions in the second complex amplitude image generation step S63. Further, processes of generating the complex amplitude image at one certain z direction position for each of the plurality of light irradiation directions in the second complex amplitude image generation step S63 and generating the two-dimensional phase image at the position in the two-dimensional phase image generation step S65 may be set as a unit, and the unit process may be repeatedly performed while scanning the z direction position. The latter case is preferable in that a capacity of image data to be stored in the storage unit 69 can be reduced.

In the three-dimensional phase image generation step S66, the three-dimensional phase image generation unit 66 generates a three-dimensional phase image based on the two-dimensional phase image at each of the plurality of positions generated by the two-dimensional phase image generation unit 65. The three-dimensional phase image generated in this step is an image in which the positions x and y in the two-dimensional phase image and the position z of the two-dimensional phase image are variables.

In the refractive index distribution calculation step S67, the refractive index distribution calculation unit 67 obtains a three-dimensional refractive index distribution of the observation object by deconvolution based on the three-dimensional phase image generated by the three-dimensional phase image generation unit 66.

The two-dimensional phase image generation step S65, the three-dimensional phase image generation step S66, and the refractive index distribution calculation step S67 in the refractive index distribution measuring method B respectively perform the same processing steps as the two-dimensional phase image generation step S4, the three-dimensional phase image generation step S5, and the refractive index distribution calculation step S6 in the refractive index distribution measuring method A.

FIG. B05 is a diagram illustrating the images and the order of the respective processing steps of the second complex amplitude image generation step S63 and the two-dimensional phase image generation step S65. This diagram illustrates a configuration in which the processing step of the phase conjugate operation step S64 is not performed. In this configuration, in the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

Each of FIG. B06 to FIG. B08 is a diagram illustrating the images and the order of the respective processing steps of the second complex amplitude image generation step S63, the phase conjugate operation step S64, and the two-dimensional phase image generation step S65. Each of these diagrams illustrates a configuration in which the processing step of the phase conjugate operation step S64 is performed before, during, or after the processing step of the second complex amplitude image generation step S63.

A first configuration illustrated in FIG. B06 corresponds to the flowchart illustrated in FIG. B04. In the first configuration, the phase conjugate operation step S64 is performed after the processing step of the second complex amplitude image generation step S63. In the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the first complex amplitude image generation step S62 by the above Formulas (1) and (2) of the formulas of the free propagation.

In the first configuration, subsequently, in the phase conjugate operation step S64, for each of the plurality of positions, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated. Further, in the two-dimensional phase image generation step S65, for each of the plurality of positions, the complex differential interference image is generated based on the complex amplitude image of each of the plurality of light irradiation directions generated in the phase conjugate operation step S64, and in addition, the phase differential image is generated.

In a second configuration illustrated in FIG. B07, the phase conjugate operation step S64 is performed before the processing step of the second complex amplitude image generation step S63. In the phase conjugate operation step S64, for each of the plurality of light irradiation directions, the phase conjugate operation is performed on the complex amplitude image at the reference position ($z = 0$) generated in the first complex amplitude image generation step S62, and the complex amplitude image of each of the plurality of light irradiation directions in the case in which the relationship between the light irradiation and the imaging for the observation object is reversed is generated.

In the second configuration, subsequently, in the second complex amplitude image generation step S63, for each of the plurality of light irradiation directions, the complex amplitude image at each of the plurality of z direction positions ($z = z_1, z_2, z_3$ in this diagram) is generated based on the complex amplitude image at the reference position ($z = 0$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In a third configuration illustrated in FIG. B08, in the case in which the second complex amplitude image generation step S63 generates the complex amplitude image at each of the plurality of positions from the complex amplitude image at the reference position through two stages, the phase conjugate operation step S64 is performed between a first stage and a second stage in the two stages.

In the third configuration, in the first stage of the second complex amplitude image generation step S63, for each of the plurality of light irradiation directions, the complex amplitude image at each of the plurality of z direction positions ($z = z_1, z_3, z_5$ in this diagram) is generated based on the complex amplitude image at the reference position ($z = 0$) generated in the first complex amplitude image generation step S62 by the above Formulas (1) and (2) of the formulas of the free propagation. Subsequently, in the phase conjugate operation step S64, the phase conjugate operation is performed on the complex amplitude image of each of the plurality of light irradiation directions, and the complex amplitude image of each of the plurality of light irradiation directions in the case in which the relationship between the light irradiation and the imaging for the observation object is reversed is generated.

In the third configuration, further subsequently, in the second stage of the second complex amplitude image generation step S63, for each of the plurality of light irradiation directions, the complex amplitude image at each of the z direction positions ($z = z_2, z_4, z_6$) is generated based on the complex amplitude images at the z direction positions ($z = z_1, z_3, z_5$) generated in the phase conjugate operation step S64 by the above Formulas (1) and (2) of the formulas of the free propagation. Further, in the two-dimensional phase image generation step S65, for each of the plurality of positions, the complex differential interference image is generated based on the complex amplitude image of each of the plurality of light irradiation directions generated in the second complex amplitude image generation step S63, and in addition, the phase differential image is generated.

In the first configuration, the second configuration, and the third configuration described above, the number of times of the phase conjugate operation on the complex amplitude image in the phase conjugate operation step S64 is different. The overall processing time of the phase conjugate operation step S64 is shorter in the third configuration than in the first configuration, and is even shorter in the second configuration.

FIG. B09 is a diagram illustrating the images and the order of the respective processing steps of the three-dimensional phase image generation step S66 and the refractive index distribution calculation step S67. In the three-dimensional phase image generation step S66, the three-dimensional phase image is generated based on the two-dimensional phase image of each of the plurality of positions generated in the two-dimensional phase image generation step S65. In this case, for the position which is relatively close to the imaging unit, the two-dimensional phase image generated based on the complex amplitude image before performing the processing step of the phase conjugate operation step S64 (the two-dimensional phase image generated in the configuration illustrated in FIG. B05) is mainly used. On the other hand, for the position which is relatively far from the imaging unit, the two-dimensional phase image generated based on the complex amplitude image after performing the processing step of the phase conjugate operation step S64 (the two-dimensional phase image generated in any one of the configurations illustrated in FIG. B06 to FIG. B08) is mainly used. Subsequently, in the refractive index distribution calculation step S67, the three-dimensional refractive index distribution of the observation object is obtained by deconvolution based on the three-dimensional phase image generated in the three-dimensional phase image generation step S66. Each refractive index distribution data constituting the three-dimensional refractive index distribution (for example, a two-dimensional refractive index distribution data constituting the three-dimensional refractive index distribution in FIG. B09) can be a refractive index cross sectional data.

The generation of the two-dimensional phase image at each position in the z direction includes the following three configurations. The phase image generated based on the complex amplitude image before performing the processing step of the phase conjugate operation step S64 (the phase image generated in the configuration illustrated in FIG. B05) is set as the first phase image $\phi_1$. The phase image generated based on the complex amplitude image after performing the processing step of the phase conjugate operation step S64 (the phase image generated in any one of the configurations illustrated in FIG. B06 to FIG. B08) is set as the second phase image $\phi_2$. A weight function $\alpha$ having a differential coefficient of 0 or less with respect to the variable z representing the distance from the imaging unit along the light propagation path is used. The value of the weight function is 0 or more and 1 or less.

In the first configuration, it is assumed that the weight function $\alpha$ has a positive value (for example, 1) in a range in which z is threshold value $z_{th}$ or less, and has a value of 0 in a range other than the above range. That is, the two-dimensional phase image is represented by the following Formula (8).

[Formula 8]

$$\phi(x, y, z) = \begin{cases} \phi_1(x, y, z) & z \le z_{th} \\ \phi_2(x, y, z) & z > z_{th} \end{cases} \qquad (8)$$

In the second configuration, it is assumed that the weight function $\alpha$ is a function having a value which continuously changes in at least a partial range in the z direction. That is, the two-dimensional phase image is represented by the following Formula (9).

[Formula 9]

$$\phi(x, y, z) = \alpha(z) \cdot \phi_1(x, y, z) + \left[1 - \alpha(z)\right] \phi_2(x, y, z) \qquad (9)$$

In the third configuration, it is assumed that the weight function $\alpha$ has a value according to the position (x, y) on the plane perpendicular to the optical axis (the z direction). That is, the two-dimensional phase image is represented by the following Formula (10).

[Formula 10]

$$\phi(x,y,z) = \alpha(x,y,z) \cdot \phi_1(x,y,z) + \left[1 - \alpha(x,y,z)\right]\phi_2(x,y,z) \qquad (10)$$

Next, the contents of the phase conjugate operation by the phase conjugate operation step S64 will be described with reference to FIG. B10 and FIG. B11.

FIG. B10 is a diagram illustrating input light $U_{in}(k_{in})$ and output light $u_{out}(r_{out})$ when the interference intensity image is imaged by the imaging unit. $U_{in}(k_{in})$ represents a complex amplitude of a wavenumber $k_{in}$ of the light with which the observation object is irradiated. $u_{out}(r_{out})$ represents a complex amplitude of a position $r_{out}$ of the light output from the observation object. The relationship between $U_{in}(k_{in})$ and $u_{out}(r_{out})$ is represented by the following Formula (11). An n-th element $U_{in}(k_{in}^n)$ of a column vector $U_{in}$ represents a complex amplitude of a plane wave of a wavenumber of $k_{in}^n$. An n-th element $u_{out}(r_{out}^n)$ of a column vector $u_{out}$ represents a complex amplitude of the light observed at a position $r_{out}^n$. A matrix $T(r_{out}, k_{in})$ of N rows and N columns represents a linear relationship between $U_{in}(k_{in})$ and $u_{out}(r_{out})$, and is referred to as a transmission matrix. A scattering process of the light in the observation object can be represented by the transmission matrix described above. An element $T_{n1,n2}$ of an n1-th row and an n2-th column of the matrix $T(r_{out}, k_{in})$ represent a complex amplitude of the light observed at a position $r_{out}^{n1}$ when the plane wave having a wavenumber of $k_{in}^{n2}$ and an amplitude of 1 is input.

[Formula 11]

$$
\begin{pmatrix} u_{out}(\mathbf{r}_{out}^1) \\ \vdots \\ u_{out}(\mathbf{r}_{out}^N) \end{pmatrix} = \begin{pmatrix} T(\mathbf{r}_{out}^1, \mathbf{k}_{in}^1) & \cdots & T(\mathbf{r}_{out}^1, \mathbf{k}_{in}^N) \\ \vdots & \ddots & \vdots \\ T(\mathbf{r}_{out}^N, \mathbf{k}_{in}^1) & \cdots & T(\mathbf{r}_{out}^N, \mathbf{k}_{in}^N) \end{pmatrix} \begin{pmatrix} U_{in}(\mathbf{k}_{in}^1) \\ \vdots \\ U_{in}(\mathbf{k}_{in}^N) \end{pmatrix}
$$

$$
= \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^1) \\ \vdots \\ U_{in}(\mathbf{k}_{in}^N) \end{pmatrix} \qquad (11)
$$

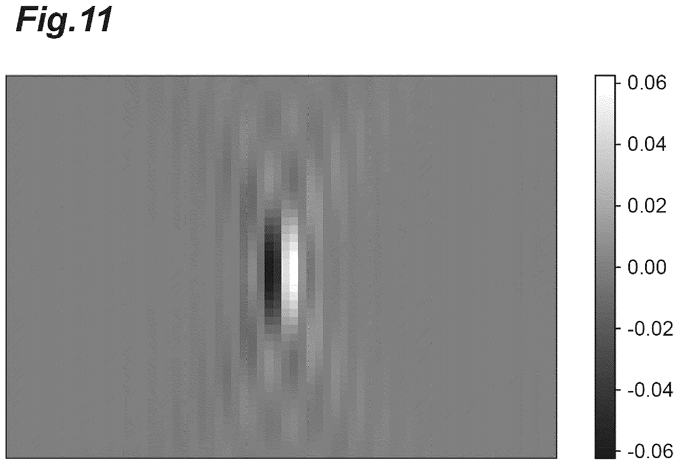

FIG. 11 is a diagram illustrating input light $U_{out}(k_{out})$ and output light $u_{in}(r_{in})$ in the case in which the relationship between the light irradiation and the imaging is reversed. In this case, $U_{out}(k_{out})$ represents a complex amplitude of a wavenumber $k_{out}$ of the light with which the observation object is irradiated. $u_{in}(r_{in})$ represents a complex amplitude of a position $r_{in}$ of the light output from the observation object. The relationship between $U_{out}(k_{out})$ and $u_{in}(r_{in})$ is represented by the following Formula (12). An n-th element $U_{out}(k_{out}^n)$ of a column vector $U_{out}$ represents a complex amplitude of a plane wave of a wavenumber of $k_{out}^n$. An n-th element $u_{in}(r_{in}^n)$ of a column vector $u_{in}$ represents a complex amplitude of the light observed at a position $r_{in}^n$. A matrix $S(r_{in}, k_{out})$ of N rows and N columns represents a linear relationship between $U_{out}(k_{out})$ and $u_{in}(r_{in})$, and is a transmission matrix in the case in which the relationship between the light irradiation and the imaging is reversed.

[Formula 12]

$$\begin{pmatrix} u_{in}(\mathbf{r}_{in}^1) \\ \vdots \\ u_{in}(\mathbf{r}_{in}^N) \end{pmatrix} = \begin{pmatrix} S(\mathbf{r}_{in}^1, \mathbf{k}_{out}^1) & \cdots & S(\mathbf{r}_{in}^1, \mathbf{k}_{out}^N) \\ \vdots & \ddots & \vdots \\ S(\mathbf{r}_{in}^N, \mathbf{k}_{out}^1) & \cdots & S(\mathbf{r}_{in}^N, \mathbf{k}_{out}^N) \end{pmatrix} \begin{pmatrix} U_{out}(\mathbf{k}_{out}^1) \\ \vdots \\ U_{out}(\mathbf{k}_{out}^N) \end{pmatrix}$$

$$= \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix} \qquad (12)$$

$U_{in}(k_{in})$ is represented by the Fourier transform of $u_{in}(r_{in})$ as shown in the following Formula (13). $U_{out}(k_{out})$ is represented by the Fourier transform of $u_{out}(r_{out})$ as shown in the following Formula (14). When Formulas (11) to (14) are used, the transmission matrix $S(r_{in}, k_{out})$ in the case in which the relationship between the light irradiation and the imaging is reversed is represented by the following Formula (15) by using a matrix representing the inverse Fourier transform and the transmission matrix $T(r_{out}, k_{in})$.

[Formula 13]

$$\begin{pmatrix} U_{in}(\mathbf{k}_{in}^1) \\ \vdots \\ U_{in}(\mathbf{k}_{in}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{in}(\mathbf{r}_{in}^1) \\ \vdots \\ u_{in}(\mathbf{r}_{in}^N) \end{pmatrix} \qquad (13)$$

[Formula 14]

$$\begin{pmatrix} U_{out}(\mathbf{k}_{out}^1) \\ \vdots \\ U_{out}(\mathbf{k}_{out}^N) \end{pmatrix} = \begin{pmatrix} F_{1,1} & \cdots & F_{1,N} \\ \vdots & \ddots & \vdots \\ F_{N,1} & \cdots & F_{N,N} \end{pmatrix} \begin{pmatrix} u_{out}(\mathbf{r}_{out}^1) \\ \vdots \\ u_{out}(\mathbf{r}_{out}^N) \end{pmatrix} \qquad (14)$$

[Formula 15]

$$\begin{pmatrix} S_{1,1} & \cdots & S_{1,N} \\ \vdots & \ddots & \vdots \\ S_{N,1} & \cdots & S_{N,N} \end{pmatrix} = \begin{pmatrix} F_{1,1} & \cdots & F_{1,N} \\ \vdots & \ddots & \vdots \\ F_{N,1} & \cdots & F_{N,N} \end{pmatrix}^{-1} \begin{pmatrix} T_{1,1} & \cdots & T_{1,N} \\ \vdots & \ddots & \vdots \\ T_{N,1} & \cdots & T_{N,N} \end{pmatrix}^{-1} \begin{pmatrix} F_{1,1} & \cdots & F_{1,N} \\ \vdots & \ddots & \vdots \\ F_{N,1} & \cdots & F_{N,N} \end{pmatrix}^{-1} \qquad (15)$$

In the phase conjugate operation step S64, first, the transmission matrix $T(r_{out}, k_{in})$ when the interference intensity image is imaged by the imaging unit is obtained based on the complex amplitude image. Next, based on the above transmission matrix $T(r_{out}, k_{in})$ and the above Formula (15), the transmission matrix $S(r_{in}, k_{out})$ in the case in which the relationship between the light irradiation and the imaging is reversed is obtained. Further, based on the above transmission matrix $S(r_{in}, k_{out})$, the complex amplitude image in the case in which the relationship between the light irradiation and the imaging is reversed is obtained.

The vector $U_{in}^{n}(k_{in})$ of the input light of the n-th light irradiation direction when the interference intensity image is imaged by the imaging unit for each of the plurality of light irradiation directions is represented by the following Formula (16), in which only the value of the n-th element is 1 and the values of the other elements are 0. For the above input light $U_{in}^{n}(k_{in})$, the output light $u_{out}^{n}(r_{out})$ is represented by the following Formula (17). The Formula (17) corresponds to the complex amplitude obtained for the n-th light irradiation direction.

[Formula 16]

$$U_{in}^{n}(\mathbf{k}_{in}) = \begin{pmatrix} U_{in}^{n}(\mathbf{k}_{in}^{1}) \\ \vdots \\ U_{in}^{n}(\mathbf{k}_{in}^{N}) \end{pmatrix} = \begin{pmatrix} 0 \\ \vdots \\ 0 \\ 1 \\ 0 \\ \vdots \\ 0 \end{pmatrix} \qquad (16)$$

[Formula 17]

$$u_{out}^{n}(\mathbf{r}_{out}) = \begin{pmatrix} u_{out}^{n}(\mathbf{r}_{out}^{1}) \\ \vdots \\ u_{out}^{n}(\mathbf{r}_{out}^{N}) \end{pmatrix} \qquad (17)$$

From the Formula (16) and the above Formula (11), the following Formula (18) is obtained. Further, the following Formula (19) is obtained by similarly obtaining for each of the plurality of light irradiation directions. In this way, the transmission matrix $T(r_{out}, k_{in})$ can be obtained. In addition, from the Formula (19) and the above Formula (15), the transmission matrix $S(r_{in}, k_{out})$ in the case in which the relationship between the light irradiation and the imaging is reversed can be obtained.

[Formula 18]

$$\begin{pmatrix} u_{out}^{n}(\mathbf{r}_{out}^{1}) \\ \vdots \\ u_{out}^{n}(\mathbf{r}_{out}^{N}) \end{pmatrix} = \begin{pmatrix} T_{1,1} & \cdots & T_{1,N} \\ \vdots & \ddots & \vdots \\ T_{N,1} & \cdots & T_{N,N} \end{pmatrix} \begin{pmatrix} U_{in}(\mathbf{k}_{in}^{1}) \\ \vdots \\ U_{in}(\mathbf{k}_{in}^{N}) \end{pmatrix} = \begin{pmatrix} T_{1,n} \\ \vdots \\ T_{N,n} \end{pmatrix} \qquad (18)$$

[Formula 19]

$$\begin{pmatrix} T_{1,1} & \cdots & T_{1,N} \\ \vdots & \ddots & \vdots \\ T_{N,1} & \cdots & T_{N,N} \end{pmatrix} = \begin{pmatrix} u_{out}^{1}(\mathbf{r}_{out}^{1}) & \cdots & u_{out}^{N}(\mathbf{r}_{out}^{1}) \\ \vdots & \ddots & \vdots \\ u_{out}^{1}(\mathbf{r}_{out}^{N}) & \cdots & u_{out}^{N}(\mathbf{r}_{out}^{N}) \end{pmatrix} \qquad (19)$$

The input light $U_{out}^n(k_{out})$ of the n-th light irradiation direction out of the plurality of light irradiation directions in the case in which the relationship between the light irradiation and the imaging is reversed is represented by the following Formula (20), in which only the value of the n-th element is 1 and the values of the other elements are 0. From this Formula, the output light $u_{in}^n(r_{in})$ for the input light $U_{out}^n(k_{out})$ is represented by the following Formula (21). The Formula (21) represents the complex amplitude when the relationship between the light irradiation and the imaging is reversed. In this way, the complex amplitude image in the case in which the relationship between the light irradiation and the imaging is reversed can be obtained.

[Formula 20]

$$U_{out}^n(\mathbf{k}_{out}) = \begin{pmatrix} U_{out}^n(\mathbf{k}_{out}^1) \\ \vdots \\ U_{out}^n(\mathbf{k}_{out}^N) \end{pmatrix} = \begin{pmatrix} 0 \\ \vdots \\ 0 \\ 1 \\ 0 \\ \vdots \\ 0 \end{pmatrix} \qquad (20)$$

[Formula 21]

$$\begin{pmatrix} u_{in}^n(\mathbf{r}_{in}^1) \\ \vdots \\ u_{in}^n(\mathbf{r}_{in}^N) \end{pmatrix} = \begin{pmatrix} S_{1,1} & \cdots & S_{1,N} \\ \vdots & \ddots & \vdots \\ S_{N,1} & \cdots & S_{N,N} \end{pmatrix} \begin{pmatrix} U_{out}(\mathbf{k}_{out}^1) \\ \vdots \\ U_{out}(\mathbf{k}_{out}^N) \end{pmatrix} = \begin{pmatrix} S_{1,n} \\ \vdots \\ S_{N,n} \end{pmatrix} \qquad (21)$$

When the transmission matrix $S(r_{in}, k_{out})$ in the case in which the relationship between the light irradiation and the imaging is reversed is obtained, it is necessary to calculate the inverse matrix of the transmission matrix $T(r_{out}, k_{in})$ as shown in the above Formula (15). Therefore, the transmission matrix T needs to be a square matrix in which the number of row elements and the number of column elements are equal to each other. That is, a matrix dimension in a light irradiation side wavenumber space for the observation object in the interference intensity image acquisition step S61 and the number of pixels of the complex amplitude image need to be equal to each other.

In order to make them equal to each other, the matrix dimension in the light irradiation side wavenumber space for the observation object in the interference intensity image acquisition step S61 may be made equal to the number of pixels, or only a partial range of the image acquired by the imaging unit may be used in the subsequent processing steps. However, in general, the number of pixels of the image acquired by the imaging unit is, for example, 1024 × 1024, and thus, it is not easy to make the matrix dimension in the light irradiation side wavenumber space for the observation object equal to the number of pixels. Further, it is not preferable to use only the partial range of the image out of the image acquired by the imaging unit in the subsequent processing steps because this leads to a decrease in resolution.

Therefore, as illustrated in FIG. B12, in the phase conjugate operation step S64, it is preferable to divide the complex amplitude image into a plurality of partial images each having the same number of pixels as the matrix dimension in the light irradiation side wavenumber space for the observation object, perform the phase conjugate operation on each of the plurality of partial images, and then combine the plurality of partial images. In this case, any two or more partial images out of the plurality of partial images may have a common region.

Next, the refractive index distribution measuring method C will be will be described. FIG. C01 to C03 are diagrams showing respective configurations of observation apparatuses 1G to 1I that can be used when measuring the refractive index distribution by the refractive index distribution measuring method C. The observation apparatus 1G illustrated in FIG. C01, as compared with the configuration of the observation apparatus 1A illustrated in FIG. A01, has the common configuration for the optical system from the light source 11 to the imaging unit 43, and further, is different in that an analysis unit 70 is provided instead of the analysis unit 50. The observation apparatus 1H illustrated in FIG. C02, as compared with the configuration of the observation apparatus 1B illustrated in FIG. A02, has the common configuration for the optical system from the light source 11 to the imaging unit 43, and further, is different in that the analysis unit 70 is provided instead of the analysis unit 50. The observation apparatus 1I illustrated in FIG. C03, as compared with the configuration of the observation apparatus 1C illustrated in FIG. A03, has the common configuration for the optical system from the light source 11 to the imaging unit 43, and further, is different in that the analysis unit 70 is provided instead of the analysis unit 50.

The analysis unit 70 is electrically connected to the imaging unit 43, and inputs the interference intensity image output from the imaging unit 43. The analysis unit 70 calculates a three-dimensional refractive index distribution of the observation object S by processing the input interference intensity image. The analysis unit 70 may be a computer. The analysis unit 70 includes an interference intensity image acquisition unit 71, a first complex amplitude image generation unit 72, a second complex amplitude image generation unit 73, a two-dimensional phase image generation unit 74, a three-dimensional phase image generation unit 75, a refractive index distribution calculation unit 76, a third complex amplitude image generation unit 77, a display unit 78, and a storage unit 79.

The interference intensity image acquisition unit 71 irradiates the observation object S with the light along each of a plurality of light irradiation directions by changing the orientation of the reflection surface of the mirror 22. Further, the interference intensity image acquisition unit 71 acquires the interference intensity image at a reference position for each of the plurality of light irradiation directions from the imaging unit 43. The interference intensity image acquisition unit 71 includes a CPU, has an output port for outputting a control signal for changing the orientation of the reflection surface of the mirror 22, and has an input port for inputting the interference intensity image from the imaging unit 43. It is not necessary to move the objective lens 25 in an optical axis direction. The reference position is an image plane position having a conjugate relationship with respect to the imaging plane of the imaging unit 43.

The first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 perform processing based on the interference intensity images, and include a processing device such as a CPU, a GPU, a DSP, or an FPGA. The display unit 78 displays an image to be processed, an image in the middle of the processing, an image after the processing, and the like, and includes, for example, a liquid crystal display. The storage unit 79 stores data of various images, and includes a hard disk drive, a flash memory, a RAM, a ROM, and the like. The first complex amplitude image generation unit 72, the second complex amplitude image generation unit 73, the two-dimensional phase image generation unit 74, the three-dimensional phase image generation unit 75, the refractive index distribution calculation unit 76, the third complex amplitude image generation unit 77, and the storage unit 79 may be constituted by a cloud computing.

The storage unit 79 also stores a program for causing the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 to execute respective steps of the processing. The program may be stored in the storage unit 79 at the time of manufacture or shipment of the observation apparatus 1G to 1I, may be acquired via a communication line after shipment and then stored in the storage unit 79, or may be recorded in a computer readable recording medium 2 and then stored in the storage unit 79. The recording medium 2 may be an arbitrary medium such as a flexible disk, a CD-ROM, a DVD-ROM, a BD-ROM, a USB memory, or the like.

The details of the processing step of each of the interference intensity image acquisition unit 71, the first complex amplitude image generation unit 72, the second complex amplitude image generation unit 73, the two-dimensional phase image generation unit 74, the three-dimensional phase image generation unit 75, the refractive index distribution calculation unit 76, and the third complex amplitude image generation unit 77 will be described later.

FIG. C04 and FIG. C05 are flowcharts of the refractive index distribution measuring method C. FIG. C05 illustrates a part of the flowchart illustrated in FIG. C04. The refractive index distribution measuring method C can be applied to each of the observation apparatus 1G to 1I. The observation method includes an interference intensity image acquisition step S71, a first complex amplitude image generation step S72, a second complex amplitude image generation step S73, a two-dimensional phase image generation step S74, a three-dimensional phase image generation step S75, a refractive index distribution calculation step S76, and a third complex amplitude image generation step S77.

The processing step of the interference intensity image acquisition step S71 is performed by the interference intensity image acquisition unit 71. The processing step of the first complex amplitude image generation step S72 is performed by the first complex amplitude image generation unit 72. The processing step of the second complex amplitude image generation step S73 is performed by the second complex amplitude image generation unit 73. The processing step of the two-dimensional phase image generation step S74 is performed by the two-dimensional phase image generation unit 74. The processing step of the three-dimensional phase image generation step S75 is performed by the three-dimensional phase image generation unit 75. The processing step of the refractive index distribution calculation step S76 is performed by the refractive index distribution calculation unit 76. The processing step of the third complex amplitude image generation step S77 is performed by the third complex amplitude image generation unit 77.

In the interference intensity image acquisition step S71, the interference intensity image acquisition unit 71 irradiates the observation object S with the light along each of the plurality of light irradiation directions by changing the orientation of the reflection surface of the mirror 22. Further, the interference intensity image acquisition unit 71 acquires the interference intensity image at the reference position for each of the plurality of light irradiation directions from the imaging unit 43.

In the first complex amplitude image generation step S72, the first complex amplitude image generation unit 72 generates, for each of the plurality of light irradiation directions, a complex amplitude image based on the interference intensity image acquired by the interference intensity image acquisition unit 71. In the case of the observation apparatus 1G (FIG. C01) or the observation apparatus 11 (FIG. C03), the first complex amplitude image generation unit 72 can generate the complex amplitude image based on one interference intensity image by a Fourier fringe analysis method. In the case of the observation apparatus 1H (FIG. C02), the first complex amplitude image generation unit 72 can generate the complex amplitude image based on three or more interference intensity images having different optical path differences (phase differences) between the object light and the reference light by a phase shift method. The complex amplitude image generated in the first complex amplitude image generation step S72 may be at the same reference position as the interference intensity image or may be at another position generated based on the complex amplitude image at the reference position.

In the second complex amplitude image generation step S73, the second complex amplitude image generation unit 73 generates, for each of the plurality of light irradiation directions, a complex amplitude image at each of a plurality of z direction positions between a first position and a second position based on the complex amplitude image at the first position with respect to a distance from the imaging unit 43 along a light propagation path.

In the two-dimensional phase image generation step S74, the two-dimensional phase image generation unit 74 generates, for each of the plurality of positions, a two-dimensional phase image based on the complex amplitude image of each of the plurality of light irradiation directions generated by the second complex amplitude image generation unit 73. The two-dimensional phase image generated in this step corresponds to a phase image centered on the focused z direction position.

In the three-dimensional phase image generation step S75, the three-dimensional phase image generation unit 75 generates a three-dimensional phase image between the first position and the second position based on the two-dimensional phase image at each of the plurality of positions generated by the two-dimensional phase image generation unit 74. The three-dimensional phase image generated in this step is an image in which the positions x and y in the two-dimensional phase image and the position z of the two-dimensional phase image are variables.

In the refractive index distribution calculation step S76, the refractive index distribution calculation unit 76 obtains a three-dimensional refractive index distribution of the observation object between the first position and the second position by deconvolution based on the three-dimensional phase image generated by the three-dimensional phase image generation unit 75.

The interference intensity image acquisition step S71, the first complex amplitude image generation step S72, the second complex amplitude image generation step S73, the two-dimensional phase image generation step S74, the three-dimensional phase image generation step S75, and the refractive index distribution calculation step S76 in the refractive index distribution measuring method C respectively perform substantially the same processing steps as the interference intensity image acquisition step S1, the first complex amplitude image generation step S2, the second complex amplitude image generation step S3, the two-dimensional phase image generation step S4, the three-dimensional phase image generation step S5, and the refractive index distribution calculation step S6 in the refractive index distribution measuring method A.

In the third complex amplitude image generation step S77, the third complex amplitude image generation unit 77 generates, for each of the plurality of light irradiation directions, a complex amplitude image at the second position based on the complex amplitude image at the first position used in the second complex amplitude image generation step S73 and the three-dimensional refractive index distribution of the observation object between the first position and the second position calculated in the refractive index distribution calculation step S76.

In the step S83 including the second complex amplitude image generation step S73, the two-dimensional phase image generation step S74, the three-dimensional phase image generation step S75, and the refractive index distribution calculation step S76, the three-dimensional refractive index distribution of the observation object between the first position and the second position is obtained based on the complex amplitude image at the first position with respect to the distance from the imaging unit 43 along the light propagation path. The processing steps of the step S83 and the third complex amplitude image generation step S77 are repeatedly performed. This will be described with reference to FIG. C04 to FIG. C07.

FIG. C06 is a diagram illustrating a relationship between a region including the observation object and first to J-th blocks. As illustrated in this diagram, the region including the observation object is divided into the first to J-th blocks in order based on the distance from the imaging unit along the light propagation path (z direction). In this diagram, it is set to J = 3. The j-th block in the first to J-th blocks is a region from $z = z_{j-1}$ to $z = z_j$. In each j-th block, a position (near end) of $z = z_{j-1}$ closest to the imaging unit is set as the first position, and a position (far end) of $z = z_j$ farthest from the imaging unit is set as the second position.

FIG. C07 is a diagram illustrating a processing procedure for the first to J-th blocks. As illustrated in this diagram, for each j-th block, in the step S83, the complex amplitude image and the two-dimensional phase image at each of the plurality of z direction positions from the first position to the second position are generated based on the complex amplitude image at the first position, the three-dimensional phase image between the first position and the second position is generated, and the three-dimensional refractive index distribution is obtained. For each j-th block, in the third complex amplitude image generation step S77, the complex amplitude image at the second position is generated based on the complex amplitude image at the first position and the three-dimensional refractive index distribution calculated in the step S83.

The complex amplitude image at the second position in the (j-1)-th block generated in the third complex amplitude image generation step S77 is used as the complex amplitude image at the first position in the next j-th block, and the processing steps of the step S83 and the third complex amplitude image generation step S77 are performed for the j-th block. When the three-dimensional refractive index distribution is obtained for each of the first to J-th blocks, the three-dimensional refractive index distribution of the entire observation object is obtained by combining these distributions. The three-dimensional refractive index distribution of each of the first to J blocks (for example, the refractive index distribution of the first block, the refractive index distribution of the second block, and the refractive index distribution of the third block in FIG. C07) can be a refractive index cross sectional data.

As illustrated in FIG. C04 and FIG. C05, in the step S81 after the first complex amplitude image generation step S72, it is set to j = 0, and in the subsequent step S82, the value of j is increased as j = 1, and the processing steps of the step S83 and the third complex amplitude image generation step S77 are performed for the first block. That is, for the first block closest to the imaging unit, based on the complex amplitude image generated in the first complex amplitude image generation step S72, a position of $z = z_0$ (near end) closest to the imaging unit is set as the first position, a position of $z = z_1$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the j-th block (in this case, j is 2 or more and less than J), based on the complex amplitude image generated for the (j-1)-th block in the third complex amplitude image generation step S77, a position of $z = z_{j-1}$ (near end) closest to the imaging unit is set as the first position, a position of $z = z_j$ (far end) farthest from the imaging unit is set as the second position, and the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 are sequentially performed. Thereafter, the process returns to the step S82.

For the J-th block which is the last block farthest from the imaging unit, based on the complex amplitude image generated for the (J-1)-th block in the third complex amplitude image generation step S77, a position of $z = z_{J-1}$ (near end) closest to the imaging unit is set as the first position, a position of $z = z_J$ (far end) farthest from the imaging unit is set as the second position, and the processing step of the step S83 (the second complex amplitude image generation step S73, the two-dimensional phase image generation step S74, the three-dimensional phase image generation step S75, and the refractive index distribution calculation step S76) is performed.

For the J-th block, it is determined to be the last block in the step S84 after the step S83, and may be ended without proceeding to the third complex amplitude image generation step S77. In addition, for the J-th block, it may be determined to be the last block after the three-dimensional phase image generation step S75, and may be ended without proceeding to the refractive index distribution calculation step S76, and in this case, the three-dimensional phase image of the entire observation object is obtained.

In addition, the region including the observation object may be divided into the two blocks in order based on the distance from the imaging unit along the light propagation path (z direction), and in this case, the processing for the first block and the processing for the last J-th block described above may be performed. Further, the region including the observation object may not be divided into the plurality of blocks, and in this case, the respective processing steps of the step S83 (the second complex amplitude image generation step S73, the two-dimensional phase image generation step S74, the three-dimensional phase image generation step S75, and the refractive index distribution calculation step S76) and the third complex amplitude image generation step S77 may be sequentially performed only once.

Next, the details of the third complex amplitude image generation step S77 will be described. When acquiring the interference intensity image by irradiating the observation object with the light, in the j-th block, a light wavefront at the second position ($z = z_j$) propagates inside the j-th block to reach the first position ($z = z_{j-1}$) and further propagates to the imaging unit. Therefore, in the third complex amplitude image generation step S77, the light wavefront at the first position ($z = z_{j-1}$) is reversely propagated inside the j-th block by numerical calculation in consideration of the refractive index distribution of the j-th block, thereby obtaining the light wavefront at the second position ($z = z_j$). That is, in the third complex amplitude image generation step S77, for each of the plurality of light irradiation directions, the complex amplitude image at the second position ($z = z_j$) of the j-th block is generated based on the complex amplitude image at the first position ($z = z_{j-1}$) of the j-th block and the refractive index distribution of the j-th block. In the above processing, a method of numerically calculating the propagation of the light wavefront in consideration of the refractive index distribution of the medium is used. A beam propagation method (BPM), a split-step non-paraxial (SSNP), and the like are known as the numerical calculation method of the inhomogeneous medium propagation described above. Hereinafter, the processing using the BPM in the third complex amplitude image generation step S77 will be described.

FIG. C08 is a diagram illustrating processing contents of the BPM. This diagram illustrates an arbitrary j-th block. As illustrated in this diagram, the j-th block is divided into M slices (7 slices in this diagram) (first to M-th slices) based on the distance from the imaging unit along the light propagation path (z direction). A thickness of each slice is about a wavelength.

The thickness of each slice may be constant. In this case, it is assumed that the thickness of each slice is a constant value of $\Delta z$. The m-th slice out of the first to M-th slices of the j-th block is from a position ($z_{j-1} + (m-1)\Delta z$) to a position ($z_{j-1} + m\Delta z$). In order from the first position ($z = z_{j-1}$) of the j-th block to the second position ($z = z_j$), a phase change according to the refractive index distribution is sequentially applied in each of the first to M-th slices, and the light wavefront is reversely propagated by $\Delta z$.

In addition, the thickness $\Delta z$ of each slice in the processing of the third complex amplitude image generation step S77 may be different from or may coincide with the position interval when generating the complex amplitude image of each of the plurality of z direction positions from the first position to the second position in the processing of the second complex amplitude image generation step S73.

The phase change $o(x, y, z)$ applied to the light wavefront when reversely propagating the slice of the thickness $\Delta z$ at the position z is represented by the following Formula (22). In the Formula (22), $k_v$ is a wavenumber of the light in vacuum. $\delta n(x, y, z)$ is a difference between the refractive index distribution $n(x, y, z)$ of the observation object at the position z and the refractive index $n_b$ of the background (medium), and is represented by the following Formula (23). Further, $\cos\theta$ is represented by the following Formula (24).

[Formula 22]

$$o(x,y,z) = \exp\left(-ik_v \delta n(x,y,z) \frac{\Delta z}{\cos\theta}\right) \qquad (22)$$

[Formula 23]

$$\delta n(x,y,z) = n(x,y,z) - n_b \qquad (23)$$

[Formula 24]

$$\cos\theta = \frac{\sqrt{n_b^2 k_V^2 - k_x^2 - k_y^2}}{n_b k_v} \qquad (24)$$

Assuming that the complex amplitude of the light at the position ($z = z_{j-1} + (m-1)\Delta z$) of the m-th slice is $u(x, y, z)$, the complex amplitude $u(x, y, z + \Delta z)$ of the light at the position ($z + \Delta z$) after the light reversely propagates inside the m-th slice is represented by the following Formula (25). In the Formula (25), $P(k_x, k_y ; \Delta z)$ is represented by the following Formula (26). The Formula (25) indicates that the complex amplitude $u(x, y, z + \Delta z)$ of the light at the position ($z + \Delta z$) after propagating the slice of the thickness $\Delta z$ is obtained by performing Fourier transform on a product of the complex amplitude u(x, y, z) of the light and the phase change o(x, y, z), and performing inverse Fourier transform on a product of a result of the above Fourier transform and P(k$_x$, k$_y$ ; Δz). P$_{Δz}$ is a function for performing calculation of the light propagation of Δz.

[Formula 25]

$$u\left(x,y,z+\Delta z\right)=P_{\Delta z}\left[o\left(x,y,z\right)\cdot u\left(x,y,z\right)\right]$$
$$\equiv F^{-1}\left[P\left(k_x,k_y;\Delta z\right)\cdot F\left[o\left(x,y,z\right)\cdot u\left(x,y,z\right)\right]\right] \qquad (25)$$

[Formula 26]

$$P\left(k_x,k_y;\Delta z\right)=\exp\left(-i\sqrt{n_b^2 k_v^2 - k_x^2 - k_y^2}\,\Delta z\right) \qquad (26)$$

The propagation of the light wavefront in each slice of the j-th block is represented by the following Formulas (27) to (29). That is, when the complex amplitude of the light at the first position (z = z$_{j-1}$) of the j-th block is set to u(x, y, z$_{j-1}$), the complex amplitude u(x, y, z$_{j-1}$ + Δz) of the light after propagating the first slice of the j-th block is represented by the following Formula (27). When the complex amplitude of the light after propagating the (m-1)-th slice of the j-th block is set to u(x, y, z$_{j-1}$ + (m-1)Δz), the complex amplitude u(x, y, z$_{j-1}$ + mΔz) of the light after propagating the m-th slice of the j-th block is represented by the following Formula (28). When the complex amplitude of the light after propagating the (M-1)-th slice of the j-th block is set to u(x, y, z$_{j-1}$ + (M-1)Δz), the complex amplitude u(x, y, z$_j$) of the light at the second position (z = z$_j$) after propagating the M-th slice of the j-th block is represented by the following Formula (29).

[Formula 27]

$$u\left(x,y,z_{j-1}+\Delta z\right)=P_{\Delta z}\left[o\left(x,y,z_{j-1}\right)\cdot u\left(x,y,z_{j-1}\right)\right] \qquad (27)$$

[Formula 28]

$$u\left(x,y,z_{j-1}+m\Delta z\right)=P_{\Delta z}\left[o\left(x,y,z_{j-1}+(m-1)\Delta z\right)\cdot u\left(x,y,z_{j-1}+(m-1)\Delta z\right)\right] \qquad (28)$$

[Formula 29]

$$u\left(x,y,z_j\right)=P_{\Delta z}\left[o\left(x,y,z_{j-1}+(M-1)\Delta z\right)\cdot u\left(x,y,z_{j-1}+(M-1)\Delta z\right)\right] \qquad (29)$$

As described above, in the third complex amplitude image generation step S77, the light wavefront at the first position (z = z$_{j-1}$) is sequentially and reversely propagated inside the j-th block for each slice by the numerical calculation in consideration of the refractive index distribution of the j-th block, and thus, the light wavefront at the second position (z = z$_j$) can be obtained.

FIG. C09 is a flowchart of the third complex amplitude image generation step S77. In a step S41, the position z is initialized to the first position (z = z$_{j-1}$) of the j-th block. In a step S42, interaction between the complex amplitude u(x, y, z) of the light at the position z and the phase change o(x, y, z) is obtained. In a step S43, the wavefront of the light after the interaction is propagated by the distance Δz, and the complex amplitude u(x, y, z + Δ z) of the light at the position z + Δ z is obtained. In a step S44, z obtained by adding Δz is set as new z. In a step S44, when it is determined that the position z has not yet reached the second position ($z = z_j$) of the j-th block, the process returns to the step S42 to repeat the steps S42 to S44. In the step S44, when it is determined that the position z reaches the second position ($z = z_j$) of the j-th block, the processing of the third complex amplitude image generation step S77 is ended. The complex amplitude of the light acquired at the end becomes the complex amplitude at the second position ($z = z_j$) of the j-th block.

Any of the refractive index distribution measuring methods A to C described above can realize three-dimensional refractive index tomography in which the influence of multiple scattered light is reduced even when the observation object is a multiple scattering object. Any of the refractive index distribution measuring methods A to C is suitable for measuring the refractive index distribution of a three-dimensional culture as an observation object.

In addition, self-interference may be used in the observation apparatus and the refractive index distribution measuring methods. For example, an observation apparatus 1J illustrated in FIG. C10 includes a light source 11, a lens 12, a lens 21, a mirror 22, a lens 23, a condenser lens 24, an objective lens 25, a mirror 44, a lens 42, an imaging unit 43, and an analysis unit 70. Compared with the configuration of the observation apparatus described above, the observation apparatus 1J is different in that the light output from the light source 11 is guided by the optical fiber 14, and then output from the light output end 18 without being split into two light beams. Further, the observation apparatus 1J is different in that the mirror 44 is provided instead of the beam splitter 41. The observation apparatus 1J does not include an interference optical system. The imaging unit 43 can image the interference intensity image at the reference position generated by self-interference of the light irradiating the observation object S along each of the plurality of light irradiation directions and passed through the observation object S. The analysis unit 70 can perform the same image processing as described above using the interference intensity image due to self-interference.

Further, the three-dimensional refractive index distribution of the observation object S from the first position to the second position may not be the refractive index distribution based on the three-dimensional phase image, and may be acquired separately by using a refractive index distribution acquisition apparatus capable of acquiring the refractive index distribution. In this case, the observation apparatus may include (1) an interference intensity image acquisition unit for acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation unit for generating, for each of the plurality of light irradiation directions, a complex amplitude image based on the interference intensity image, (3) a refractive index distribution acquisition unit for acquiring a three-dimensional refractive index distribution of the observation object between a first position and a second position with respect to a distance from the imaging unit along a light propagation path, and (4) a second complex amplitude image generation unit for generating, for each of the plurality of light irradiation directions, a complex amplitude image at the second position based on a complex amplitude image at the first position and the three-dimensional refractive index distribution (corresponding to the third complex amplitude image generation unit provided in the observation apparatuses 1A to 1D).

Further, in this case, the refractive index distribution measuring method may include (1) an interference intensity image acquisition step of acquiring, for each of a plurality of light irradiation directions, an interference intensity image at a reference position from an imaging unit for imaging the interference intensity image at the reference position of light irradiating an observation object along each of the plurality of light irradiation directions and passed through the observation object, (2) a first complex amplitude image generation step of generating, for each of the plurality of light irradiation directions, a complex amplitude image based on the interference intensity image, (3) a refractive index distribution acquisition step of acquiring a three-dimensional refractive index distribution of the observation object between a first position and a second position with respect to a distance from the imaging unit along a light propagation path, and (4) a second complex amplitude image generation step of generating, for each of the plurality of light irradiation directions, a complex amplitude image at the second position based on a complex amplitude image at the first position and the three-dimensional refractive index distribution.

In the selection step or selection unit, it is selected whether or not an object included in the refractive index distribution data is an evaluation object. The selection step or the selection unit may include a step of acquiring at least one refractive index cross sectional data in a certain direction from the refractive index distribution data, and a step of selecting whether or not an object included in the refractive index cross sectional data is an evaluation object. In the selection step or the selection unit, it is possible to select whether or not the object is an evaluation object based on at least one indicator selected from the size, shape (circularity, sphericity and the like), density, and average refractive index of the object included in the refractive index distribution data. Which index is used may vary depending on the object.

For example, when the observation object is a cell aggregate, an object having a maximum diameter of 30 to 70 $\mu$m may be an evaluation object, and an object having a maximum diameter of 50 $\mu$m may be an evaluation object. When the observation object is a cell aggregate, an object having a sphericity of 90% or more may be an evaluation object. When the observation object is a cell aggregate, an object existing in a region having a density of $10^{-4}$ cells/$\mu$m$^3$ or more may be an evaluation object.

Whether or not an observation object is an evaluation object may be physically selected by passing the object through a physical filter or performing density gradient centrifugation, whether or not the object is an evaluation object may be selected by prior observation using a phase contrast microscope, whether or not the object is an evaluation object may be selected by an interference intensity image, or a homogenous portion of the object may be selected as an evaluation object on the software. In the case of physical selection or selection by prior observation, it is assumed to be performed before the data acquisition step. In the case of software selection, it is assumed that the selection is performed between the data acquiring step and the evaluating step. By performing the above-described selection, it is possible to suppress a decrease in data reproducibility due to heterogeneity of an observation object.

In the evaluation step or evaluation unit, the refractive index distribution of the evaluation object is evaluated. The evaluation method in the evaluation step or evaluation unit may include a step of extracting a region having a refractive index greater or less than a certain threshold value (threshold method). The certain threshold value may vary depending on the evaluation target, but, for example, if the threshold value is set to 1.375, the region of the refractive index distribution image to be evaluated can be divided into a region of lipid droplets (a region having a refractive index greater than 1.375) and the other region (a region having a refractive index less than or equal to 1.375). Two threshold values of a lower limit and an upper limit may be set to extract a region within a certain refractive index range and the other region. For example, when the lower limit and the upper limit are 1.3575 and 1.3625, respectively, the region to be evaluated described above can be divided into a region including mitochondria (a region having a refractive index greater than 1.3575 and less than 1.3625) and the other region (a region having a refractive index less than or equal to 1.3575 or greater than or equal to 1.3625). When the lower limit and the upper limit are 1.335 and 1.340, respectively, the region to be evaluated described above can be divided into a region including vesicles (a region having a refractive index greater than 1.335 and less than 1.340) and the other region (a region having a refractive index less than or equal to 1.335 or greater than or equal to 1.340). When the lower limit and the upper limit are 1.3525 and 1.3575, respectively, the region to be evaluated described above can be divided into a region including nucleoli (a region having a refractive index greater than 1.3525 and less than 1.3575) and the other region (a region having a refractive index less than or equal to 1.3525 or greater than or equal to 1.3575). When the lower limit and the upper limit are 1.3475 and 1.3525, respectively, the region to be evaluated described above can be divided into a region containing DNA (a region having a refractive index greater than 1.3475 and less than 1.3525) and the other region (a region having a refractive index less than or equal to 1.3475 or greater than or equal to 1.3525). The region to be evaluated may be divided into three or more regions by setting two or more threshold values. For example, if the evaluation object is a cell culture in a medium, and 1.337 and 1.375 are set as threshold values, the evaluation object region can be divided into a medium region (n: refractive index, $n \leq 1.337$), a cell region ($1.337 < n \leq 1.375$), and a lipid droplet region ($1.375 < n$).

The evaluation method in the evaluation step or the evaluation unit may include a step of dividing a region having a refractive index greater or less than a certain threshold value according to the shape (sphericity, circularity or the like), the size, the density or the position of the region in the observation object (division method). The division method is usually used in combination with the threshold method.

For example, it may be divided into a region having a maximum diameter of 5 $\mu$m or more and the other region. It may be divided into a region having a circularity or sphericity of 90% or more and the other region. It may be divided into a region having a density of $10^{-4}/\mu m^3$ or more and the other region. It may be divided into a region existing within a range of 30 $\mu$m or less from the center of the observation object and the other region, and it may be divided into a region existing within a range of 30 $\mu$m or more and 50 $\mu$m or less from the center of the observation object and the other region.

By performing the division method, for example, even if the total area of regions greater or less than a certain threshold value in the evaluation object is the same, it is possible to divide an evaluation object having one large region and an evaluation object having a set of small regions. When the observation object is a cell aggregate, the degree of supply of oxygen, nutrients, drugs and the like to the inner part of the cell aggregate is different depending on the size or shape of the cell aggregate. Thus, it is possible to evaluate the cell aggregate in accordance with the degree of supply of oxygen, nutrients, drugs and the like.

The evaluation method in the evaluation step or the evaluation unit may include a step of identifying one or more positions in the evaluation object and dividing the evaluation object into a region existing at a certain distance from the position and a region existing at a distance farther than the certain distance from the position (method of dividing a cell population). The method of dividing a cell population is usually used in combination with the threshold method.

One or more positions in the evaluation object may be a center of the evaluation object, a periphery of the evaluation object and an approximate curve thereof, or a spherical surface existing inside the evaluation object. When the observation object is a cell aggregate, the degree of supply of oxygen, nutrients, drugs and the like to the inner part of the cell aggregate is different depending on the size or shape of the cell aggregate. Thus, it is possible to evaluate the cell aggregate in accordance with the degree of supply of oxygen, nutrients, drugs and the like.

Preferred embodiments of the evaluation method according to the present invention include the following Embodiments 1 to 4 and Embodiments T1 to T4. The present invention is not limited to the following embodiments.

(Embodiment 1)

Cell aggregates of liver cells are prepared by a known method and the formation of lipid droplets is induced by oleic acid. The refractive index distribution data of the cell aggregates are acquired by the refractive index measuring method A1 to A3, B or C. In the obtained refractive index distribution data, a region having a refractive index greater than 1.375 is determined as a region of lipid droplets, and the area of the region is measured. For example, the effect of a mutation in a gene (or a disease) on lipid metabolism can be assessed by comparing the amount of lipid droplets in a cell aggregate that is wild-type (or derived from a healthy person) with that of a cell aggregate that is the mutant (or derived from a patient) that lacks the function of the gene.

(Embodiment 2)

Cell aggregates of liver cells are prepared by a known method and the formation of lipid droplets is induced by oleic acid. The refractive index distribution data of the cell aggregates are acquired over time by the refractive index measuring method A1 to A3, B or C. The refractive index distribution data over time can be obtained by photographing the refractive index distribution image at a desired time interval. In each refractive index distribution image, a region having a refractive index greater than 1.375 is determined as a region of lipid droplets, and the area, shape and the like of the region are measured. The time variation of the obtained area, shape, etc. is analyzed. For example, the effect of a gene mutation (or a disease) on lipid metabolism can be assessed by comparing the time course of lipid droplet formation between a cell aggregate that is wild-type (or derived from a healthy person) and a cell aggregate that is the mutant (or derived from a patient) that lacks the function of the gene.

(Embodiment3)

Cell aggregates of liver cells are prepared by a known method and the formation of lipid droplets is induced by oleic acid. This is regarded as a fatty liver model. The fatty liver model is then divided into two groups and a drug is added to one group. The refractive index distribution data of a fatty liver model without administration of the drug (control) and a fatty liver model with administration of the drug are acquired by a refractive index measurement method A1 to A3, B or C. In the obtained refractive index distribution data, a region having a refractive index greater than 1.375 is determined as a region of lipid droplets, and the area of the region is measured. For example, the effect of the drug on lipid metabolism can be assessed by comparing the area of the region of the lipid droplets (the amount of the lipid droplets) in the control with the amount of the lipid droplets in the fatty liver model with administration of the drug.

(Embodiment 4)

Cell aggregates of liver cells are prepared by a known method and the formation of lipid droplets is induced by oleic acid. This is regarded as a fatty liver model. This fatty liver model is divided into two groups . The refractive index distribution data of each fatty liver model are acquired over time by the refractive index measurement methods A1 to A3, B or C. The refractive index distribution data over time can be obtained by photographing the refractive index distribution image at a desired time interval. In one group, a drug is added after the start of taking the refractive index distribution image. In each of the refractive index distribution images of a fatty liver model without administration of the drug (control) and a fatty liver model with administration of the drug, a region having a refractive index greater than 1.375 is determined as a region of lipid droplets, and the area, shape and the like of the region are measured. The time variation of the obtained area, shape, etc. is analyzed. For example, the effect of the drug on lipid metabolism can be assessed by comparing the time course in the area, shape, or the like of the lipid droplet in the control with those in the fatty liver model with administration of the drug.

An important step in the method of dividing a cell population is to associate cells in a three-dimensional culture to positions in the three-dimensional culture. For this purpose, it is necessary to specify one or more positions in the three-dimensional culture. Hereinafter, an additional embodiment of T1 to T4 will be described.

(Embodiment T1)

(Method of Dividing Cell Population)

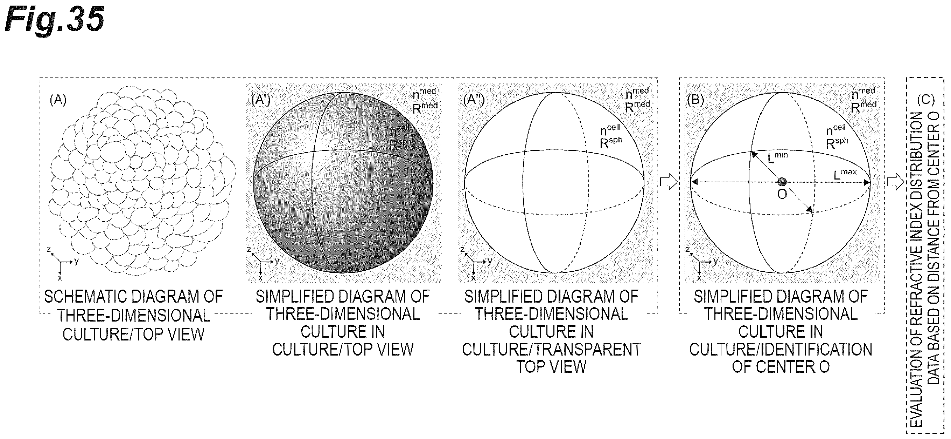

Assume a cell aggregate shown in FIG. 35(A) as a schematic diagram (top view). FIG. 35(A') is a simplified diagram (top view) in which a cell aggregate in culture is regarded as a spherical body, and FIG. 35(A") is a transparent view of FIG. 35(A'). The refractive index distribution data of such a cell aggregate can also be analyzed by the above-described "method of dividing a cell population". To be more specific, first, refractive index distribution data of the medium and the cell aggregate in the medium are obtained by the refractive index measurement methods A1 to A3, B or C. Next, in the obtained refractive index distribution data, the cell aggregate region is determined from the difference between the refractive index $n^{med}$ of the medium and the refractive index $n^{cell}$ of the cells constituting the cell aggregate (FIG. 35(A'), (A"))$_0$ In general, $n^{med}$ is close to the refractive index of water, and can be considered to be, for example, $n^{med}$ = 1.33. In general, $n^{cell}$ has a value higher than $n^{med}$, and can be considered to be, for example, $n^{cell}$ = 1.35.

A region in which the value of the refractive index n is greater than or equal to the value of $n^{cell}$ is defined as a cell aggregate region ($R^{sph}$). A region where the value of the refractive index n is less than the value of $n^{cell}$ is defined as a medium region ($R^{med}$) (FIG. 35(A'), (A")). By considering $R^{sph}$ as a sphere and determining its center O, the center of $R^{sph}$ is specified. For example, a straight line $L^{max}$ having the largest diameter of $R^{sph}$ and a straight line $L^{min}$ having the smallest diameter of $R^{sph}$ are designated, and an intersection point thereof is defined as O (FIG. 35(B)). $L^{max}$ may be equal to $L^{min}$.

Next, the refractive index distribution data is evaluated based on distances from the center O (FIG. 35(C)). It is considered that cells closer to the center O have a lower degree of supply of oxygen, nutrients and the like, and a lower degree of permeation of chemical substances such as drugs. It is considered that cells with a greater distance from the center O have a higher degree of supply of oxygen, nutrients and the like, and a higher degree of permeation of chemical substances such as drugs. Further, an arbitrary point V in the three-dimensional culture may be set instead of the center O, and the refractive index distribution data may be evaluated based on distances from V.

(Embodiment T2)

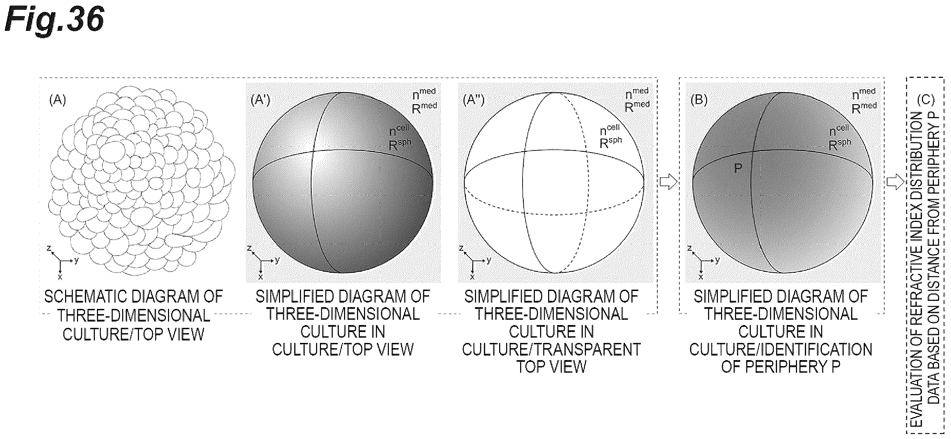

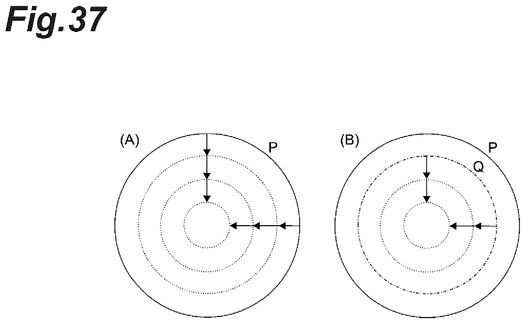

The cell aggregate region ($R^{sph}$) and the medium region ($R^{med}$) are determined in the same manner as in Embodiment T1 (FIG. 36(A'), (A")). The boundary region between $R^{sph}$ and $R^{med}$ (periphery P of the cell aggregate) is specified (FIG. 36(B)). The refractive index distribution data is evaluated based on distances from the periphery P. It is considered that the cells in the vicinity of the periphery P have a higher degree of supply of oxygen, nutrients and the like, and a higher degree of permeation of chemical substances such as drugs. It is considered that as the distance from the periphery P increases, the degree of supply of oxygen, nutrients and the like and the degree of permeation of chemical substances such as drugs and the like are lower (FIG. 37(A)). Instead of the periphery P, an arbitrary plane Q in the three-dimensional culture may be set, and the refractive index distribution data may be evaluated based on distances from Q (FIG. 37(B)). When the cell aggregate is a spherical body, the equidistant line from the periphery P is set to be concentric (FIG. 37(A) and 37(B)). When the cell aggregate includes a distorted shape, for example, the distance from the periphery P can be obtained by using a distance conversion method generally used in image processing.

(Embodiment T3)

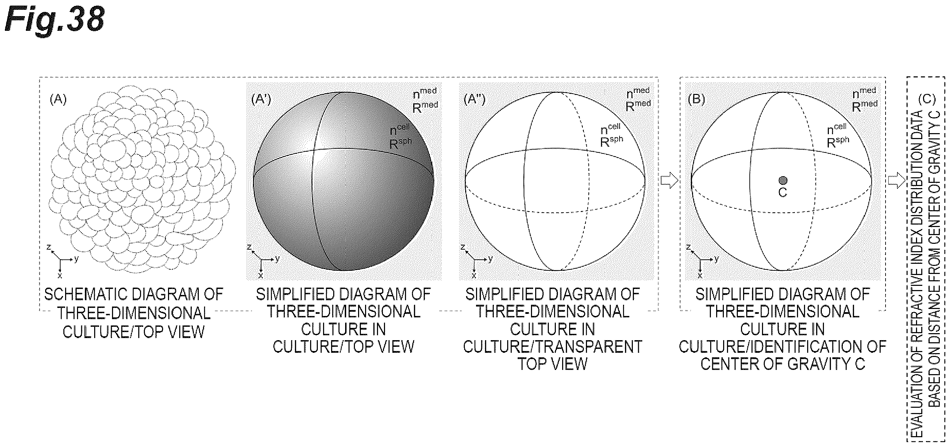

The cell aggregate region ($R^{sph}$) is determined in the same manner as in Embodiment T1 (FIG. 38 (A'), (A")). The center of gravity of the refractive index distribution of $R^{sph}$ (center of gravity C of the cell aggregate) is specified (FIG. 38(B)). This method is considered to be effective for evaluation of an observation object having a large gradient in refractive index distribution, for example. Next, the refractive index distribution data is evaluated based on distances from the center of gravity C (FIG. 38(C)). It is considered that the closer the cell is to the center of gravity C, the lower the degree of supply of oxygen, nutrition and the like, and the degree of permeation of chemical substances such as drugs. It is considered that cells having a greater distance from the center of gravity C have a higher degree of supply of oxygen, nutrients and the like, and a higher degree of permeation of chemical substances such as drugs.

(Embodiment T4)

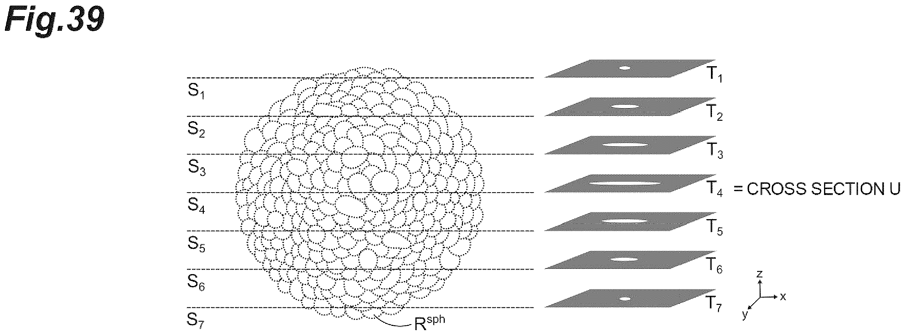

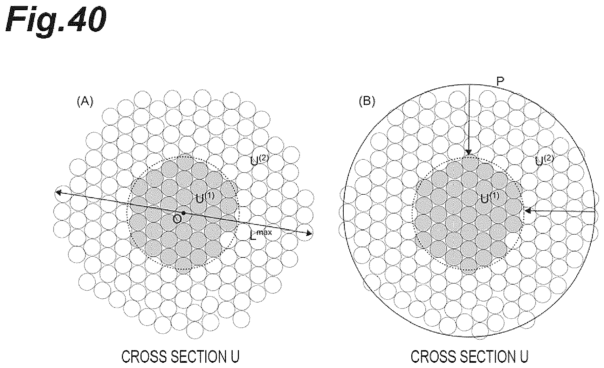

The cell aggregate region ($R^{sph}$) is determined in the same manner as in Embodiment T1 (FIG. 38(A'), (A")). FIG. 39 is a schematic view (side view) of the spheroid. The cross sectional image T is extracted from $R^{sph}$. For example, a plurality of cross sectional images T obtained by slicing $R^{sph}$ along a specific direction S are extracted (FIG. 39). By comparing the cross sections $T_1$ to $T_n$, the cross section U having the largest area is extracted, and one or more positions of the cross section U are specified. For example, $L^{max}$ at which the diameter L of U is maximum is specified, and the midpoint (center O) thereof is specified (FIG. 40(A)). Alternatively, a circle P approximating the periphery of U is specified (FIG. 40(B)).

Next, the refractive index distribution data is evaluated based on distances from the center O or the circle P (FIG. 40(A) and 40(B)). For example, when a specific cell is aggregated in a region located at a specific distance from the center O or the circle P, the cell group may be referred to as U(1), and other cell groups may be distinguished from U(2) (FIG. 40(A) and 40(B)).

In the present embodiment, the section W having the smallest area may be extracted instead of the section U having the largest area. A cross section having a characteristic refractive index distribution may be extracted, or any other cross section may be extracted. The cross sectional image T does not have to be plural. In the extracted cross section, an arbitrary point other than the center O may be designated, or an arbitrary line other than the approximate circle P of the periphery may be designated. Further, the three-dimensional refractive index distribution data may be evaluated based on distances from any point or line specified in the cross section (not limited to the evaluation in the cross section).

**Examples**

(Example 1)

Human liver cancer-derived cells (HepG2) were cultured for one day in a cell culture vessel ("EZSPHERE" manufactured by AGC-Techno Glass Co., Ltd.) having low-cell-adhesive compartments to prepare cell aggregates. The low-cell-adhesive compartments are provided with a low-protein-adhesive coat. Cell aggregates were formed in a DMEM medium containing 10% fetal bovine serum in the low-cell-adhesive compartments.

A portion of the cell aggregates was cultured for one day in a DMEM medium containing 1% fatty acid-free bovine serum albumin instead of fetal bovine serum, with addition of sodium oleate to a final concentration of 0.5 mM, to induce formation of lipid droplets. The remaining portion of the cell aggregates was cultured for an additional day in a DMEM medium containing 10% fetal bovine serum without the addition of oleic acid (control).

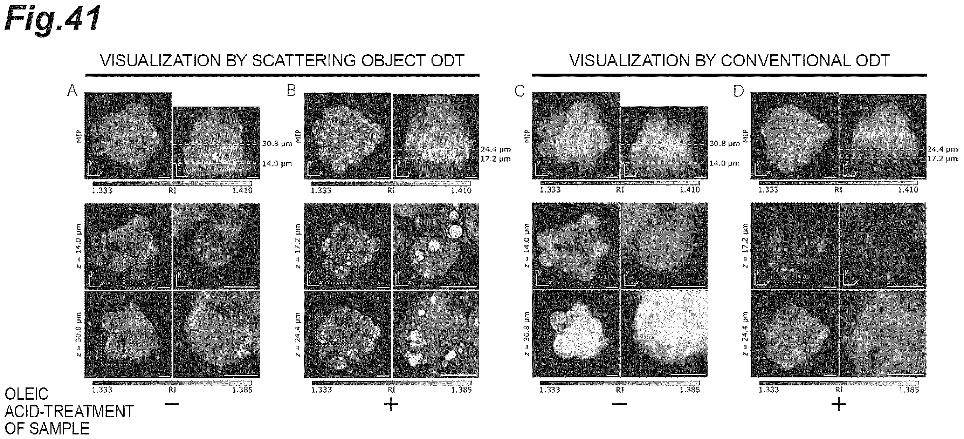

The refractive index distribution data of the control (A, without oleic acid) and the cell aggregate to which oleic acid was added (B) were obtained by the refractive index distribution measuring method A2 (scattering object ODT). FIG. 41 shows the results of coloring for each refractive index.

In the obtained refractive index distribution data, a region where the refractive index (n) was greater than 1.337 was determined as a cell aggregate region. A cell aggregate region having a maximum diameter of 50 to 100 $\mu$m was used as an evaluation target. A region having a refractive index (n) of greater than 1.375 was determined as a lipid droplet region. The area of the lipid droplet region present in the cell aggregate region of interest was measured.

Since many remarkable structures with high refractive index were observed in FIG. 41B, it was confirmed that the addition of oleic acid induced the formation of lipid droplets. The area of the lipid droplet region per cell aggregate in B was estimated to be 2.75 times greater than that in A. This result is consistent with reports on two-dimensional cultured hepatocytes (Non-Patent Documents 5 and 6), and it was shown that lipid droplets can be quantified by the refractive index distribution measuring method A2.

(Comparative Example 1)

The area of the lipid droplet region in the cell aggregate were measured in the same manner as in Example 1 except that the conventional ODT was used instead of the refractive index distribution measuring method A2. The results are shown in FIG. 41. In FIG. 41, C shows the measurement result of the control (without oleic acid), and D shows the measurement result of the cell aggregate to which oleic acid was added. Since a method of suppressing multiple scattered light is not incorporated in the conventional ODT, it is difficult to accurately specify the structure of the lipid droplet in an inner part of the cell aggregate, and it was confirmed that it is practically impossible to quantify the lipid droplet (C and D). In FIG. 41, A and C, and B and D represent the same cross section of the same cell aggregate, respectively.

(Example 2)

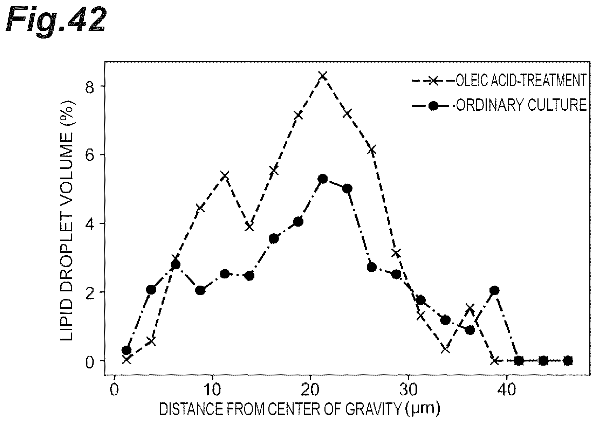

Preparation of cell aggregates, homogenization, and induction of lipid droplet formation were carried out in the same manner as in Example 1. The refractive index distribution data was evaluated in accordance with Embodiment T3 (FIG. 42). The refractive index distribution data was obtained by the refractive index distribution measuring method C. In the obtained refractive index distribution data, the center of gravity C was calculated, and the refractive index distribution data was evaluated based on distances from the center of gravity C. It was divided into concentric circles at intervals of 2.5 $\mu$m from the center of gravity C, and the ratio of the volume of lipid droplets present therein was calculated. The lipid droplet region was set as a region having a refractive index n>1.37, cell region of spheroid as a region having a refractive index n> 1.34 and a medium region as a region having a refractive index n≤1.34 and the calculation was carried out. The results of the cell aggregate of the oleic acid-treated HepG2 (fatty liver model) and the control (without oleic acid) were compared. In both cases, it can be read that many lipid droplets exist in a portion of about 20 $\mu$m from the center of gravity, and the oleic acid treatment (fatty liver model) has a greater ratio of the volume of lipid droplets.

(Example 3)

Human liver cancer-derived cells (HepG2) were cultured for 2 days in a cell culture vessel ("EZSPHERE" manufactured by AGC-Techno Glass Co., Ltd.) having low-cell-adhesive compartments to prepare a cell aggregate. The low-cell-adhesive compartments are provided with a low-protein-adhesive coat. Cell aggregates were formed in a DMEM medium containing 10% fetal bovine serum in the low-cell-adhesive compartments.

To the cell aggregate, sodium oleate was added at a final concentration of 75 $\mu$M, 150 $\mu$M or 300 $\mu$M in a DMEM medium containing 1% fatty acid-free bovine serum albumin instead of fetal bovine serum, and the cell aggregate was cultured for 2 days to induce formation of lipid droplets. A cell aggregate cultured for 2 days in a DMEM medium containing 1% fatty acid-free bovine serum albumin instead of fetal bovine serum without adding sodium oleate was also prepared.

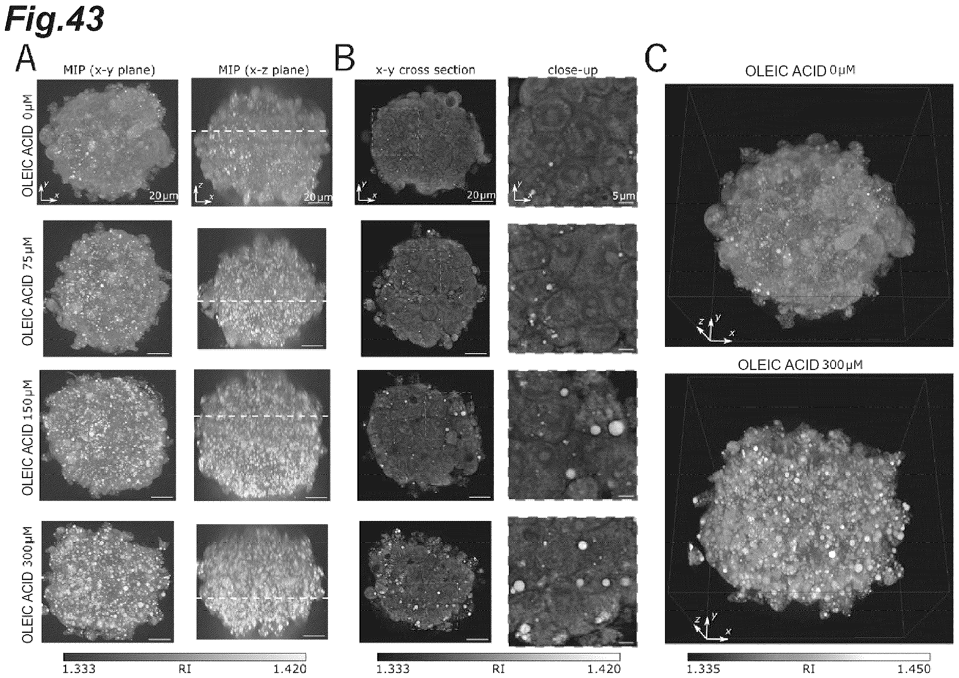

For each sample in which the concentration of oleic acid was 0 $\mu$M, 75 $\mu$M, 150 $\mu$M or 300 $\mu$M, the refractive index distribution data of the cell aggregate was acquired by the refractive index distribution measuring method C. The results of coloring for each refractive index are shown in A to C of FIG. 43.

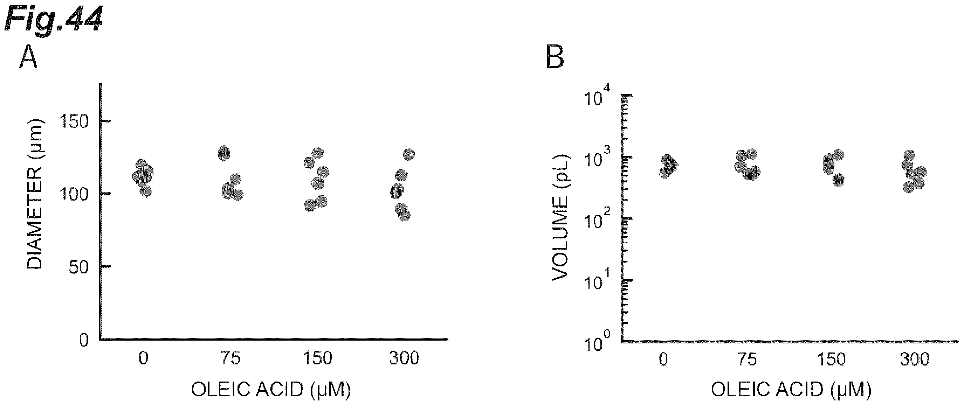

In the obtained refractive index distribution data, a region where the refractive index (n) was greater than 1.340 was determined as a cell aggregate region. In each sample, six cell aggregate regions having diameters of 75 to 150 $\mu$m and volumes of 500 to 5000 pL were extracted as evaluation objects. The diameters and volumes of the six cell aggregates of interest are shown in A and B of FIG. 44, respectively. The average diameter (and standard deviation) of the six cell aggregates of interest was 111.5 $\mu$m (6.1 $\mu$m) for the sample having an oleic acid concentration of 0 $\mu$M, 111.5 $\mu$m (13.3 $\mu$m) for the sample having an oleic acid concentration of 75 $\mu$M, 109.6 $\mu$m (14.4 $\mu$m) for the sample having an oleic acid concentration of 150 $\mu$M, and 103.0 $\mu$m (15.3 $\mu$m) for the sample having an oleic acid concentration of 300 $\mu$M. The average diameter and shape of the cell aggregates of interest were approximately the same among the samples. In the cell aggregate region of interest, a region having a refractive index (n) of greater than 1.375 was determined as a lipid droplet region. The ratio of the volume of the lipid droplet region to the volume of the cell aggregate region of interest (also referred to as the "ratio of the lipid droplet region"). ) was calculated.

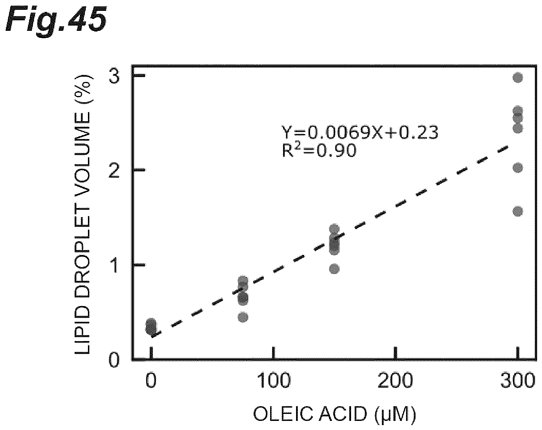

FIG. 45 shows the correlation between the concentration of oleic acid and the percentage of lipid droplet region. The dashed line in FIG. 45 corresponds to an approximate straight line, and the expression thereof is represented by Y=0.0069X+0.23. The correlation coefficient ($R^2$) between the concentration of oleic acid and the ratio of lipid droplet regions was 0.90, and it was confirmed that there was a high correlation between the concentration of oleic acid and the ratio of lipid droplet region. This example further confirms that a quantitative assay of lipid droplets can be achieved by the method of the present invention.

Reference Signs List

1A - 1J - observation apparatus, 2 - recording medium, 11 - light source, 12 - lens, 13 - light input end, 14 - optical fiber, 15 - fiber coupler, 16, 17 - optical fiber, 18, 19 - light output end, 21 - lens, 22 - mirror, 23 - lens, 24 - condenser lens, 25 - objective lens, 31 - lens, 32 - mirror, 33 - drive unit, 34 - lens, 41 - beam splitter, 42 - lens, 43 - imaging unit, 44 - mirror, 50 - analysis unit, 51 - interference intensity image acquisition unit, 52 - first complex amplitude image generation unit, 53 - second complex amplitude image generation unit, 54 - two-dimensional phase image generation unit, 55 - three-dimensional phase image generation unit, 56 - refractive index distribution calculation unit, 57 - display unit, 58 - storage unit, 60 - analysis unit, 61 - interference intensity image acquisition unit, 62 - first complex amplitude image generation unit, 63 - second complex amplitude image generation unit, 64 - phase conjugate operation unit, 65 - two-dimensional phase image generation unit, 66 - three-dimensional phase image generation unit, 67 - refractive index distribution calculation unit, 68 - display unit, 69 - storage unit, 70 - analysis unit, 71 - interference intensity image acquisition unit, 72 - first complex amplitude image generation unit, 73 - second complex amplitude image generation unit, 74 - two-dimensional phase image generation unit, 75 - three-dimensional phase image generation unit, 76 - refractive index distribution calculation unit, 77 - third complex amplitude image generation unit, 78 - display unit, 79 - storage unit.

**Claims**

1.  A method for evaluating a refractive index distribution comprising:

    a data acquisition step of acquiring refractive index distribution data of an observation object;
    a selection step of selecting whether or not an object included in the refractive index distribution data is an evaluation object; and
    an evaluation step of evaluating the refractive index distribution of the evaluation object.

2.  The evaluation method according to claim 1, wherein
    the data acquisition step includes:

acquiring for each of a plurality of light irradiation directions, an interference intensity image generated by interference between light irradiating the evaluation object along each of the plurality of light irradiation directions and passed through the evaluation object and reference light;

a step of generating, for each of the plurality of light irradiation directions, a complex amplitude image at each of a plurality of positions based on the interference intensity image;

a step of generating, for each of the plurality of positions, a complex differential interference image of each of the plurality of light irradiation directions based on the complex amplitude image of each of the plurality of light irradiation directions, and generating a two-dimensional phase image based on the complex differential interference image of each of the plurality of light irradiation directions; and

a step of calculating a refractive index distribution based on the two-dimensional phase image at each of the plurality of positions.

3. The evaluation method according to claim 1 or 2, wherein
the selection step includes:

a step of acquiring at least one refractive index cross sectional data in a certain direction from the refractive index distribution data; and
a step of selecting whether or not an object included in the refractive index cross sectional data is an evaluation object.

4. The evaluation method according to claim 1 or 2, wherein
an evaluation method in the evaluation step includes:
a step of extracting a region having a refractive index greater than or less than a threshold.

5. The evaluation method according to claim 4, wherein
an evaluation method in the evaluation step includes:
a step of dividing the region by its shape, its size, its density or its position in the observation object.

6. The evaluation method according to claim 4, wherein
an evaluation method in the evaluation step includes:
a step of specifying one or more positions in the evaluation object and dividing the evaluation object into a region present at a predetermined distance from the position and a region present at a distance farther than the predetermined distance from the position.

7. The evaluation method according to claim 1 or 2, wherein the observation object is a three-dimensional culture.

8. The evaluation method according to claim 1 or 2, wherein
the refractive index distribution data includes:
at least one refractive index data selected from the group consisting of a lipid droplet, a mitochondrion, a vesicle, a nucleolus and DNA.

9. An apparatus for evaluating a refractive index distribution comprising:

a data acquisition unit for acquiring refractive index distribution data of an observation object;
a selection unit for obtaining an indicator for an object included in the refractive index distribution data from the refractive index distribution data and selecting whether or not the object is an evaluation object based on the indicator; and
an evaluation unit for evaluating the refractive index distribution of the evaluation object.

10. An information processing program for causing a computer to execute steps:

a data acquisition step of acquiring refractive index distribution data of an observation object;
a selection step of obtaining an indicator for an object included in the refractive index distribution data from the refractive index distribution data and selecting whether or not the object is an evaluation object based on the indicator; and
an evaluation step of evaluating the refractive index distribution of the evaluation object.

# Fig.1

*Fig.2*

ANALYSIS UNIT — 50

INTERFERENCE INTENSITY IMAGE ACQUISITION UNIT — 51

FIRST COMPLEX AMPLITUDE IMAGE GENERATION UNIT — 52

SECOND COMPLEX AMPLITUDE IMAGE GENERATION UNIT — 53

TWO-DIMENSIONAL PHASE IMAGE GENERATION UNIT — 54

THREE-DIMENSIONAL PHASE IMAGE GENERATION UNIT — 55

REFRACTIVE INDEX DISTRIBUTION CALCULATION UNIT — 56

DISPLAY UNIT — 57

STORAGE UNIT — 58

RECORDING MEDIUM — 2

EP 4 431 915 A1

Fig.3

1C

ANALYSIS UNIT 50

- INTERFERENCE INTENSITY IMAGE ACQUISITION UNIT 51
- FIRST COMPLEX AMPLITUDE IMAGE GENERATION UNIT 52
- SECOND COMPLEX AMPLITUDE IMAGE GENERATION UNIT 53
- TWO-DIMENSIONAL PHASE IMAGE GENERATION UNIT 54
- THREE-DIMENSIONAL PHASE IMAGE GENERATION UNIT 55
- REFRACTIVE INDEX DISTRIBUTION CALCULATION UNIT 56
- DISPLAY UNIT 57
- STORAGE UNIT 58

RECORDING MEDIUM 2

# Fig.4

INTERFERENCE INTENSITY IMAGE ACQUISITION ~S1

INTERFERENCE INTENSITY IMAGE FOR
EACH LIGHT IRRADIATION DIRECTION

FIRST COMPLEX AMPLITUDE
IMAGE GENERATION ~S2

COMPLEX AMPLITUDE IMAGE FOR
EACH LIGHT IRRADIATION DIRECTION

SECOND COMPLEX AMPLITUDE
IMAGE GENERATION ~S3

COMPLEX AMPLITUDE IMAGE FOR
EACH POSITION AND EACH
LIGHT IRRADIATION DIRECTION

TWO-DIMENSIONAL PHASE IMAGE GENERATION ~S4

TWO-DIMENSIONAL PHASE IMAGE
FOR EACH POSITION

THREE-DIMENSIONAL PHASE IMAGE GENERATION ~S5

THREE-DIMENSIONAL
PHASE IMAGE

REFRACTIVE INDEX DISTRIBUTION CALCULATION ~S6

THREE-DIMENSIONAL REFRACTIVE
INDEX DISTRIBUTION

*Fig.5*

(a)

(b)

(c)

Fig.6

## Fig.7

(a)

(b)

## Fig.8

EP 4 431 915 A1

# Fig.9

```
┌─────────────────────────────────┐
│   COMPLEX AMPLITUDE IMAGE FOR    │
│ EACH LIGHT IRRADIATION DIRECTION │
└─────────────────────────────────┘
                 │
┌─────────────────────────────────┐
│  PHASE CORRECTION AND SUMMATION OF │ ～S11
│     COMPLEX AMPLITUDE IMAGES     │
└─────────────────────────────────┘
                 │
┌─────────────────────────────────┐
│    COMPLEX AMPLITUDE SUMMATION   │
│     IMAGE FOR EACH POSITION      │
└─────────────────────────────────┘
                 │
┌─────────────────────────────────┐
│      PHASE IMAGE GENERATION      │ ～S12
└─────────────────────────────────┘
                 │
┌─────────────────────────────────┐
│    TWO-DIMENSIONAL PHASE IMAGE   │
│        FOR EACH POSITION         │
└─────────────────────────────────┘
```

# Fig.10

```
┌─────────────────────────────────┐
│   COMPLEX AMPLITUDE IMAGE FOR    │
│      EACH POSITION AND EACH      │
│    LIGHT IRRADIATION DIRECTION   │
└─────────────────────────────────┘
                 │
┌─────────────────────────────────┐
│   COMPLEX DIFFERENTIAL INTERFERENCE   │──── S21
│         IMAGE GENERATION         │
└─────────────────────────────────┘
                 │
┌─────────────────────────────────┐
│  COMPLEX DIFFERENTIAL INTERFERENCE   │
│     IMAGE FOR EACH POSITION AND  │
│   EACH LIGHT IRRADIATION DIRECTION   │
└─────────────────────────────────┘
                 │
┌─────────────────────────────────┐
│  PHASE DIFFERENTIAL IMAGE GENERATION  │──── S22
└─────────────────────────────────┘
                 │
┌─────────────────────────────────┐
│     PHASE DIFFERENTIAL IMAGE     │
│        FOR EACH POSITION         │
└─────────────────────────────────┘
                 │
┌─────────────────────────────────┐
│       PHASE IMAGE GENERATION     │──── S23
└─────────────────────────────────┘
                 │
┌─────────────────────────────────┐
│   TWO-DIMENSIONAL PHASE IMAGE    │
│        FOR EACH POSITION         │
└─────────────────────────────────┘
```

Fig.11

# Fig.12

COMPLEX AMPLITUDE IMAGE FOR
EACH POSITION AND EACH
LIGHT IRRADIATION DIRECTION

DIVIDING INTO BATCHES AND PHASE CORRECTION
AND SUMMATION OF COMPLEX AMPLITUDE IMAGES
FOR EACH BATCH ～S31

COMPLEX AMPLITUDE SUMMATION
IMAGE FOR EACH POSITION
AND EACH BATCH

COMPLEX DIFFERENTIAL INTERFERENCE
IMAGE GENERATION ～S32

COMPLEX DIFFERENTIAL
INTERFERENCE IMAGE FOR
EACH POSITION AND EACH BATCH

PHASE DIFFERENTIAL IMAGE GENERATION ～S33

PHASE DIFFERENTIAL IMAGE
FOR EACH POSITION

PHASE IMAGE GENERATION ～S34

TWO-DIMENSIONAL PHASE IMAGE
FOR EACH POSITION

**Fig.13**

EP 4 431 915 A1

ANALYSIS UNIT — 60

INTERFERENCE INTENSITY IMAGE ACQUISITION UNIT — 61

FIRST COMPLEX AMPLITUDE IMAGE GENERATION UNIT — 62

SECOND COMPLEX AMPLITUDE IMAGE GENERATION UNIT — 63

PHASE CONJUGATE OPERATION UNIT — 64

TWO-DIMENSIONAL PHASE IMAGE GENERATION UNIT — 65

THREE-DIMENSIONAL PHASE IMAGE GENERATION UNIT — 66

REFRACTIVE INDEX DISTRIBUTION CALCULATION UNIT — 67

DISPLAY UNIT — 68

STORAGE UNIT — 69

RECORDING MEDIUM — 2

1D

**Fig.14**

ANALYSIS UNIT — 60

INTERFERENCE INTENSITY IMAGE ACQUISITION UNIT — 61

FIRST COMPLEX AMPLITUDE IMAGE GENERATION UNIT — 62

SECOND COMPLEX AMPLITUDE IMAGE GENERATION UNIT — 63

PHASE CONJUGATE OPERATION UNIT — 64

TWO-DIMENSIONAL PHASE IMAGE GENERATION UNIT — 65

THREE-DIMENSIONAL PHASE IMAGE GENERATION UNIT — 66

REFRACTIVE INDEX DISTRIBUTION CALCULATION UNIT — 67

DISPLAY UNIT — 68

STORAGE UNIT — 69

RECORDING MEDIUM — 2

EP 4 431 915 A1

# Fig.15

ANALYSIS UNIT — 60

INTERFERENCE INTENSITY IMAGE ACQUISITION UNIT — 61

FIRST COMPLEX AMPLITUDE IMAGE GENERATION UNIT — 62

SECOND COMPLEX AMPLITUDE IMAGE GENERATION UNIT — 63

PHASE CONJUGATE OPERATION UNIT — 64

TWO-DIMENSIONAL PHASE IMAGE GENERATION UNIT — 65

THREE-DIMENSIONAL PHASE IMAGE GENERATION UNIT — 66

REFRACTIVE INDEX DISTRIBUTION CALCULATION UNIT — 67

DISPLAY UNIT — 68

STORAGE UNIT — 69

RECORDING MEDIUM — 2

EP 4 431 915 A1

# Fig.16

INTERFERENCE INTENSITY IMAGE ACQUISITION — S61

NTERFERENCE INTENSITY IMAGE FOR
EACH LIGHT IRRADIATION DIRECTION

FIRST COMPLEX AMPLITUDE IMAGE GENERATION — S62

COMPLEX AMPLITUDE IMAGE FOR
EACH LIGHT IRRADIATION DIRECTION

SECOND COMPLEX AMPLITUDE IMAGE GENERATION — S63

COMPLEX AMPLITUDE IMAGE FOR
EACH POSITION AND EACH
LIGHT IRRADIATION DIRECTION

PHASE CONJUGATE OPERATION — S64

COMPLEX AMPLITUDE IMAGE FOR
EACH POSITION AND EACH
LIGHT IRRADIATION DIRECTION

TWO-DIMENSIONAL PHASE IMAGE GENERATION — S65

TWO-DIMENSIONAL PHASE IMAGE
FOR EACH POSITION

THREE-DIMENSIONAL PHASE IMAGE GENERATION — S66

THREE -DIMENSIONAL PHASE IMAGE

REFRACTIVE INDEX DISTRIBUTION CALCULATION — S67

THREE -DIMENSIONAL REFRACTIVE
INDEX DISTRIBUTION

Fig.17

# Fig.18

SECOND COMPLEX AMPLITUDE
IMAGE GENERATION STEP S63

PHASE
CONJUGATE
OPERATION
STEP S64

TWO-DIMENSIONAL PHASE
IMAGE GENERATION STEP S65

$z = z_1$

$z = z_1$

$z = z_0$

$z = z_2$

$z = z_2$

COMPLEX
AMPLITUDE IMAGE

$z = z_3$

$z = z_3$

COMPLEX
AMPLITUDE IMAGE

COMPLEX
AMPLITUDE IMAGE

COMPLEX DIFFERENTIAL
INTERFERENCE IMAGE

EP 4 431 915 A1

## Fig.19

SECOND COMPLEX AMPLITUDE
IMAGE GENERATION STEP S63

TWO-DIMENSIONAL PHASE
IMAGE GENERATION STEP S65

PHASE
CONJUGATE
OPERATION
STEP S64

$z = z_0$

$z = z_0$

$z = z_1$

$z = z_2$

$z = z_3$

COMPLEX
AMPLITUDE IMAGE

COMPLEX
AMPLITUDE IMAGE

COMPLEX
AMPLITUDE IMAGE

COMPLEX DIFFERENTIAL
INTERFERENCE IMAGE

# Fig.20

SECOND COMPLEX
AMPLITUDE IMAGE
GENERATION STEP S63
(FIRST STAGE)

PHASE
CONJUGATE
OPERATION
STEP S64

SECOND COMPLEX
AMPLITUDE IMAGE
GENERATION STEP S63
(SECOND STAGE)

TWO-DIMENSIONAL
PHASE IMAGE
GENERATION STEP S65

$z = z_1$

$z = z_1$

$z = z_1$

$z = z_2$

$z = 0$
COMPLEX
AMPLITUDE
IMAGE

$z = z_3$

$z = z_3$

$z = z_3$

$z = z_4$

$z = z_5$

$z = z_5$

$z = z_5$

$z = z_6$

COMPLEX
AMPLITUDE IMAGE

COMPLEX
AMPLITUDE IMAGE

COMPLEX
AMPLITUDE IMAGE

COMPLEX
DIFFERENTIAL
INTERFERENCE
IMAGE

EP 4 431 915 A1

# Fig.21

THREE-DIMENSIONAL
PHASE IMAGE
GENERATION STEP S66

PHASE IMAGE GENERATED FROM
COMPLEX AMPLITUDE IMAGE AFTER
PHASE CONJUGATE OPERATION
IN TWO-DIMENSIONAL PHASE
IMAGE GENERATION STEP S65

REFRACTIVE INDEX
DISTRIBUTION
CALCULATION
STEP S67

PHASE IMAGE GENERATED FROM
COMPLEX AMPLITUDE IMAGE BEFORE
PHASE CONJUGATE OPERATION
IN TWO-DIMENSIONAL PHASE
IMAGE GENERATION STEP S65

THREE -DIMENSIONAL
PHASE IMAGE

THREE -DIMENSIONAL
REFRACTIVE INDEX
DISTRIBUTION

EP 4 431 915 A1

# Fig.22

LIGHT
IRRADIATION $\quad U_{in}(k_{in})$

OBSERVATION
OBJECT $\quad T(r_{out}, k_{in})$

$u_{out}(r_{out})$

IMAGING

IMAGING UNIT

# Fig.23

IMAGING UNIT

IMAGING

$u_{in}(r_{in})$

OBSERVATION OBJECT

$S(r_{in}, k_{out})$

LIGHT IRRADIATION

$U_{out}(k_{out})$

# Fig.24

# Fig.25

ANALYSIS UNIT 70

INTERFERENCE INTENSITY
IMAGE ACQUISITION UNIT 71

FIRST COMPLEX AMPLITUDE
IMAGE GENERATION UNIT 72

SECOND COMPLEX AMPLITUDE
IMAGE GENERATION UNIT 73

TWO-DIMENSIONAL PHASE
IMAGE GENERATION UNIT 74

THREE-DIMENSIONAL PHASE
IMAGE GENERATION UNIT 75

REFRACTIVE INDEX DISTRIBUTION
CALCULATION UNIT 76

THIRD COMPLEX AMPLITUDE
IMAGE GENERATION UNIT 77

DISPLAY UNIT 78

STORAGE UNIT 79

RECORDING MEDIUM 2

Fig.26

EP 4 431 915 A1

**Fig.27**

| | |
|---|---|
| ANALYSIS UNIT | ~70 |
| INTERFERENCE INTENSITY IMAGE ACQUISITION UNIT | ~71 |
| FIRST COMPLEX AMPLITUDE IMAGE GENERATION UNIT | ~72 |
| SECOND COMPLEX AMPLITUDE IMAGE GENERATION UNIT | ~73 |
| TWO-DIMENSIONAL PHASE IMAGE GENERATION UNIT | ~74 |
| THREE-DIMENSIONAL PHASE IMAGE GENERATION UNIT | ~75 |
| REFRACTIVE INDEX DISTRIBUTION CALCULATION UNIT | ~76 |
| THIRD COMPLEX AMPLITUDE IMAGE GENERATION UNIT | ~77 |
| DISPLAY UNIT | ~78 |
| STORAGE UNIT | ~79 |
| RECORDING MEDIUM | ~2 |

EP 4 431 915 A1

## Fig.28

INTERFERENCE INTENSITY IMAGE ACQUISITION ──S71

INTERFERENCE INTENSITY IMAGE FOR
EACH LIGHT IRRADIATION DIRECTION

FIRST COMPLEX AMPLITUDE IMAGE GENERATION ──S72

COMPLEX AMPLITUDE IMAGE FOR
EACH LIGHT IRRADIATION DIRECTION

$j = 0$ ──S81

$j \leftarrow j + 1$ ──S82

──S83

SECOND COMPLEX AMPLITUDE IMAGE GENERATION ──S73

TWO-DIMENSIONAL PHASE IMAGE GENERATION ──S74

THREE-DIMENSIONAL PHASE IMAGE GENERATION ──S75

REFRACTIVE INDEX DISTRIBUTION CALCULATION ──S76

THREE -DIMENSIONAL REFRACTIVE
INDEX DISTRIBUTION

──S84
$j < J$    false → END

true

THIRD COMPLEX AMPLITUDE IMAGE GENERATION ──S77

COMPLEX AMPLITUDE IMAGE FOR
EACH LIGHT IRRADIATION DIRECTION

*Fig.29*

COMPLEX AMPLITUDE IMAGE FOR
EACH LIGHT IRRADIATION DIRECTION

S83

SECOND COMPLEX AMPLITUDE IMAGE GENERATION — S73

COMPLEX AMPLITUDE IMAGE FOR
EACH POSITION AND EACH
LIGHT IRRADIATION DIRECTION

TWO-DIMENSIONAL PHASE IMAGE GENERATION — S74

TWO-DIMENSIONAL PHASE
IMAGE FOR EACH POSITION

THREE-DIMENSIONAL PHASE IMAGE GENERATION — S75

THREE-DIMENSIONAL PHASE IMAGE

REFRACTIVE INDEX DISTRIBUTION CALCULATION — S76

THREE -DIMENSIONAL
REFRACTIVE INDEX DISTRIBUTION

## Fig.30

OBSERVATION OBJECT

$z = z_3$
$z = z_2$
$z = z_1$
$z = z_0$

THIRD BLOCK

$z = z_3$
$z = z_2$

SECOND BLOCK

$z = z_2$
$z = z_1$

FIRST BLOCK

$z = z_1$
$z = z_0$

## Fig.31

COMPLEX AMPLITUDE
IMAGE IN THIRD
BLOCK

STEP S83 →

$z = z_3$
$z = z_2$

REFRACTIVE
INDEX DISTRIBUTION
OF THIRD BLOCK

THIRD COMPLEX
AMPLITUDE IMAGE
GENERATION STEP S77

COMPLEX AMPLITUDE
IMAGE IN SECOND
BLOCK

STEP S83 →

$z = z_2$
$z = z_1$

REFRACTIVE
INDEX DISTRIBUTION
OF SECOND BLOCK

THIRD COMPLEX
AMPLITUDE IMAGE
GENERATION STEP S77

COMPLEX AMPLITUDE
IMAGE IN FIRST
BLOCK

STEP S83 →

$z = z_1$
$z = z_0$

z
y
x

REFRACTIVE
INDEX DISTRIBUTION
OF FIRST BLOCK

$z = z_3$
$z = z_2$
$z = z_1$
$z = z_0$

z
y
x

REFRACTIVE INDEX
DISTRIBUTION OF
OBSERVATION OBJECT

EP 4 431 915 A1

## Fig.32

$z = z_{j-1} + 7\Delta z = z_j$
$z = z_{j-1} + 6\Delta z$
$z = z_{j-1} + 5\Delta z$
$z = z_{j-1} + 4\Delta z$
$z = z_{j-1} + 3\Delta z$
$z = z_{j-1} + 2\Delta z$
$z = z_{j-1} + \Delta z$
$z = z_{j-1}$

j-TH BLOCK

# Fig.33

```
┌─────────────────────────────────┐
│   COMPLEX AMPLITUDE IMAGE       │
│   AT FIRST POSITION z_{j-1}     │
└─────────────────────────────────┘
              │
┌─────────────────────────────────┐
│           z ← z_{j-1}           │──── S41
└─────────────────────────────────┘
              │
              ▼
┌─────────────────────────────────┐
│  INTERACTION BETWEEN THE COMPLEX│
│  AMPLITUDE AT POSITION z AND    │──── S42
│  REFRACTIVE INDEX DISTRIBUTION  │
│  AT POSITION z                  │
└─────────────────────────────────┘
              │
┌─────────────────────────────────┐
│   PROPAGATION OF DISTANCE Δz    │──── S43
└─────────────────────────────────┘
              │
┌─────────────────────────────────┐
│   COMPLEX AMPLITUDE IMAGE       │
│   AT POSITION z+Δz              │
└─────────────────────────────────┘
              │
┌─────────────────────────────────┐
│           z ← z+Δz              │──── S44
└─────────────────────────────────┘
              │
        ╱───────────╲  ─── S45
  true ╱   z < z_j    ╲
◄─────╲               ╱
        ╲───────────╱
              │ false
┌─────────────────────────────────┐
│   COMPLEX AMPLITUDE IMAGE       │
│   AT SECOND POSITION z_j        │
└─────────────────────────────────┘
```

Fig.34

EP 4 431 915 A1

# Fig.35

(A) SCHEMATIC DIAGRAM OF THREE-DIMENSIONAL CULTURE/TOP VIEW

(A') SIMPLIFIED DIAGRAM OF THREE-DIMENSIONAL CULTURE IN CULTURE/TOP VIEW
$n^{med}$ $R^{med}$ $n^{cell}$ $R^{sph}$

(A'') SIMPLIFIED DIAGRAM OF THREE-DIMENSIONAL CULTURE IN CULTURE/TRANSPARENT TOP VIEW
$n^{med}$ $R^{med}$ $n^{cell}$ $R^{sph}$

(B) SIMPLIFIED DIAGRAM OF THREE-DIMENSIONAL CULTURE IN CULTURE/IDENTIFICATION OF CENTER O
$n^{med}$ $R^{med}$ $n^{cell}$ $R^{sph}$ $L^{min}$ $L^{max}$ O

(C) EVALUATION OF REFRACTIVE INDEX DISTRIBUTION DATA BASED ON DISTANCE FROM CENTER O

Fig.36

# Fig.37

*Fig.38*

Fig.39

## Fig.40

(A)

CROSS SECTION U

(B)

CROSS SECTION U

# Fig.41

VISUALIZATION BY SCATTERING OBJECT ODT

VISUALIZATION BY CONVENTIONAL ODT

OLEIC
ACID-TREATMENT
OF SAMPLE

EP 4 431 915 A1

# Fig.42

**Fig.43**

A
MIP (x-y plane)   MIP (x-z plane)

OLEIC ACID 0μM
OLEIC ACID 75μM
OLEIC ACID 150μM
OLEIC ACID 300μM

20μm   20μm

1.333   RI   1.420

B
x-y cross section   close-up

20μm   5μm

1.333   RI   1.420

C
OLEIC ACID 0μM

OLEIC ACID 300μM

1.335   RI   1.450

EP 4 431 915 A1

## Fig.44

A

B

# *Fig.45*

# EP 4 431 915 A1

## INTERNATIONAL SEARCH REPORT

| | International application No. |
|---|---|
| | **PCT/JP2022/026854** |

**A. CLASSIFICATION OF SUBJECT MATTER**

*G01N 21/41*(2006.01)i; *G01N 21/45*(2006.01)i; *G02B 21/00*(2006.01)i; *G02B 21/36*(2006.01)i
FI: G01N21/41 Z; G01N21/45 A; G02B21/00; G02B21/36

According to International Patent Classification (IPC) or to both national classification and IPC

**B. FIELDS SEARCHED**

Minimum documentation searched (classification system followed by classification symbols)

G01N21/00-21/01; G01N21/17-21/61; G02B21/00; G02B21/36

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | KR 10-1855366 B1 (TOMOCUBE, INC.) 09 May 2018 (2018-05-09) | 1, 3-5, 7-10 |
| | paragraphs [0038]-[0099], fig. 1-6 | |
| Y | paragraphs [0038]-[0099], fig. 1-6 | 6 |
| A | | 2 |
| Y | 石丸伊知郎, 生きたままの細胞内3次元成分分布時系列計測 単一細胞分光トモグラフィ, Bio Industry. vol. 25, no. 2, 2008, pages 55-66, (ISHIMARU, Ichirou. 3D-Time Series Compositional Distribution Measurement of Single Living Cells: Spectroscopy-Tomography of Single Cells.) pages 58-59, fig. 5 | 6 |
| A | PARK, S. et al. Label-Free Tomographic Imaging of Lipid Droplets in Foam Cells for Machine-Learning-Assisted Therapeutic Evaluation of Targeted Nanodrugs. ACS Nano 2020. vol. 14, 07 January 2020, pages 1856-1865, https://doi.org/10.1021/acsnano.9b07993 fig. 1, etc. | 1-10 |
| A | WO 2020/013325 A1 (THE UNIV. OF TOKYO) 16 January 2020 (2020-01-16) | 1-10 |

☐ Further documents are listed in the continuation of Box C.　　☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 August 2022** | **30 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/026854**

| Patent document cited in search report | | | Publication date (day/month/year) | Patent family member(s) | | | Publication date (day/month/year) |
|---|---|---|---|---|---|---|---|
| KR | 10-1855366 | B1 | 09 May 2018 | (Family: none) | | | |
| WO | 2020/013325 | A1 | 16 January 2020 | EP | 3822685 | A1 | |
| | | | | CN | 112771432 | A | |

Form PCT/ISA/210 (patent family annex) (January 2015)

80

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016223893 A

**Non-patent literature cited in the description**

- **HEYENS et al.** Liver fibrosis in non-alcoholic fatty liver disease: From liver biopsy to non-invasive biomarkers in diagnosis and treatment. *Front Med.,* 2021
- **DEPRINCE et al.** Dysregulated lipid metabolism links NAFLD to cardiovascular disease. *Mol Metab.,* 2020
- **CHANG et al.** Monolayer and spheroid culture of human liver hepatocellular carcinoma cell line cells demonstrate distinct global gene expression patterns and functional phenotypes. *Tissue Eng Part A,* 2009
- **NISHIKAWA et al.** Optimization of albumin secretion and metabolic activity of cytochrome P450 1A1 of human hepatoblastoma HepG2 cells in multicellular spheroids by controlling spheroid size. *BiolPharm Bull.,* 2017
- **KIM et al.** Three-dimensional label-free imaging and quantification of lipid droplets in live hepatocytes. *Sci Rep.,* 2016
- **GOMEZ-LECHON et al.** A human hepatocellular in vitro model to investigate steatosis. *Chem Biol Interact.,* 2007
- **SUNGSAM KANG et al.** Imaging deep within a scattering medium using collective accumulation of single-scattered waves. *NATURE PHOTONICS,* 2015, vol. 9, 253-258